# EUROPEAN PATENT SPECIFICATION

(11) **EP 2 866 837 B1**
(45) Date of publication and mention of the grant of the patent: **14.12.2022**
(21) Application number: 13758976.8
(22) Date of filing: 27.06.2013
(51) Int. Cl.: A61K 9/10, A61K 47/34, A61K 31/445, A61K 31/565, A61K 31/57, A61K 38/13

(54) **BIODEGRADABLE DRUG DELIVERY FOR HYDROPHOBIC COMPOSITIONS**
BIOLOGISCH ABBAUBARE WIRKSTOFFFREISETZUNG FÜR HYDROPHOBE ZUSAMMENSETZUNGEN
ADMINISTRATION DE MÉDICAMENT BIODÉGRADABLE POUR COMPOSITIONS HYDROPHOBES

(30) Priority: 27.06.2012 US 201261665192 P
(43) Date of publication of application: 06.05.2015
(73) Proprietor: MEDINCELL S.A., 34830 Jacou (FR)
(72) Inventor: GAUDRIAULT, Georges, 34080 Montpellier (FR); ROBERGE, Christophe, 34920 Le Crès (FR)
(74) Representative: D Young & Co LLP
(86) International application number: PCT/IB2013/001547
(87) International publication number: WO 2014/001904

(56) References cited:
- EP-A2- 2 359 860
- WO-A1-02/45689
- WO-A1-93/00070
- WO-A1-93/24154
- WO-A1-97/10849
- WO-A2-2007/019439
- WO-A2-2009/129509
- WO-A2-2012/090070
- US-A- 4 745 160
- US-A1- 2005 112 170
- US-B1- 6 350 812
- US-B2- 6 592 899
- DATABASE WPI Section Ch, Week 201064 Thomson Scientific, London, GB; Class A96, AN 2010-L68935 XP002716536, LI X; LIU Y; ZHANG Y; ZHOU Y: "Polymer micelle composition used for preventing transplant rejection after organism or tissue transplant, or autoimmune or autoimmune related disease, comprises cyclosporine A and block copolymer", -& CN 101 810 560 A ((UYPK) UNIV PEKING) 25 August 2010 (2010-08-25)
- DATABASE MEDLINE [Online] US NATIONAL LIBRARY OF MEDICINE (NLM), BETHESDA, MD, US; 2005, CHEN SIBAO ET AL: "In vitro release of levonorgestrel from phase sensitive and thermosensitive smart polymer delivery systems", XP002716537, Database accession no. NLM15926681 & CHEN SIBAO ET AL: "In vitro release of levonorgestrel from phase sensitive and thermosensitive smart polymer delivery systems", PHARMACEUTICAL DEVELOPMENT AND TECHNOLOGY 2005, vol. 10, no. 2, 2005, pages 319-325, ISSN: 1083-7450

## Description

### FIELD OF THE INVENTION

The present invention relates to biodegradable drug delivery compositions comprising a triblock copolymer containing a polyester and a polyethylene glycol and a diblock copolymer containing a polyester and an end-capped polyethylene glycol, as well as a pharmaceutically hydrophobic active principle one of which is medroxyprogesterone acetate, levonorgestrel, cyclosporine, progesterone or bupivacaine. The ratio of triblock copolymer to diblock copolymer in this formulation is 3:2 to 1:19. Methods for producing these biodegradable drug compositions using organic solvents are also disclosed.

### BACKGROUND OF THE PRESENT INVENTION

Drug delivery systems such as diblock and triblock copolymers have been used to deliver a variety of drugs and are generally formulated to deliver specific drugs whether they are hydrophobic drugs or hydrophilic drugs. Depending on the drug solubility these drug formulations differ in polymer concentrations, types of polymers utilized, molecular weights of the polymers and solvents used in the formulations.

Also the type of environment in which the drug is delivered is an important consideration in formulating a drug delivery system. Thus, there exist drug delivery compositions that are prepared using temperature sensitive polymers, phase sensitive polymers, pH sensitive polymers and photosensitive polymers. See, for example, K. Al-Tahami and J. Singh "Smart Polymer Based Delivery Systems for Peptide and Proteins," Recent Patents on Drug Delivery & Formulation, 1: pages: 65-71 Bentham Science Publishers, LTD. 2007.

U.S. Patent No. 6,592,899 describes a PLA/PLGA oligomer combined with a block copolymer for enhancing the solubility of a hydrophobic drug into a hydrophilic environment. More specifically this polymer composition has a polyester oligomer having a molecular weight of between 400 and 10,000 daltons and a biodegradable AB-type, ABA-type or BAB type block copolymer. The hydrophobic A part is a polyester, while the hydrophilic B part is a polyethylene glycol having a molecular weight of between 2,400 and 4,999 daltons. This polymeric composition is soluble in an aqueous environment.

U.S. Patent 6, 541,033 describes a sustained release pharmaceutical composition based on thermosensitive, biodegradable hydrogels, consisting of a block copolymer of PLA or PLGA and PEG, for the sustained delivery of biologically active agents, such as leptin. The sustained release is for a period of a week or more and preferably up to one month.

Hydrogels containing triblock copolymers are described in U.S. Patent 6,350,812. These hydrogels retain water weight at least equal to the water weight of the copolymer and are soft hydrogels.

U.S. Patent 7,875,677 provides micelle-forming compositions comprising a hydrophobic drug, a biocompatible block copolymer, which has a hydrophilic protein comprising a polyethylene oxide and a hydrophobic portion having a polyester and a biocompatible water soluble polymer, wherein the water soluble polymer is present in a sufficient amount to make the micelle-forming composition injectable.

It is well known in the art that poorly water soluble or hydrophobic drugs often result in slow drug absorption leading to inadequate and variable bioavailability and gastrointestinal mucosal toxicity. Hence, formulating hydrophobic drugs is a challenge well known in this art.

None of the patents nor the literature cited above describes drug delivery compositions that are injectable, *in situ* forming and are biodegradable and turn into solid implants when injected into the body and deliver pharmaceutically hydrophobic active principles.

WO 02/45689 A1 discloses liquid compositions for the sustained delivery of a hydrophobic drug comprising: i) an amphiphilic diblock copolymer composed of a hydrophilic polyalkylene glycol block and a hydrophobic biodegradable polymer block, preferably methoxy-polyethylene glycol-polylactic acid, methoxy-polyethylene glycol-polycaprolactone or methoxy-polyethylene glycol-poly(lactide-co-glycolide), ii) a hydrophobic drug, iii) a biodegradable polymer and iv) a liquid polyethylene glycol. The compositions form an implant upon injection.

The biodegradable drug compositions of the present invention comprise triblock copolymers and diblock copolymers formulated in such a manner that the diblock copolymer serves as a reservoir while the triblock copolymer acts as a frame in the formulations and increases the lifespan of the diblock copolymer.

Furthermore, the biodegradable drug delivery compositions of the present invention can be long acting formulations, which reduce the initial burst release of the drug and modulate the release rate of the drug or hydrophobic drug over time. This phenomenon is illustrated in the flattening of the drug release curves.

### SUMMARY OF THE INVENTION

The invention is defined by the claims. Any subject-matter falling outside the scope of the claims is provided for information purposes only.

The present invention provides a biodegradable drug delivery composition comprising:
(a) a biodegradable triblock copolymer having the formula:

   poly(lactic acid)ᵥ-poly(ethylene glycol)_{w}-poly(lactic acid)ₓ

   wherein v, w and x are the number of repeat units ranging from 4 to 1090 or 6 to 1090 and v=x or v≠x;
(b) a biodegradable diblock copolymer having the formula:

   methoxy poly(ethylene glycol)_{y}-(polylactic acid)_{z}

   wherein y and z are the number of repeat units ranging from 7 to 371 or 3 to 237, wherein the ratio of the biodegradable triblock copolymer of (a) and the biodegradable diblock copolymer of (b) is 3:2 to 1:19 in said biodegradable drug composition; and
(c) at least one pharmaceutically active hydrophobic principle wherein said at least one pharmaceutically active hydrophobic principle is levonorgestrel, cyclosporine, progesterone, bupivacaine or medroxyprogesterone acetate.

In yet another aspect a biodegradable drug delivery composition is provided, which comprises:(a) a biodegradable triblock copolymer present in an amount of 3.0% to 45% (w%/w%) or 2.0% to 45% (w%/w%) or 1.2% to 30% (w%/w%)of the total composition having the formula:

PLAᵥ-PEG_{w}-PLAₓ

wherein v, w and x are the number of repeat units ranging from 4 to 1090 or 6 to 1090 and v=x or v≠x; (b) a biodegradable diblock copolymer present in an amount of 8.0% to 50% (w%/w%) or 1% to 28% (w%/w%) of the total composition having the formula:

mPEG_{y}-PLA_{z}

wherein y and z are the number of repeat units ranging from 7 to 371 or 3 to 237, wherein the ratio of the biodegradable triblock copolymer of (a) and the biodegradable diblock copolymer of (b) is 3:2 to 1:19 in said biodegradable drug composition, and wherein the PEG in the diblock is methoxy-capped; and (c) at least one pharmaceutically hydrophobic active principle one of which is medroxyprogesterone acetate, levonorgestrel, cyclosporine, progesterone or bupivacaine present in an amount of 10% to 40% (w%/w%) or 1% to 40% (w%/w%)of the total composition or the at least one pharmaceutically hydrophobic active principle is present in an amount of 1 to 200 mg/ml or 0.1 to 200 mg/ml.

The biodegradable drug delivery compositions of the invention can have a lactic acid to ethylene oxide molar ratio in the composition of between 0.5 to 3.5 or from 0.5 to 2.5 or 0.5 to 22.3 for the triblock copolymer and between 2 to 6 or 0.8 to 13 for the diblock copolymer.

In another aspect the biodegradable drug delivery compositions of the invention can have a lactic acid to ethylene oxide molar ratio in the composition of between 0.5 to 22.3 for the triblock copolymer and between 0.8 to 13 for the diblock copolymer.

In yet another aspect the biodegradable drug delivery compositions of the invention can have a lactic acid to ethylene oxide molar ratio in the composition of between 0.5 to 2.5 for the triblock copolymer and between 3 to 5 for the diblock copolymer.

In one aspect the biodegradable drug delivery composition is an injectable liquid that when it is inserted into the body of an animal or plant becomes a hardened implant.

In yet another aspect the biodegradable delivery drug composition can be used as a spatial formulation such that it can be applied onto or inside the body of an animal or plant. For example, it can be dispensed during surgery to treat a wound or inside a plant to treat a virus.

In another aspect the biodegradable drug composition is prepared as small solid particles, which are placed directly on the injured site of the body of an animal or plant.

In another aspect the biodegradable drug composition is in the form of a rod implant.

The present invention also provides a method for preparing a biodegradable drug delivery composition, said method comprising:
(i) dissolving in an organic solvent
   (a) a biodegradable ABA type block copolymer having the formula:

      poly(lactic acid)ᵥ-poly(ethylene glycol)_{w}-poly(lactic acid)ₓ

      wherein v, w and x are the number of repeat units ranging from 6 to 1090 or 4 to 1090 and v=x or v≠x; and
   (b) a biodegradable diblock copolymer having the formula:

      methoxy poly(ethylene glycol)_{y}-(polylactic acid)_{z}

      wherein y and z are the number of repeat units ranging from 7 to 371 or 3 to 237, in a ratio of 3:2 to 1:19 (a):(b) to form a polymer mixture; and
(ii) adding at least one pharmaceutically active hydrophobic principle to said polymer mixture, wherein said at least one pharmaceutically active hydrophobic principle is levonorgestrel, cyclosporine, progesterone, bupivacaine or medroxyprogesterone acetate.

In the above method, the organic solvent can be present in an amount of 40% to 74% (w%/w%) or 30% to 70% (w%/w%) or 26% to 90% (w%/w%) of the total composition. Mixtures of solvents can also be used. In an embodiment, the method further comprises (iii) evaporating said solvent.

Other aspects and embodiments are set forth below, or will readily arise from the following description of the preferred embodiments.

### BRIEF DESCRIPTION OF THE DRAWINGS

Only the compositions comprising the claimed combination of triblock copolymer, diblock copolymer and pharmaceutically active hydrophobic principle are according to the invention.

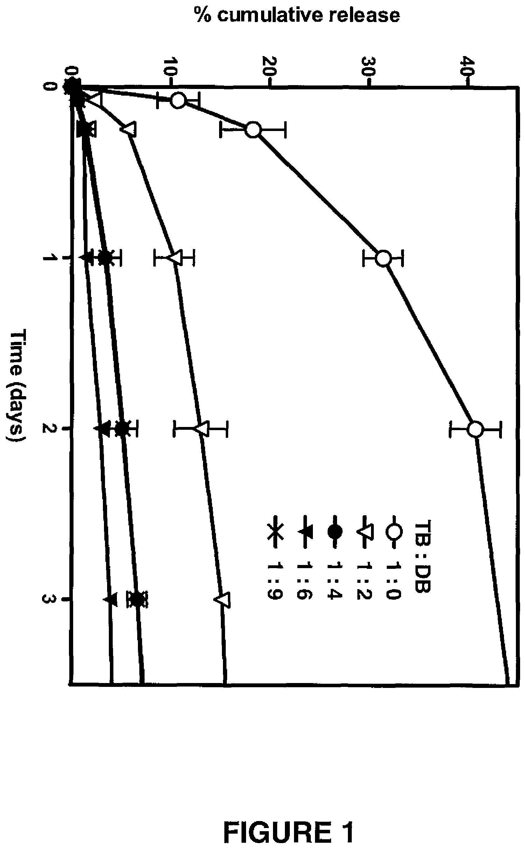

Fig. 1 is a graph showing the *in vitro* release rate of the drug from formulations based on 40% P6R1(TB):dP2R4(DB) in ratios of 1:0 (-∘-), 1:2 (-Δ-), 1:4 (-•-), 1:6 (-▼-) and 1:9 (-*-) over time in days. This graph shows that formulations based on TB:DB are sustaining the release for more than 30 days.

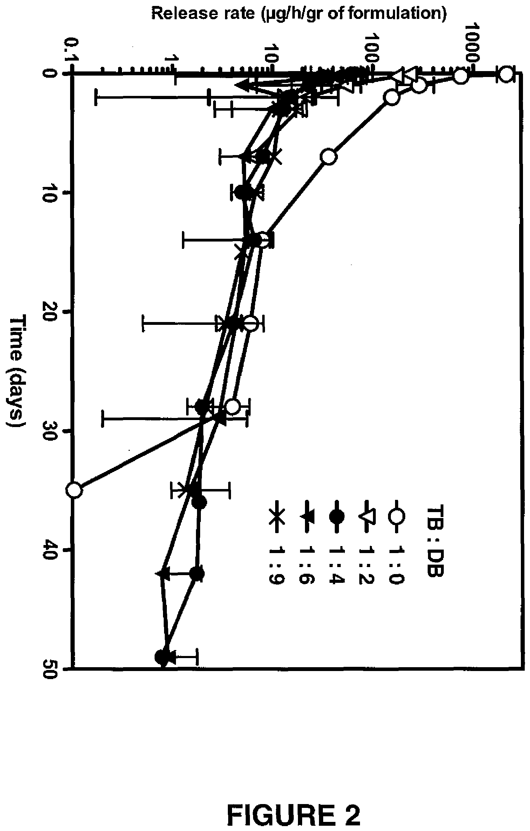

Fig. 2 is a graph showing the *in vitro* cumulative percent release curve from candidate formulations of Figure 1 over time (days). This graph illustrates that the initial burst is reduced and the drug release curve is flattened in the combination of triblock copolymer and diblock copolymer compositions compared to the triblock copolymer composition alone. It should be noted that the 1:9 curve is overlapping the 1:4 curve.
Fig. 3 is a graph showing the injectability of formulations based on 40% P6R1 (TB);dP2R4(DB) in various ratios ranging from 1:0 triblock copolymer to diblock copolymer to 0:1 triblock copolymer to diblock copolymer. This graph illustrates that all formulations are injectable using a classical injection device.
Fig. 4 is a graph showing the *in vitro* cumulative percentage release curve from candidate formulations over time (days) of various compositions of the invention. The compositions described as numbers 177, 246, 224, 225 and 250 are described in Table1.
Fig. 5 is a graph showing the *in vitro* release rate from candidate formulations in micrograms per hour per gram of formulation (µg/h/gr of formulation) The compositions described as numbers 177, 246, 224, 225 and 250 are described in Table1.
Fig. 6 is a graph showing the M53 plasma concentration in nanograms per milliliter (ng/ml) over time in days. Day zero is the day that the composition was administered subcutaneously. The compositions indicated as numbers 177, 246, 224, 225 and 250 are described in Table1.
Fig. 7 is a graph showing the *in vitro* cumulative percent release of acetaminophen over time (days) from formulations based on triblock copolymer P0.2R5 (4 units of ethylene oxide and 24 units of lactic acid) mixed with various diblock copolymers (see Table 2 for details).
Fig. 8 is a graph showing the *in vitro* cumulative percent release of acetaminophen over time (days) from formulations based on triblock copolymer P0.2R14 (4 units of ethylene oxide and 58 units of lactic acid) mixed with various diblock copolymers (see Table 2 for details).
Fig. 9 is a graph showing the *in vitro* cumulative percent release of acetaminophen over time (days) from formulations based on triblock copolymer P0.2R22 (4 units of ethylene oxide and 89 units of lactic acid) mixed with various diblock copolymers (see Table 2 for details).
Fig. 10 is a graph showing the *in vitro* cumulative percent release of acetaminophen over time (days) from formulations based on triblock copolymer P0.4R4 (9 units of ethylene oxide and 41 units of lactic acid) mixed with various diblock copolymers (see Table 2 for details).
Fig. 11 is a graph showing the *in vitro* cumulative percent release of acetaminophen over time (days) from formulations based on triblock copolymer P0.4R7 (9 units of ethylene oxide and 67 units of lactic acid) mixed with various diblock copolymers (see Table 2 for details).
Fig. 12 is a graph showing the *in vitro* cumulative percent release of acetaminophen over time (days) from formulations based on triblock copolymer P0.6R1 (13 units of ethylene oxide and 26 units of lactic acid) mixed with various diblock copolymers (see Table 2 for details).
Fig. 13 is a graph showing the *in vitro* cumulative percent release of acetaminophen over time (days) from formulations based on triblock copolymer P0.6R3 (13 units of ethylene oxide and 40 units of lactic acid) mixed with various diblock copolymers (see Table 2 for details).
Fig. 14 is a graph showing the *in vitro* cumulative percent release of acetaminophen over time (days) from formulations based on triblock copolymer P0.6R4 (13 units of ethylene oxide and 55 units of lactic acid) mixed with various diblock copolymers (see Table 2 for details).
Fig. 15 is a graph showing the *in vitro* cumulative percent release of acetaminophen over time (days) from formulations based on triblock copolymer P1R2 (22 units of ethylene oxide and 47 units of lactic acid) mixed with various diblock copolymers (see Table 2 for details).
Fig. 16 is a graph showing the *in vitro* cumulative percent release of acetaminophen over time (days) from formulations based on triblock copolymer P1R3 (22 units of ethylene oxide and 68 units of lactic acid) mixed with various diblock copolymers (see Table 2 for details).
Fig. 17 is a graph showing the *in vitro* cumulative percent release of acetaminophen over time (days) from formulations based on triblock copolymer P1R4 (22 units of ethylene oxide and 88 units of lactic acid) mixed with various diblock copolymers (see Table 2 for details).
Fig. 18 is a graph showing the *in vitro* cumulative percent release of acetaminophen over time (days) from formulations based on triblock copolymer P2R2 (45 units of ethylene oxide and 88 units of lactic acid) mixed with various diblock copolymers (see Table 2 for details).
Fig. 19 is a graph showing the *in vitro* cumulative percent release of acetaminophen over time (days) from formulations based on triblock copolymer P2R3 (45 units of ethylene oxide and 157 units of lactic acid) mixed with various diblock copolymers (see Table 2 for details).
Fig. 20 is a graph showing the *in vitro* cumulative percent release of acetaminophen over time (days) from formulations based on triblock copolymer P2R5 (45 units of ethylene oxide and 216 units of lactic acid) mixed with various diblock copolymers (see Table 2 for details).
Fig. 21 is a graph showing the *in vitro* cumulative percent release of acetaminophen over time (days) from formulations based on triblock copolymer P3R1 (68 units of ethylene oxide and 66 units of lactic acid) mixed with various diblock copolymers (see Table 2 for details).
Fig. 22 is a graph showing the *in vitro* cumulative percent release of acetaminophen over time (days) from formulations based on triblock copolymer P3R2 (68 units of ethylene oxide and 154 units of lactic acid) mixed with various diblock copolymers (see Table 2 for details).
Fig. 23 is a graph showing the *in vitro* cumulative percent release of acetaminophen over time (days) from formulations based on triblock copolymer P3R3 (68 units of ethylene oxide and 218 units of lactic acid) mixed with various diblock copolymers (see Table 2 for details).
Fig. 24 is a graph showing the *in vitro* cumulative percent release of acetaminophen over time (days) from formulations based on triblock copolymer P6R0.9 (136 units of ethylene oxide and 125 units of lactic acid) mixed with various diblock copolymers (see Table 2 for details).
Fig. 25 is a graph showing the *in vitro* cumulative percent release of acetaminophen over time (days) from formulations based on triblock copolymer P6R1.6 (136 units of ethylene oxide and 218 units of lactic acid) mixed with various diblock copolymers (see Table 2 for details).
Fig. 26 is a graph showing the *in vitro* cumulative percent release of acetaminophen over time (days) from formulations based on triblock copolymer P6R2 (136 units of ethylene oxide and 272 units of lactic acid) mixed with various diblock copolymers (see Table 2 for details).

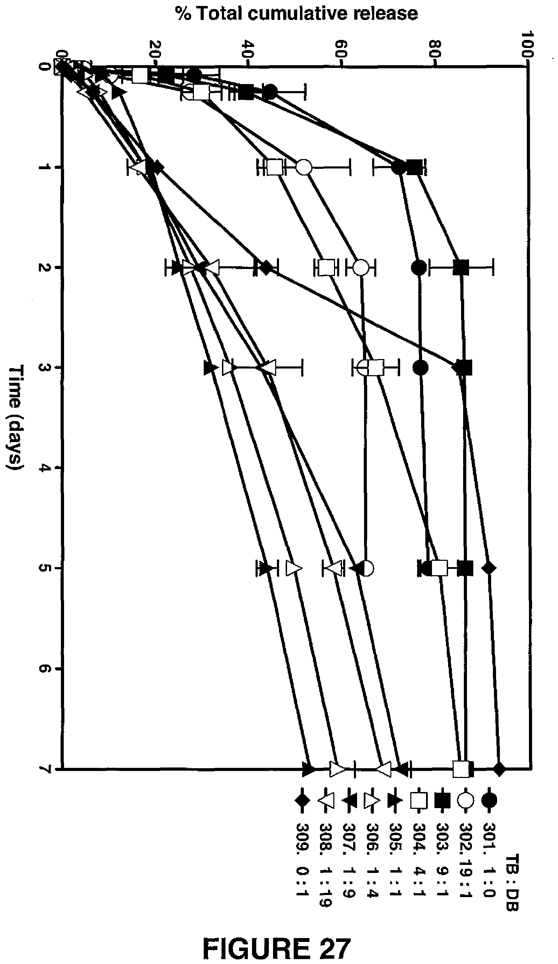

Fig. 27 is a graph showing the *in vitro* cumulative percent release of acetaminophen over time (days) from formulations based on triblock copolymer P2R4 (45 units of ethylene oxide and 157 units of lactic acid) mixed with diblock copolymer dP0.4R6 (7 units of ethylene oxide and 42 units of lactic acid) at different ratios (see Table 2 for details).

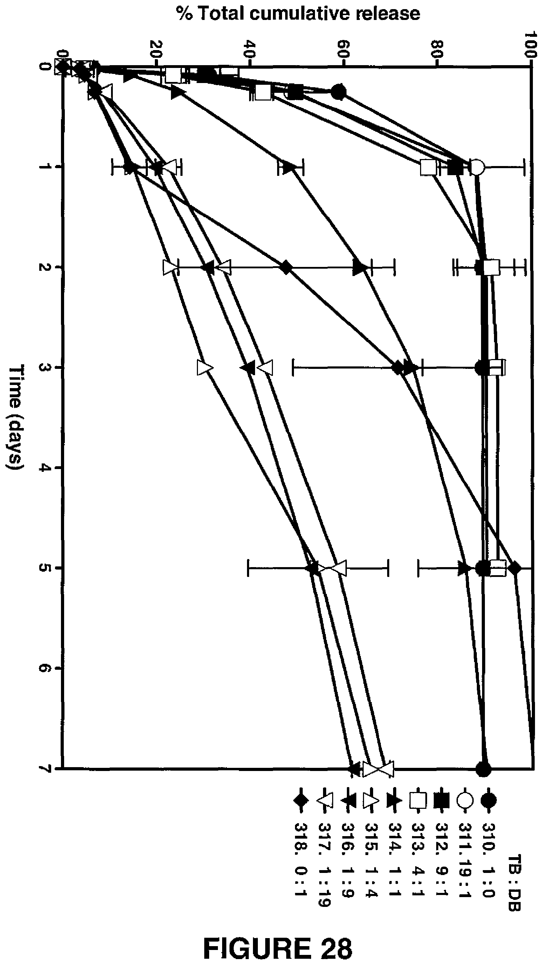

Fig. 28 is a graph showing the *in vitro* cumulative percent release of acetaminophen over time (days) from formulations based on triblock copolymer P2R4 (45 units of ethylene oxide and 157 units of lactic acid) mixed with diblock copolymer dP0.6R5 (12 units of ethylene oxide and 54 units of lactic acid) at different ratios (see Table 2 for details).

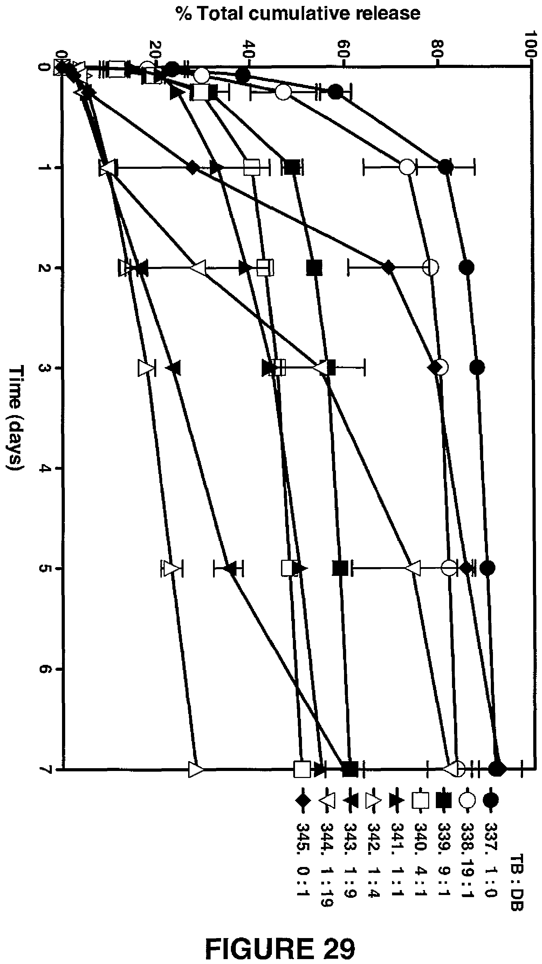

Fig. 29 is a graph showing the *in vitro* cumulative percent release of acetaminophen over time (days) from formulations based on triblock copolymer P2R5 (45 units of ethylene oxide and 216 units of lactic acid) mixed with diblock copolymer dP0.2R13 (3 units of ethylene oxide and 39 units of lactic acid) at different ratios (see Table 2 for details).
Fig. 30 is a graph showing the *in vitro* release rate of buprenorphine over time (days) from formulations n°33 (10%BN/ 8%P2R2/ 32%dP0.4R10), n°47 (10%BN/ 8%P2R2/ 32%dP1R3) and n°58 (10%BN/ 10%P0.4R8/ 40%dP1R2).
Fig. 31 is a graph showing the plasma concentration of buprenorphine over time (days) in rats injected with formulations n°33 (10%BN/ 8%P2R2/ 32%dP0.4R10), n°47 (10%BN/ 8%P2R2/ 32%dP1R3) and n°58 (10%BN/ 10%P0.4R8/ 40%dP1R2).
Fig. 32 is a graph showing the *in vitro* release rate of risperidone over time (days) from formulations based on triblock polymer P2R5 (45 units of ethylene oxide and 216 units of lactic acid) mixed with diblock polymer dP0.2R13 (3 units of ethylene oxide and 39 units of lactic acid) at different ratios (see Table 2 for details).
Fig. 33 is a graph showing the plasma concentration of risperidone and 9-OH risperidone over time (days) in rats injected with formulations n°10 (5%RSP/ 16%P2R2/ 24%dP2R2/ DMSO), n°29 (10%RSP/ 24%P1R4/ 16%dP0.4R5/ DMSO) and n°31 (10%RSP/ 18%P2R4/ 12%dP0.4R5/ DMSO).
Fig. 34 is a graph showing the plasma concentration of ivermectin over time (days) in dogs injected with formulations n°7 (5%IVM/ 15%P3R3/ 25%dP0.4R5/ DMSO), n°9 (5%IVM/ 15%P2R4/ 25%dP2R3/ DMSO) and n°10 (5%IVM/ 15%P2R5/ 25%dP2R21 DMSO).
Fig. 35 is a graph showing the *in vitro* release rate of medroxyprogesterone acetate (MPA) from candidate formulations in milligrams per gram of formulation per day (mg MPA/gr of formulation/day). The formulations described as numbers 33, 34 and 49 are described in Table 6. *In vitro* release obtained with Depo-SubQ Provera is shown as a control.

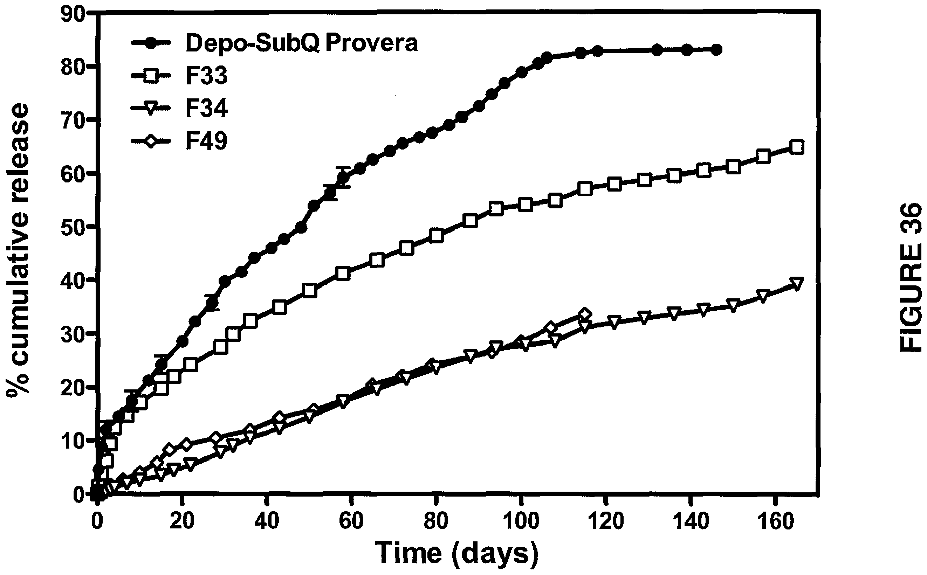

Fig. 36 is a graph showing the *in vitro* cumulative percent release of medroxyprogesterone acetate over time (days) from formulations 33, 34 and 49 described in Table 6. *In vitro* release obtained with Depo-SubQ Provera is shown as a control.

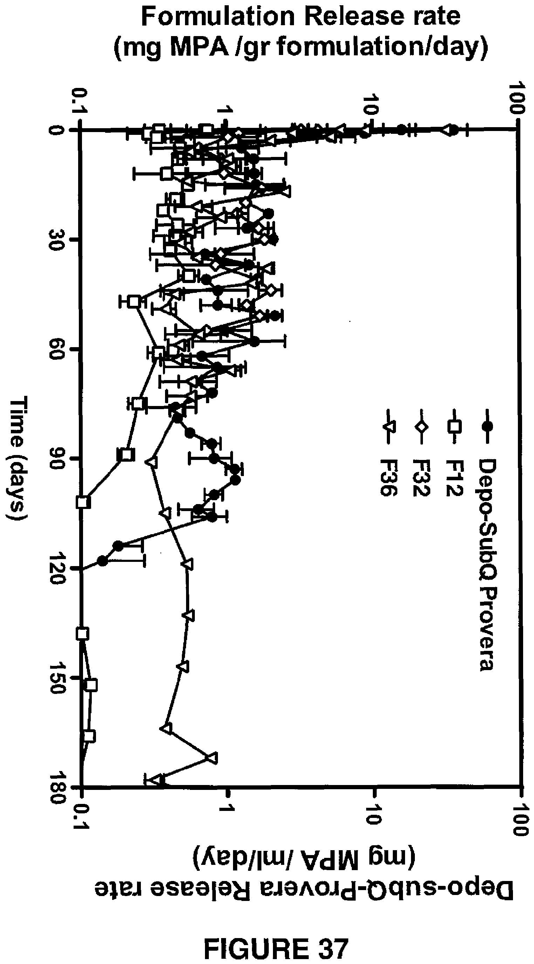

Fig. 37 is a graph showing the *in vitro* release rate of medroxyprogesterone acetate from candidate formulations in milligrams per gram of formulation per day (mg/gr of formulation/day). The formulations described as numbers 12, 32 and 36 are described in Table 6. In vitro release obtained with Depo-SubQ Provera is shown as a control.

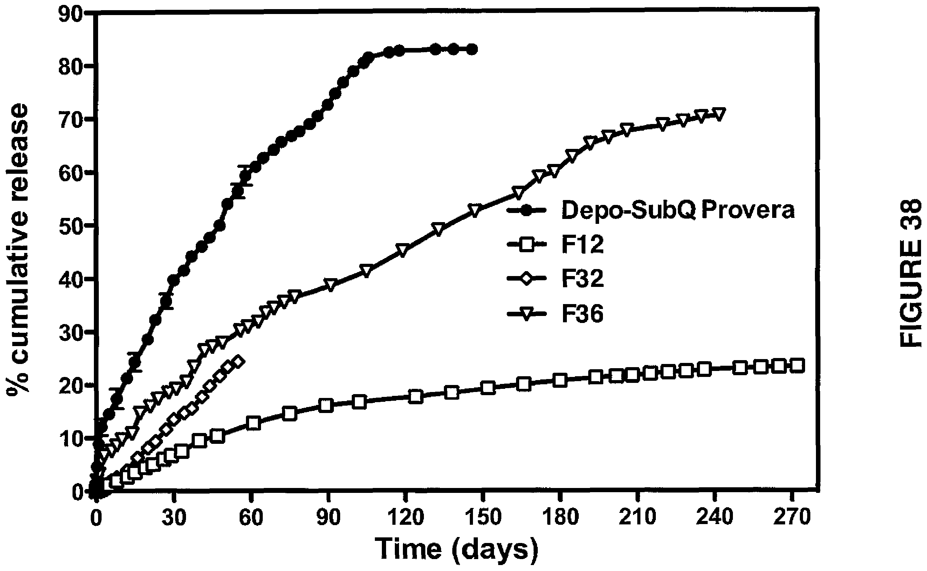

Fig. 38 is a graph showing the *in vitro* cumulative percent release of medroxyprogesterone acetate from formulations 12, 32 and 36 per days described in Table 6. *In vitro* release obtained with Depo-SubQ Provera is shown as a control.

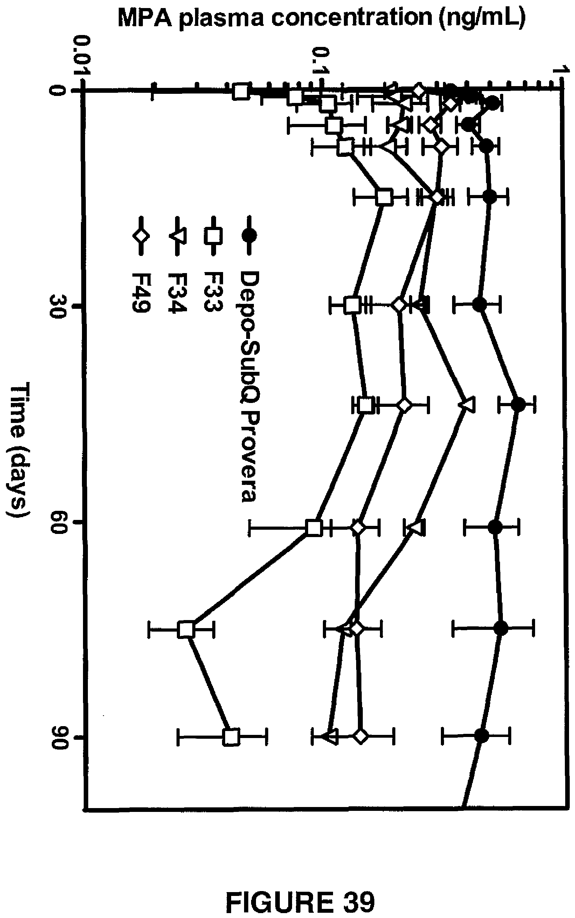

Fig. 39 is a graph showing the plasma concentration of medroxyprogesterone acetate (MPA) in female dogs over time (days) injected with formulations 33, 34 and 49 described in Table 6. Each dog received a single 3 mg/kg dose of MPA.

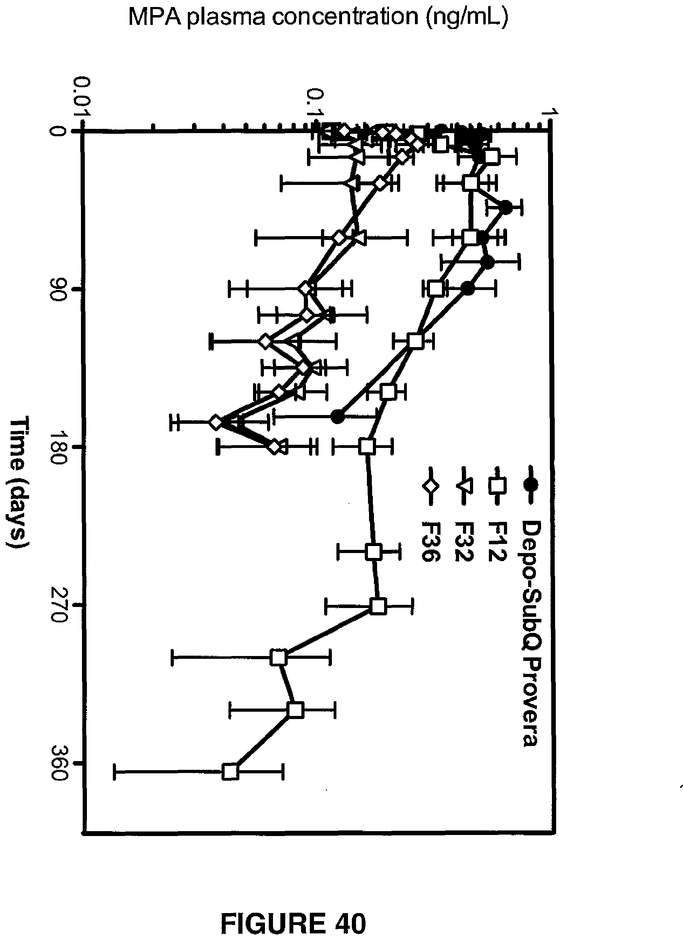

Fig. 40 is a graph showing the plasma concentration of medroxyprogesterone acetate (MPA) in dogs over time (days) injected with formulations 12, 32 and 36 described in Table 6. For formulations 32, 36 and the control group (receiving Depo-subQ-Provera), each dog received a single 3 mg/kg MPA dose. The group receiving formulation 12 was dosed at 6 mg/kg MPA.

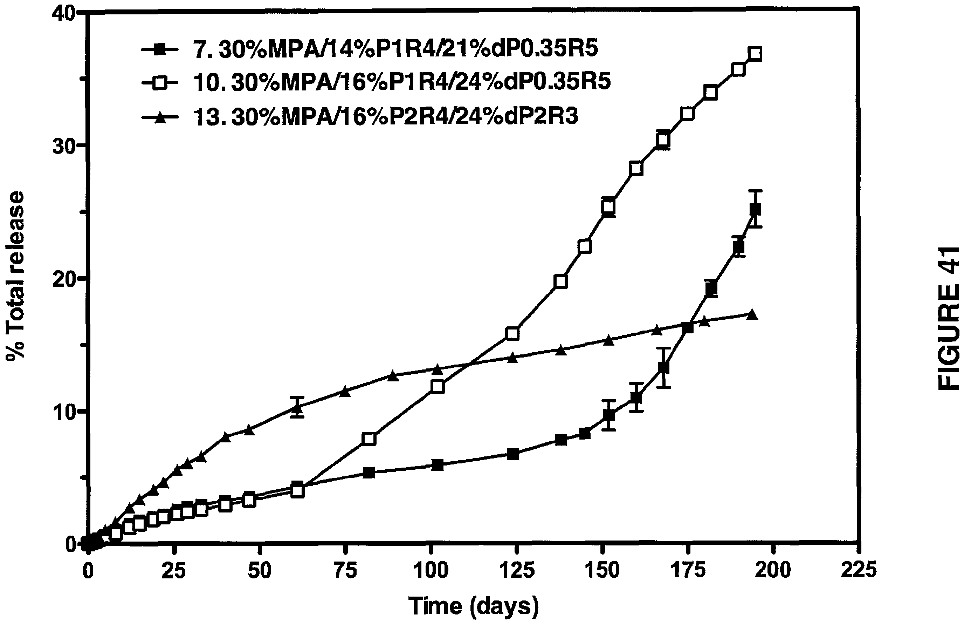

Fig. 41 is a graph showing the *in vitro* percent total release of medroxyprogesterone acetate (MPA) overtime (days) from formulations 7, 10 and 13 described in Table 6.

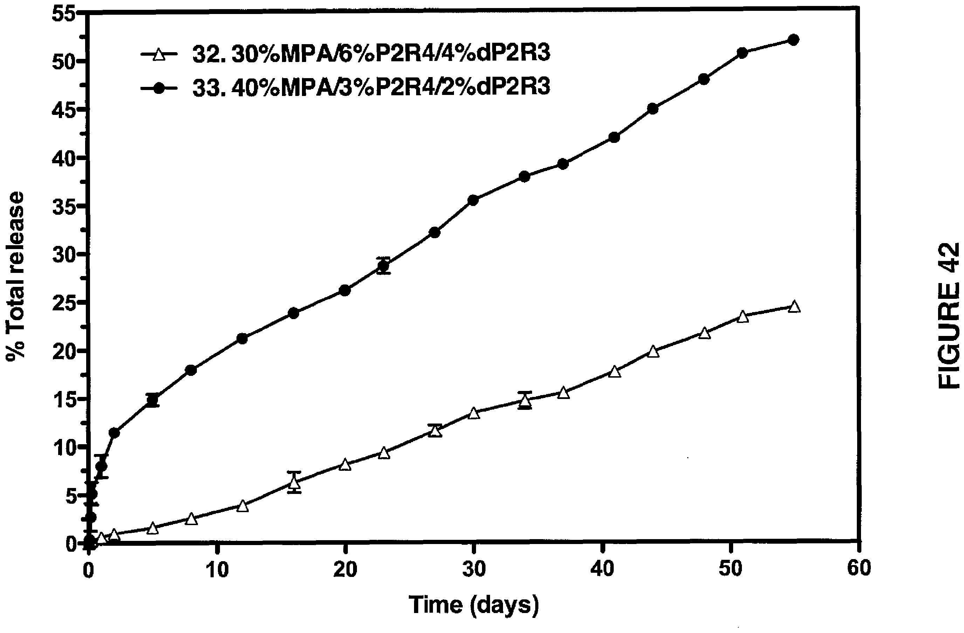

Fig. 42 is a graph showing the *in vitro* percent total release of medroxyprogesterone acetate (MPA) over time (days) from formulations 32 and 33 described in Table 6.

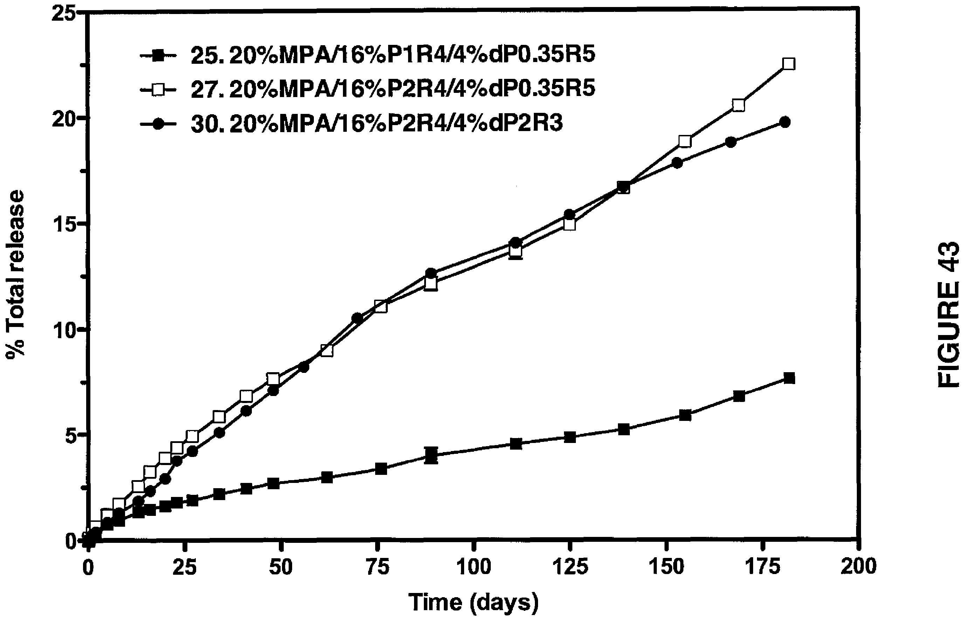

Fig 43 is a graph showing the *in vitro* percent total release of medroxyprogesterone acetate (MPA) over time (days) from formulations 25, 27 and 30 described in Table 6.

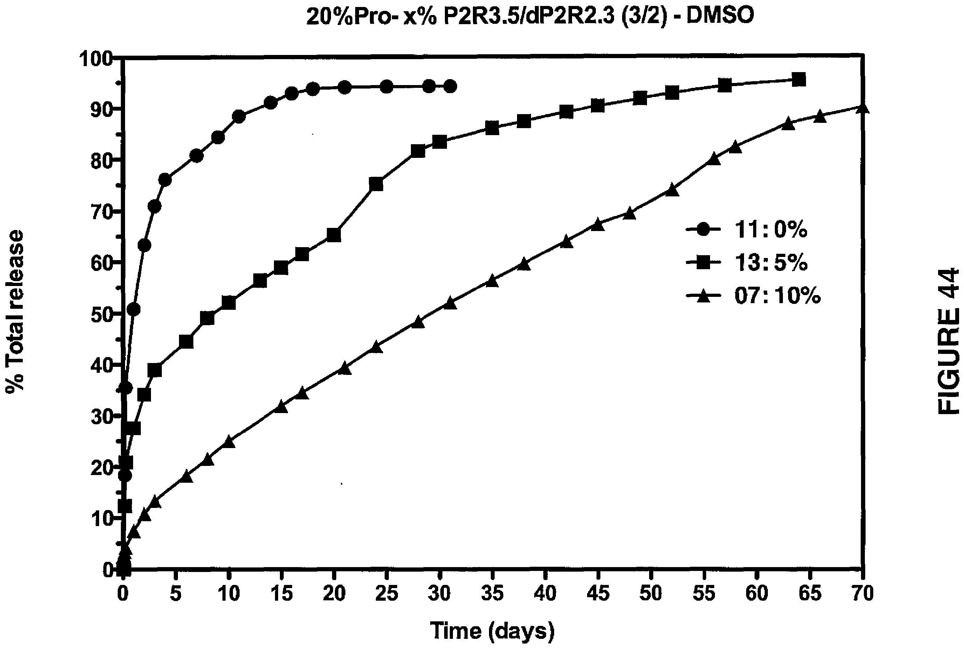

Fig 44 is a graph showing the *in vitro* percent total release of progesterone (Pro) over time (days) from formulations 11, 13 and 7 described in Table 7.

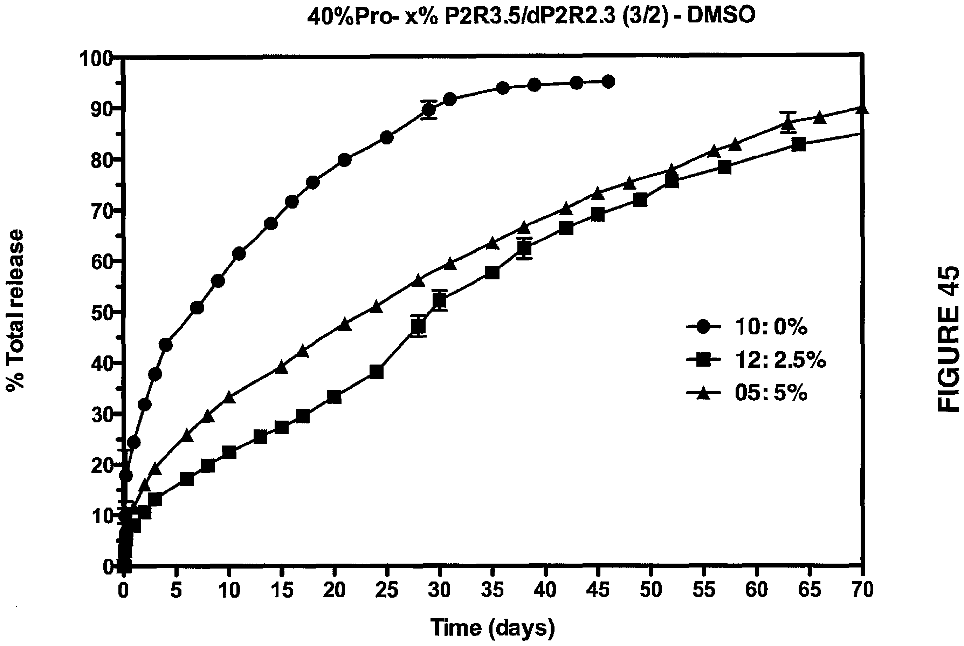

Fig. 45 is a graph showing the *in vitro* percent total release of progesterone (Pro) over time (days) from formulations 10, 12 and 5 described in Table 7 .

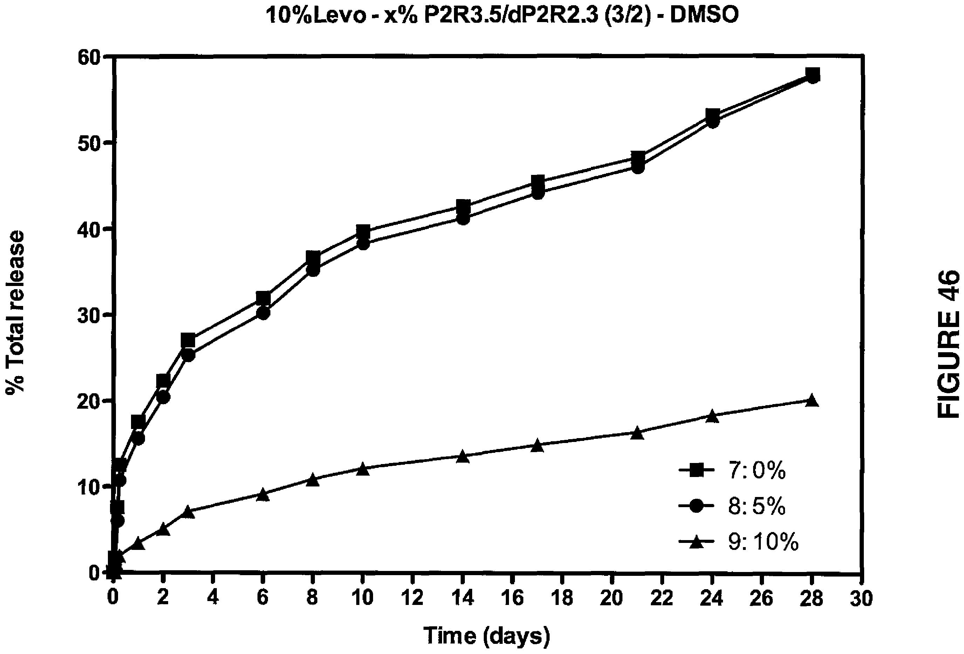

Fig. 46 is a graph showing the *in vitro* percent total release of Levonorgestrel (Levo) over time (days) from formulations 7, 8 and 9 described in Table 8.

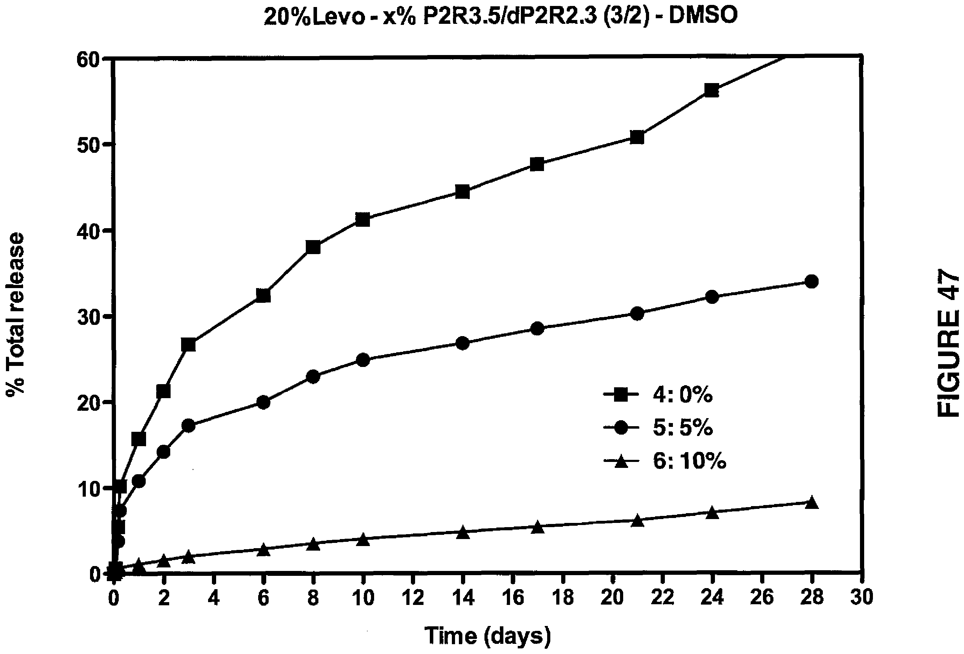

Fig 47 is a graph showing the *in vitro* percent total release of Levonorgestrel (Levo) over time (days) from formulations 4, 5 and 6 described in Table 8.

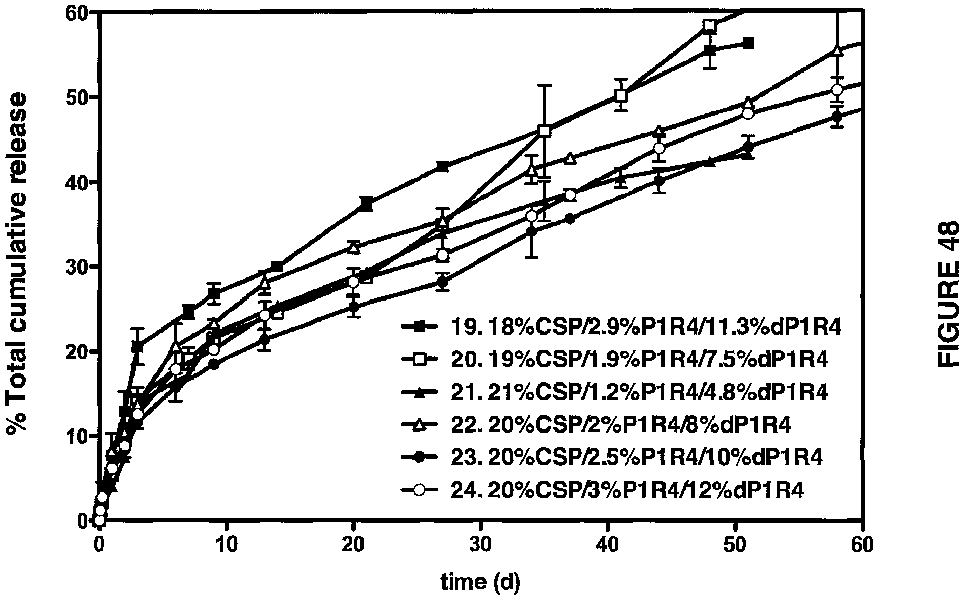

Fig. 48 is a graph showing the *in vitro* percent total release of cyclosporine (CSP) over time (days) from formulations 19, 20, 21, 22, 23 and 24 described in Table 9.

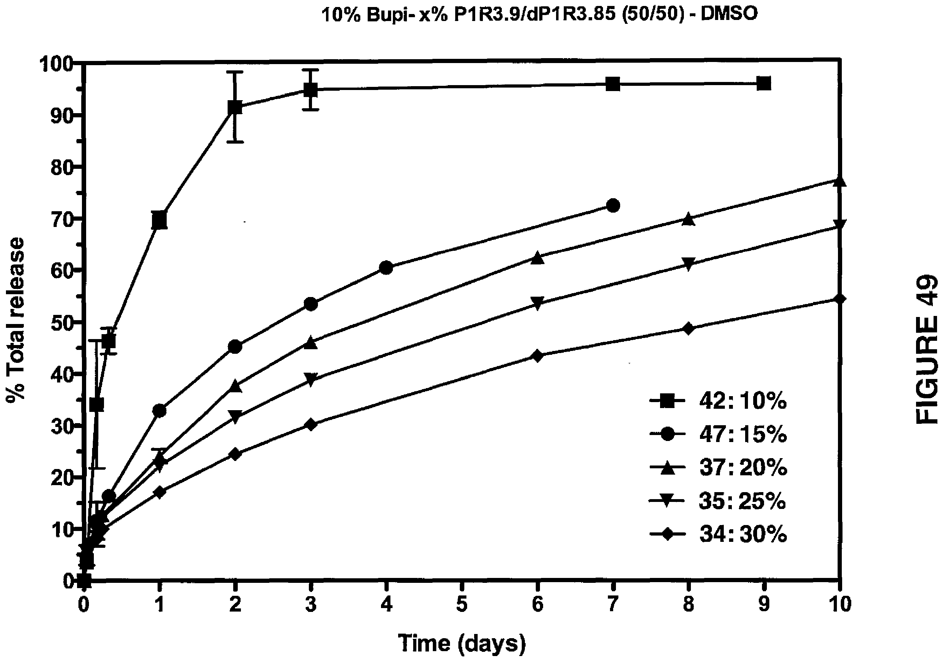

Fig. 49 is a graph showing the *in vitro* percent total release of Bupivacaine base (Bupi) over time (days) from formulations based on formulations 42, 47, 37, 35 and 34 described in Table 10.

### DESCRIPTION OF THE PREFERRED EMBODIMENTS

As used herein the term "biodegradable" means that the triblock and diblock copolymers will after a period of time erode or degrade *in vivo* to form smaller nontoxic components.

The term "parenteral administration" encompasses intramuscular, intraperitoneal, intra-abdominal, subcutaneous, intravenous and intraarterial. It also encompasses intradermal, intracavernous, intravitreal, intracerebral, intrathecal, epidural and intraosseous administration.

The term "animals" encompasses all members of the Kingdom Animalia.

As used herein the term "plant" encompasses all members of the Plant Kingdom.

"Active principle" means a drug or medicine for treating various medical illnesses. Thus active principles, drugs and medicines are used interchangeably. The term drug or active principle as used herein includes without limitation physiologically or pharmacologically active substances that act locally or systemically in the body of an animal or plant. The biodegradable drug delivery composition of the invention comprises at least one pharmaceutically active hydrophobic principle, which is levonorgestrel, cyclosporine, progesterone, bupivacaine or medroxyprogesterone acetate.

As used herein "disease" means any disorder in a human, animal or plant caused by infection, diet, or by faulty functioning of a process.

The term "implant" means that the drug delivery compositions are injectable, are *in situ* forming and are biodegradable and turn into solid implants when injected into the body. Thus, that the formulations that are synthesized are liquids such that they can be easily injected through a syringe without excessive force.

The term "spatial formulations" encompass any formulations that can be applied on or into the animal or plant body and do not necessarily have to be administered through a syringe.

As used herein "repeat units" are the fundamental recurring units of a polymer.

By "end-capped polyethylene glycol" (cPEG) refers to PEG's in which one terminal hydroxyl group is reacted and includes alkoxy-capped PEG's, urethane-capped PEG's, ester-capped PEG's and like compounds. The capping group is a chemical group which does not contain a chemical function susceptible to react with cyclic esters like lactide, glycolactide, caprolactone and the like or other esters and mixtures thereof. The reaction of an end-capped PEG polymer with lactide generates a diblock cPEG-PLA copolymer. The diblock copolymer of the present invention comprises a methoxy-capped poly(ethylene glycol) block according to the claims.

As used herein polyethylene glycol, as abbreviated PEG throughout the application, is sometimes referred to as poly(ethylene oxide) or poly(oxyethylene) and the terms are used interchangeably in the present invention.

The abbreviation of "PLA" refers to poly(lactic acid).

The abbreviation of "PLGA" refers to poly(lactic-co-glycolic acid).

The abbreviation "T" or "TB" refers to a triblock copolymer(s), while the abbreviation "D" or "DB" refers to a diblock copolymer(s).

The term "diblock" as used herein refers to the diblock copolymer as claimed. "mPEG" refers to methoxy polyethylene glycol.

The term "triblock" refers to the triblock copolymer as claimed.

As used herein the term "partial suspension" means that the pharmaceutically active principle is in a partly soluble and partly solid form.

As used herein "hydrophobic" when referring to the pharmaceutically active principles means drugs that have poor solubility in aqueous solutions. The International Union of Pure and Applied Chemistry (IUPAC) defines solubility as "the analytical composition of a saturated solution expressed as a proportion of a designated solute in a designated solvent." A substance is said to be soluble if more than 0.1 g of that substance dissolves in 100 ml of distilled water at 250°C. If less than 0.1 g dissolves in 100 ml of distilled water at 250°C the substance is sparingly soluble or insoluble at a particular temperature.

The LA/EO ratio refers to the molar ratio of lactic acid units to ethylene oxide units that is present in the biodegradable drug delivery composition. It is determined experimentally by NMR. The LA/EO molar ratio of the combined triblock copolymer can range from 0.5 to 3.5. In another aspect the LA/EO molar ratio in the triblock can range from 0.5 to 2.5 in the biodegradable drug delivery composition described herein. In yet another aspect the LA/EO ratio in the triblock can range from 0.5 to 22.3.

The LA/EO ratio in the diblock can range from 2 to 6. In another aspect the LA/EO ratio in the diblock can range from 3 to 5 in the biodegradable drug delivery composition. In another aspect the LA/EO ratio in the diblock can range from 0.8 to 13.

The degree of polymerization or DP is the number of repeat units in an average polymer chain at time *t* in a polymerization reaction. For example, the degree of polymerization for PEG is about 45 to 170 or it can be 4 to 273 or 3 to 45, while for PLA it can range from about 84 to 327 or it can be 24 to 682 or 7 to 327 or 39.9 to 170.

The present invention thus relates to a biodegradable drug composition as claimed comprising a triblock copolymer and a diblock copolymer. The biodegradable triblock copolymer has the formula: Aᵥ-B_{w}-Aₓ, wherein A is a polyester and B is polyethylene glycol and v, w and x are the number of repeat units ranging from 4 to 1090 or from 6 to 1090 and v=x or v≠x. w is the degree of polymerization (number of repeat units) for PEG. The degree of polymerization for DP-PEG is calculated by dividing the PEG molecular weight by the EO unit molecular weight (44 Da). v + x equals the degree of polymerization (number of repeat units) for PLA. DP-PLA is calculated by multiplying DP-PEG by the LA/EO ratio.

However the number of repeat units of v, w and x in the triblock composition may vary due to the targeted time of release of the active principle and the type of active principle itself. Therefore the number of repeat units in the triblock of v, w and x can range from 4 to 1090 or from 6 to 1090 or from 8 to 1090, from 10 to 850, from 20 to 700, from 30 to 650 and v=x or v≠x. For instance, w can be 273, while x + y can be 682 and v=x or v≠x or w can be 136 and x + y can be 273 and v=x or v≠x or w can be 45.5 and x + y can be 546 or w can be 273 and x + y can be 136.

The size of the PEG in the triblock can range from 194 Da to 12,000 Da.

The polyester in the triblock is polylactic acid (PLA).

The triblock copolymer is then combined with a biodegradable diblock copolymer as claimed. This combination has a ratio of triblock copolymer to diblock copolymer as claimed.

Examples of end-capped polyethylene glycols include alkoxy capped PEG's such as methoxyPEG or ethoxyPEG, urethane-capped PEG's, ester-capped PEG's, amine-capped PEG's and amide-capped PEG's. This list of end-capped PEG's is not exhaustive and a person skilled in the art would recognize additional end-capped PEG's, which are not listed. The diblock copolymer of the present invention comprises a methoxy-capped poly(ethylene glycol) block according to the claims.

The number of repeat units (degree of polymerization (DP)) of y and z in the diblock composition may also vary. Thus, y can, for example, range from 7 to 43 or 3 to 45 and z can range from 32 to 123 or 7 to 327 or 39.9 to 170. For example, y can be 25 and z can be 123, y can be 34.5 and z can be 123 or y can be 45 and z can be 32.The degree of polymerization for DP-PEG is calculated by dividing the PEG molecular weight of the capped PEG by the EO unit molecular weight (44 Da). The DP-PLA is calculated by multiplying DP-PEG by the LA/EO ratio.

The polyester in the diblock is polylactic acid (PLA).

The ratio of the biodegradable triblock copolymer of (a) and the biodegradable diblock copolymer of (b) is 3:2 to 1:19 in said biodegradable drug composition. In one embodiment the ratio of the biodegradable triblock copolymer of and the biodegradable diblock copolymer is selected from the group of 1:3, 1:4, 1:5, 1:6, 1:7 and 1:8 or 1:1, 1:2, 1:3, 1:4, 1:5, 1:6, 1:7, 1:8, 1:9, 1:10, 1:11, 1:12, 1:13, 1:14, 1:15, 1:16, 1:17, 1:18 and 1:19. It can also be 3:2 or 2:3. In another aspect the ratio of the triblock to the diblock is 1:6.

The length of the polyester chain is defined by the lactic acid to ethylene oxide molar ratio, which is between 0.5 to 3.5 or 0.5 to 2.5 or 0.5 to 22.3 for the triblock copolymer and 3 to 5 or 2 to 6 or 0.8 to 13 for the diblock copolymer.

The mass of the end-capped polyethylene glycol can range from 164 Da to 2,000 Da or from 100 Da to 2 kDa. It can range in the lower 100 to 300 Da range or in the 1 kDa to 2 kDa range. The diblock copolymer of the present invention comprises a methoxy-capped poly(ethylene glycol) block according to the claims.

The size of the polyethylene glycol chain ranges from 200 Da to 12 kDa in the biodegradable drug delivery composition or it can range from 400 Da to 12 kDa or 194 Da to 12 kDA.

The polymers are present in an amount of 20% to 50% (w%/w%) of the total weight of the composition. In another aspect the total weight of the polymers present in the biodegradable drug composition is 30% to 50% (w%/w%) of the total weight of the composition. In yet another aspect the polymers are present in the biodegradable drug composition at 40% to 50% (w%/w%) of the total weight of the composition. In another aspect the polymers are present in an amount of 5% to 40% (w%/w%) of the total composition or 5% to 50% (w%/w%) of the total composition. In yet another aspect the polymers are present in the biodegradable drug composition at 2.5% to 40% (w%/w%) or 2.5% to 50% (w%/w%) of the total weight of the composition.

Thus, the triblock copolymer is present in an amount of 3.0% to 45% (w%/w%) of the total weight of the composition. In another aspect the triblock copolymer is present in an amount of 6% to 10% (w%/w%) of the total weight of the composition. In yet another aspect the triblock copolymer is present in an amount of 20% to 40% (w%/w%) of the total weight of the composition. In yet another aspect the triblock copolymer is present in an amount of 1.2 % to 30% (w%/w%) of the total weight of the composition or 1.2% to 45% (w%/w%) of the total weight of the composition.

In another embodiment the triblock copolymer is present in 3.3% to 4.0% (w%/w%) or 3.5% (w%) or 4.0% (w%) or 1.9% to 4.0%(w%/w%) of the total weight of the composition.

Likewise the diblock copolymer can be present in the biodegradable drug composition in an amount of 8% to 50% (w%/w%) of the total weight of the composition. In another aspect the diblock copolymer is present in an amount of 10% to 20% (w%/w%) of the total weight of the composition. In yet another aspect the diblock copolymer is present in an amount of 20% to 40% (w%/w%) of the total weight of the composition. In yet another aspect the diblock copolymer is present in an amount of 1% to 28% (w%/w%) of the total weight of the composition or 1% to 50% (w%/w%) of the total weight of composition.

In yet another embodiment the diblock is present in an amount of 2.48% to 5.02% (w%/w%) or 2.3% to 5.4% (w%/w%) or 2.5% to 5.1% (w%/w%) or 2.3% (w%) or 2.3% to 5.8% (w%/w%) of the total weight of the composition.

The at least one pharmaceutically active principle is entrapped in the triblock:diblock biodegradable drug delivery composition.

Hydrophobic drugs have a low solubility or are insoluble in aqueous solutions, and include, for example, amphotericin, anthralin, beclomethasone, betamethasone, camptothecin, curcumin, dexamethasone, genistein, indomethacin, lidocaine, taxol, tetracycline, tretinoin, therapeutic proteins that are insoluble in water and the like. In the present invention, the pharmaceutically active hydrophobic principle is at least one of medroxyprogesterone acetate, levonorgestrel, cyclosporine, progesterone or bupivacaine. These include uncharged molecules and salts, which are biologically activated when injected into the animal or plant or used as a spatial formulation such that it can be applied on or inside the body of an animal or plant or as a rod implant.

The pharmaceutically effective amount of the hydrophobic active principle as claimed may vary depending on the active principle, the extent of the animal's or plants medical condition and the time required to deliver the hydrophobic active principle. There is no critical upper limit on the amount of hydrophobic active principle incorporated into the polymer solution except for that of an acceptable solution or dispersion viscosity for injection through a syringe needle and that it can effectively treat the medical condition without subjecting the animal or plant to an overdose. The lower limit of the hydrophobic active principle incorporated into the delivery system is dependent simply upon the activity of the hydrophobic active principle and the length of time needed for treatment.

For instance some hydrophobic active principles as claimed may be present in the biodegradable drug delivery composition from 10 to 200 mg/ml. In another aspect the drugs should be present in the amount of 10 to 40 µg/ml.

In another aspect the drugs should be present in the amount of 10 to 500 mg/ml. For a small molecule, for instance, the active principle can be loaded as high as 100 to 200 mg per ml.

Generally the pharmaceutically active principle is present in an amount of 1 % to 20% (w%/w%) of the total weight of the composition. In another aspect the active principle is present in 1 % to 4% (w%/w%) of the total weight of the composition. In another aspect the active principle is present in 2% to 4% (w%/w%) of the total weight of the composition. In yet another aspect the active principle, which is a small molecule, is present in an amount of 10% to 20% (w%/w%) of the total weight of the composition. In another aspect the active principle is present in an amount of 10% to 40% (w%/w%) of the total composition. In another embodiment the pharmaceutically active hydrophobic active principle is present in the amounts of 1% to 40% (w%/w%).

As examples, the medroxyprogesterone acetate can be present in an amount of 10% to 40% (w%/w%) of the total weight of the biodegradable drug delivery compositions; the progesterone can be present in an amount of 20% to 40% (w%/w%) of the total weight of the biodegradable drug delivery compositions; the cyclosporine can be present in an amount of 5% to 21.1% (w%/w%) of the total weight of the biodegradable drug delivery compositions; levonorgestrel can be present in an amount of 10% to 20% (w%/w%) of the total weight of the biodegradable drug delivery compositions; and the bupivacaine can be present in an amount of 1% to 15% (w%/w%) of the total weight of the biodegradable drug delivery compositions.

In the biodegradable drug delivery composition of the present invention, the pharmaceutically effective amount can be released gradually over an extended period of time. This slow release can be continuous or discontinuous, linear or nonlinear and can vary due to the composition of the triblock copolymer and diblock copolymer. Thus, the higher the lactic acid content of the triblock and diblock copolymers in comparison with the polyethylene glycol content, as well as the amount of triblock and diblock copolymers present in the biodegradable drug composition the longer the release of the hydrophobic active principle or drug. In other words, the higher the LA/EO molar ratio and the greater weight percentage of the triblock and diblock copolymers, the longer it will take for the hydrophobic active principle to be released from the drug composition.

The hydrophobic active principle can be released for a duration of between 7 days to 1 year or longer depending upon the type of treatment needed and the biodegradable drug delivery composition used. In one aspect the biodegradable drug delivery composition can deliver the hydrophobic active principle for at least 7 days. In another aspect the biodegradable drug delivery composition can deliver the hydrophobic active principle for at least 30 days. In one aspect the biodegradable drug delivery composition can deliver the hydrophobic active principle for at least 90 days. In yet another aspect the biodegradable drug delivery composition can deliver the hydrophobic active principle for 1 year or longer.

The biodegradable drug delivery composition can be an injectable liquid or a partial suspension at room temperature and be injected through a syringe without excessive force. But these biodegradable drug delivery compositions are also *in situ* forming and biodegradable and turn into solid implants when injected into the animal or plant. Alternatively the biodegradable drug composition is produced as a solid, prepared as small particles and used as a powder which is sprinkled on the injured site. In another aspect the drug delivery composition is a rod implant, which can be implanted under the skin or in another compartment in the body. In another aspect the drug delivery composition can be prepared and applied as a film. In yet another aspect the biodegradable delivery drug composition can be used as a spatial formulation such that it can be applied onto or inside the body of an animal or plant. It can be applied anywhere on the body, including in the eye. In another aspect the biodegradable drug composition can be produced as a partial suspension, the drug being in between the state of being partly soluble and partly solid.

The biodegradable drug delivery composition can further comprise a pharmaceutically acceptable carrier, adjuvant or vehicle. An acceptable carrier can be saline, buffered saline and the like. It can be added to the biodegradable drug delivery composition after its formulation with the drug and diblock copolymer and triblock copolymer.

The adjuvant can be formulated simultaneously when mixing the drug. In this regard the adjuvants that can be used are alum, aluminum phosphate, calcium phosphate, MPL^{™}, CpG motifs, modified toxins, saponins, endogenous stimulatory adjuvants such as cytokines, Freunds complete and incomplete adjuvants, ISCOM type adjuvants, muramyl peptides and the like.

The vehicle can be any diluent, additional solvent, filler or binder that may alter the delivery of the active principle when needed in the biodegradable drug delivery composition. Examples include small amounts of triglycerides such as triacetin or tripropionin. The amount that can be used in the present biodegradable drug deliver compositions of the present invention can vary from 12% to 20% (w%/w%). In one aspect a triacetin can be added in the formulation at 17.0% (w%/w%). In another aspect tripropionin (abbreviated herein as Tripro) can be added at 16% (w%/w%). In yet another aspect benzyl alcohol can be added at 15% to 35% (w%/w%).

The invention also encompasses a method for preparing a biodegradable drug delivery composition, said method comprising:
(i) dissolving in an organic solvent
   (a) a biodegradable ABA type block copolymer having the formula:

      poly(lactic acid)ᵥ-poly(ethylene glycol)_{w}-poly(lactic acid)ₓ

      wherein v, w and x are the number of repeat units ranging from 6 to 1090 or 4 to 1090 and v=x or v≠x; and
   (b) a biodegradable diblock copolymer having the formula:

      methoxy poly(ethylene glycol)_{y}-(polylactic acid)_{z}

      wherein y and z are the number of repeat units ranging from 7 to 371 or 3 to 237, in a ratio of 3:2 to 1:19 (a):(b) to form a polymer mixture; and
(ii) adding at least one pharmaceutically active hydrophobic principle to said polymer mixture, wherein said at least one pharmaceutically active hydrophobic principle is levonorgestrel, cyclosporine, progesterone, bupivacaine or medroxyprogesterone acetate.

In an embodiment, the method further comprises (iii) evaporating said solvent. In this aspect, no solvent is present in the biodegradable drug delivery composition.

The organic solvent that can be used in the method decribed herein is selected from the group of: benzyl alcohol, benzyl benzoate, diethylene glycol dimethyl ether (Diglyme), diethylene glycol monoethyl ether (DEGMEE), dimethyl isosorbide (DMI), dimethyl sulfoxide (DMSO), ethyl acetate, ethyl benzoate, ethyl lactate, ethylene glycol monoethyl ether acetate, glycerol formal, methyl ethyl ketone, methyl isobutyl ketone, N-ethyl-2-pyrrolidone, N-methyl-2-pyrrolidone(NMP), pyrrolidone-2, tetraglycol, triacetin, tributyrin, tripropionin (tripro), or triethylene glycol dimethyl ether (triglyme) and mixtures thereof.

The organic solvent is present in an amount of 40% to 74% (w%/w%) of the total composition. In another aspect the organic solvent used in the preparation of the biodegradable drug delivery composition is present in an amount of 50% to 60% (w%/w%) of the total composition. In yet another aspect the solvent used in the preparation of the biodegradable drug delivery composition is present in an amount of 60% to 70% (w%/w%) of the total composition. In yet another aspect, the solvent used in the preparation of the biodegradable drug delivery system is present in the amount of 30%% to 70% (w%/w%) of the total composition. In another embodiment the organic solvent is present in the amount of 30% to 90% (w%/w%) of the total composition.

As examples, when medroxyprogesterone acetate is the active principle 30% to 70% (w%/w%) of the total composition of solvent is used; when progesterone is the active principle 40% to 80% (w%/w%) of the total composition of solvent is used; when cyclosporine is the active principle 55% to 72.9% (w%/w%) of the total composition of solvent is used; when levonorestrel is the active principle 70% to 90% (w%/w%) of the total composition of solvent is used; and when bupivacaine base is the active principle 62.5 % to 80% (w%/w%) of the total composition of solvent is used.

Some mPEG-OH are contaminated with a small amount of OH-PEG-OH. By following the methods of the present invention and using the contaminated mPEG-OH the final product would be mPEG-PLA contaminated with a small amount of PLA-PEG-PLA, which is encompassed by the present invention. This contamination is less than 2%.

Another aspect of the present invention is the use of diblock and triblock copolymers for the manufacture of a biodegradable drug composition as claimed. In this respect the biodegradable triblock copolymer is as claimed.

The triblock copolymer is then combined with a biodegradable diblock copolymer as claimed.

The pharmaceutically active principle as claimed is then combined with the triblock and diblock copolymers.

The ratio of the biodegradable triblock copolymer of (a) and the biodegradable diblock copolymer of (b) is as claimed in said biodegradable drug composition. In one embodiment the ratio of the biodegradable triblock copolymer and the biodegradable diblock copolymer is selected from the group of 1:3, 1:4, 1:5, 1:6, 1:7 and 1:8 or 1:1, 1:2, 1:3, 1:4, 1:5, 1:6, 1:7, 1:8, 1:9, 1:10, 1:11, 1:12, 1:13, 1:14, 1:15, 1:16, 1:17, 1:18 and 1:19. In another aspect the ratio of the triblock to the diblock is 1:6. It can also be 3:2 or 2:3.

The length of the polyester chain is defined by the lactic acid to ethylene oxide molar ratio, which is between 0.5 to 3.5 or 0.5 to 2.5 or 0.5 to 22.3 for the triblock and 3 to 5 or 2 to 6 or 0.8 to 13 for the diblock.

The mass of the end-capped polyethylene glycol can range from 100 Da to 2 kDa or 164 Da to 2 kDa. It can range in the 100 to 300 Da range or in the 1 kDa to 2 kDa range. The diblock copolymer of the present invention comprises a methoxy-capped poly(ethylene glycol) block according to the claims.

The size of the polyethylene glycol chain ranges from 200 Da to 12 kDa in the biodegradable drug delivery composition or it can range from 400 Da to 12 kDa or 194 Da to 12 kDa.

### EXAMPLES

### Example 1- Polymer synthesis

Copolymers were synthesized according to the method described in the U.S. Patent No. 6,350,812, with minor modifications. Typically, the necessary amount of PEG (gives the triblock coploymer) or methoxyPEG (gives the diblock copolymer) was heated at 65°C and dried under vacuum for 2 hours in a reactor vessel. DL-lactide (corresponding to the targeted LA/EO molar ratio) and zinc lactate (1/1000 of amount of lactide) were added. The reaction mixture was first dehydrated by three short vacuum/N₂ cycles. The reaction mixture was heated at 140°C and rapidly degassed under vacuum. The reaction was conducted for four days at 140°C under constant nitrogen flow (0.2 bar). The reaction was cooled to room temperature and its content was dissolved in acetone and then subjected to precipitation with ethanol. The product obtained was subsequently dried under reduced pressure. The final product was characterized by ¹H NMR for its lactate content. The triblock PLA-PEG-PLA polymers described herein were labeled PxRy where x represents the size of the PEG chain in kDa and y is the LA/EO molar ratio. The diblock mPEG-PLA polymers described herein were labeled dPxRy where represents the size of the PEG chain in kDa and y is the LA/EO molar ratio.

### Example 2-Formulation Preparation Specific for the peptide M53 (not according to the invention)

The formulations described herein were based on organic solution of polymers containing as the drug, the peptide M53, a GLP-1 analogue. Typically, 0.4 grams of polymers, corresponding to a mix of a diblock copolymer and a triblock copolymer in defined mass ratio, were dissolved in 0.57 grams of a biocompatible solvent at room temperature overnight under constant magnetic stirring. The solvent was either a single solvent or a combination of solvents. The next day, 20 mg of drug was added to the polymer solution and stirred until complete dissolution. When the drug was not soluble in the solvent, a suspension of the drug in a polymer solution was obtained. Alternatively, the drug was dissolved or suspended in the biocompatible solvent and the polymer(s) added subsequently. The formulations were loaded in a syringe before use.

### Example 3-The Formulations that were prepared (not according to the invention)

### Following Examples 1 and 2 various formulations were prepared, which are set forth in Table 1 for the peptide M53

**Table 1**

| | | **M5 3** | **Triblock copolymer (TB)** | | | | | | **Diblock copolymer (DB)** | | | | | | **Solvent 1** | | **Solvent 2** | |
|---|---|---|---|---|---|---|---|---|---|---|---|---|---|---|---|---|---|---|
| **N°** | **Ratio DB/T B** | **% (w/w )** | **% (w/w)** | **Cod e** | **PEG size (kDa )** | **Rati o (LA/ EO)** | **DP-PEG** | **DP-PLA** | **% (w/w )** | **Cod e** | **PEG size (kDa )** | **Rati o (LA/ EO)** | **DP-PEG** | **DP-PLA** | **Nam e** | **% (w/w )** | **Nam e** | **% (w/w )** |
| 10 | 4,0 | 4,0 | 10,0% | P12 R0. 5 | 12 | 0,5 | 273 | 136 | 40,0 % | dP2 R3 | 2 | 3,2 | 45 | 143 | DE GM EE | 46,0 % | | |
| 12 | 4,0 | 4,0 | 10,0% | P12 R3 | 12 | 2,5 | 273 | 682 | 40,0 % | dP2 R3 | 2 | 3,2 | 45 | 143 | DE GM EE | 46,0 % | | |
| 21 | 4,0 | 4,0 | 10,0% | P12 R0. 5 | 12 | 0,5 | 273 | 136 | 40,0 % | dP2 R3 | 2 | 3,2 | 45 | 143 | Digl yme | 46,0 % | | |
| 23 | 4,0 | 4,0 | 10,0% | P12 R3 | 12 | 2,5 | 273 | 682 | 40,0 % | dP2 R3 | 2 | 3,2 | 45 | 143 | Digl yme | 46,0 % | | |
| 34 | 4,0 | 4,0 | 10,0% | P12 R0. 5 | 12 | 0,5 | 273 | 136 | 40,0 % | dP2 R3 | 2 | 3,2 | 45 | 143 | DMI | 46,0 % | | |
| 45 | 4,0 | 4,0 | 10,0% | P12 R3 | 12 | 2,5 | 273 | 682 | 40,0 % | dP2 R3 | 2 | 3,2 | 45 | 143 | DMI | 46,0 % | | |
| 66 | 4,0 | 4,0 | 10,0% | P12 R0. 5 | 12 | 0,5 | 273 | 136 | 40,0 % | dP2 R3 | 2 | 3,2 | 45 | 143 | Digl yme | 46,0 % | | |
| 68 | 4,0 | 4,0 | 10,0% | P12 R3 | 12 | 2,5 | 273 | 682 | 40,0 % | dP2 R3 | 2 | 3,2 | 45 | 143 | Digl yme | 46,0 % | | |
| 76 | 4,0 | 4,0 | 10,0% | P12 R0. 5 | 12 | 0,5 | 273 | 136 | 40,0 % | dP2 R3 | 2 | 3,2 | 45 | 143 | DM SO | 46,0 % | | |
| 78 | 4,0 | 4,0 | 10,0% | P12 R3 | 12 | 2,5 | 273 | 682 | 40,0 % | dP2 R3 | 2 | 3,2 | 45 | 143 | DM SO | 46,0 % | | |
| 80 | 4,0 | 4,0 | 10,0% | P12 R0. 5 | 12 | 0,5 | 273 | 136 | 40,0 % | dP2 R3 | 2 | 3,2 | 45 | 143 | Et Lact ate | 46,0 % | | |
| 82 | 4,0 | 4,0 | 10,0% | P12 R3 | 12 | 2,5 | 273 | 682 | 40,0 % | dP2 R3 | 2 | 3,2 | 45 | 143 | Et Lact ate | 46,0 % | | |
| 105 | 4,0 | 4,0 | 8,0% | P6R 0.9 | 6 | 0,9 | 136 | 123 | 32,0 % | dP2 R4 | 2 | 4,4 | 45 | 200 | Digl yme | 56,0 % | | |
| 116 | 4,0 | 4,0 | 8,0% | P6R 0.9 | 6 | 0,9 | 136 | 123 | 32,0 % | dP2 R4 | 2 | 4,4 | 45 | 200 | Digl yme | 56,0 % | | |
| 123 | 4,0 | 4,0 | 8,0% | P3R 1 | 3 | 1,0 | 68 | 68 | 32,0 % | dP2 R4 | 2 | 4,3 | 45 | 195 | DM SO | 56,0 % | | |
| 124 | 4,0 | 4,0 | 8,0% | P6R 0.9 | 6 | 0,9 | 136 | 123 | 32,0 % | dP2 R4 | 2 | 4,3 | 45 | 195 | DM SO | 56,0 % | | |
| 153 | 4,0 | 4,0 | 7,0% | P12 R0.5 | 12 | 0,5 | 273 | 136 | 28,0 % | dP2 R4 | 2 | 4,3 | 45 | 195 | DM SO | 61,0 % | | |
| 159 | 4,0 | 4,0 | 7,0% | P12 R0. 5 | 12 | 0,5 | 273 | 136 | 28,0 % | dP2 R4 | 2 | 4,3 | 45 | 195 | DM SO | 44,0 % | Tra ceti n | 17,0 % |
| 169 | 5,7 | 2,0 | 6,0% | P6R 0.9 | 6 | 0,9 | 136 | 123 | 34,0 % | dP2 R4 | 2 | 4,3 | 45 | 195 | DM SO | 58,0 % | | |
| 177 | 5,7 | 2,0 | 7,5% | P6R 0.9 | 6 | 0,9 | 136 | 123 | 42,5 % | dP2 R4 | 2 | 4,3 | 45 | 195 | DM SO | 48,0 % | | |
| 198 | 9,0 | 4,0 | 4,0% | P6R 0.9 | 6 | 0,9 | 136 | 123 | 36,0 % | dP2 R4 | 2 | 4,3 | 45 | 195 | Digl yme | 37,0 % | Trip ro | 19,0 % |
| 200 | 9,0 | 2,0 | 5,0% | P6R 0.9 | 6 | 0,9 | 136 | 123 | 45,0 % | dP2 R3 | 2 | 3 | 45 | 136 | DM SO | 48,0 % | | |
| 203 | 4,0 | 2,0 | 10,0% | P6R 0.9 | 6 | 0,9 | 136 | 123 | 40,0 % | dP2 R7 | 2 | 7,2 | 45 | 327 | DM SO | 48,0 % | | |
| 207 | 5,7 | 4,0 | 6,0% | P6R 0.9 | 6 | 0,9 | 136 | 123 | 34,0 % | dP2 R4 | 2 | 4,3 | 45 | 195 | Digl yme | 40,0 % | Trip ro | 16,0 % |
| 209 | 4,0 | 2,0 | 9,0% | P6R 0.9 | 6 | 0,9 | 136 | 123 | 36,0 % | dP2 R7 | 2 | 7,2 | 45 | 327 | DM SO | 53,0 % | | |
| 210 | 4,0 | 2,0 | 8,0% | P6R 0.9 | 6 | 0,9 | 136 | 123 | 32,0 % | dP2 R7 | 2 | 7,2 | 45 | 327 | DM SO | 58,0 % | | |
| 221 | 9,0 | 4,0 | 5,0% | P6R 0.9 | 6 | 0,9 | 136 | 123 | 45,0 % | dP2 R4 | 2 | 4,3 | 45 | 195 | Digl yme | 33,0 % | Trip ro | 13,0 % |
| 224 | 5,7 | 2,0 | 6,0% | P6R 0.9 | 6 | 0,9 | 136 | 123 | 34,0 % | dP2 R4 | 2 | 4,3 | 45 | 195 | Digl yme | 41,4 % | Trip ro | 16,6 % |
| 225 | 9,0 | 2,0 | 5,0% | P6R 0.9 | 6 | 0,9 | 136 | 123 | 45,0 % | dP2 R4 | 2 | 4,3 | 45 | 195 | Digl yme | 34,0 % | Trip ro | 13,6 % |
| 230 | 5,7 | 2,0 | 7,5% | P6R 0.9 | 6 | 0,9 | 136 | 123 | 42,5 % | dP1 R5 | 1 | 5,4 | 23 | 123 | DM SO | 48,0 % | | |
| 234 | 5,7 | 2,0 | 6,0% | P6R 0.9 | 6 | 0,9 | 136 | 123 | 34,0 % | dP1 R5 | 1 | 5,4 | 23 | 123 | Digl yme | 41,4 % | Trip ro | 16,6 % |
| 241 | 5,9 | 2,0 | 6,5% | P6R 0.9 | 6 | 0,9 | 136 | 123 | 38,5 % | dP1 R5 | 1 | 5,4 | 23 | 123 | DM SO | 53,0 % | | |
| 245 | 5,9 | 2,0 | 6,5% | P2R 2 | 2 | 2 | 45 | 91 | 38,5 % | dP1 R5 | 1 | 5,4 | 23 | 123 | DM SO | 53 % | | |
| 246 | 5,7 | 2,0 | 7,5% | P2R 2 | 2 | 2 | 45 | 91 | 42,5 % | dP1 R5 | 1 | 5,4 | 23 | 123 | DM SO | 48,0 % | | |
| 247 | 9,0 | 2,0 | 5,0% | P2R 2 | 2 | 2 | 45 | 91 | 45,0 % | dP1 R5 | 1 | 5,4 | 23 | 123 | DM SO | 48,0 % | | |
| 250 | 9,0 | 4,0 | 5,0% | P6R 0.9 | 6 | 0,9 | 136 | 123 | 45,0 % | dP2 R4 | 2 | 4,3 | 45 | 195 | Digl yme | 33,2 % | Trip ro | 12,8 % |

### Example 4-Acetaminophen's formulations preparation (not according to the invention)

The formulations described herein were based on organic solution of polymers prepared as in Example 1, containing as the drug, acetaminophen. Typically, 0.4 grams of polymers, corresponding to a mix of a diblock copolymer and a triblock copolymer in defined mass ratio, were dissolved in 0.55 grams of dimethyl sulfoxide at room temperature overnight under constant magnetic stirring. The next day, 50 mg of acetaminophen was added to the polymer solution and stirred until complete dissolution. The formulations were loaded in a syringe before use. The composition of the various formulations is shown in Table 2 below, where the solvent used is DMSO.

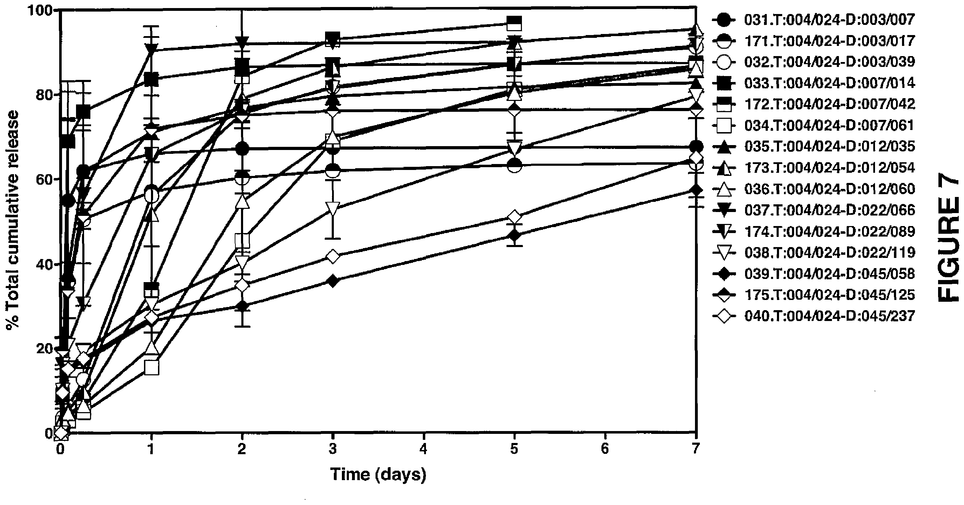

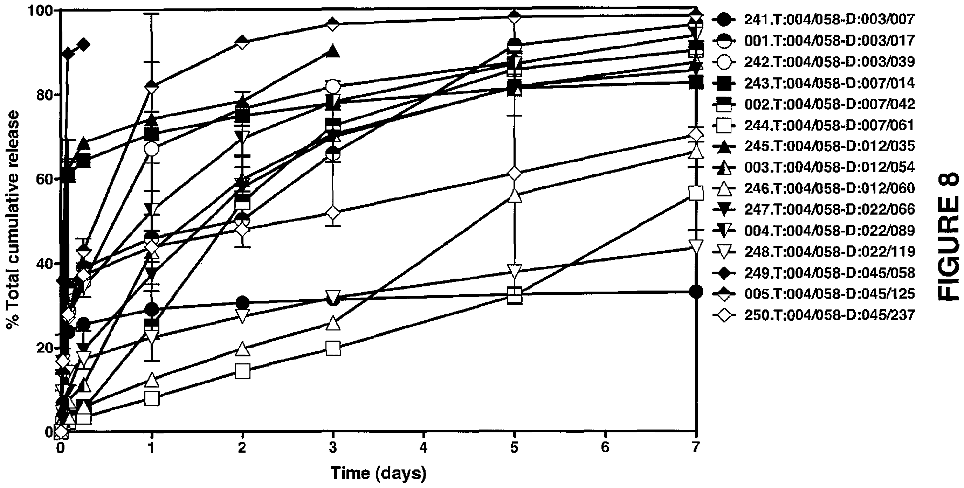

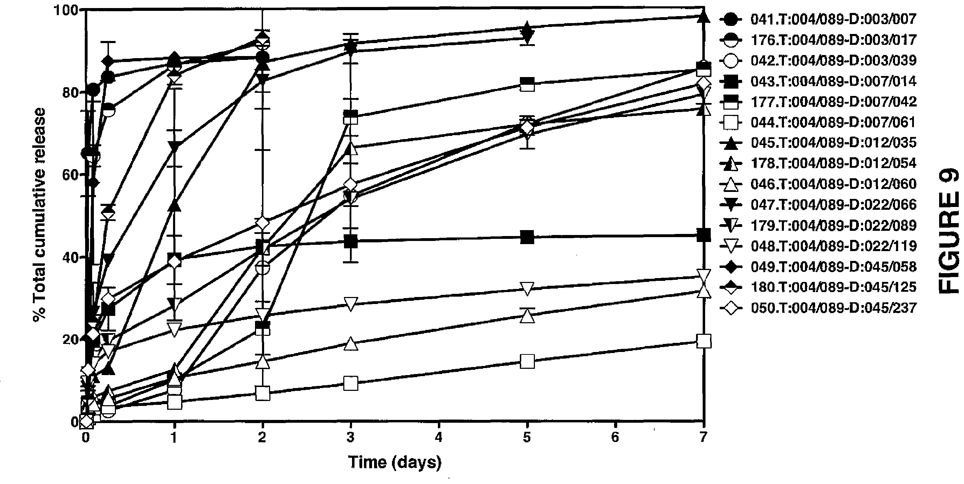

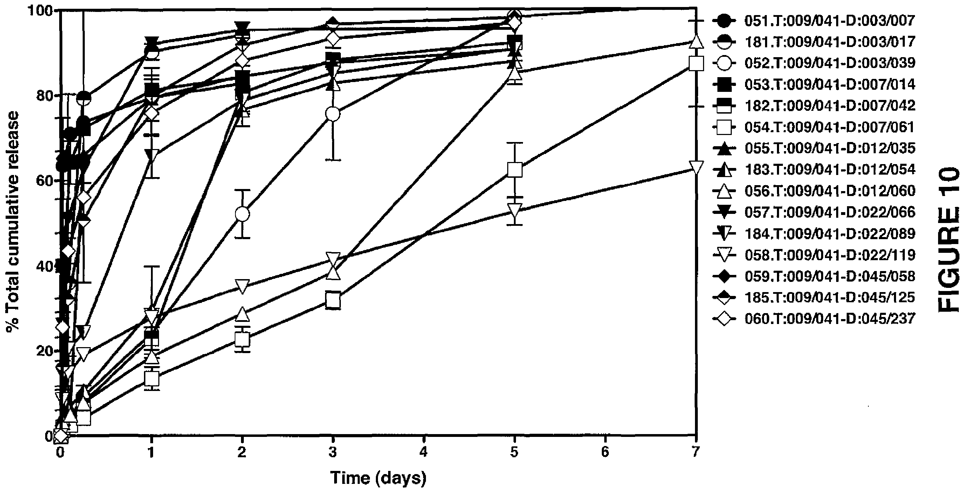

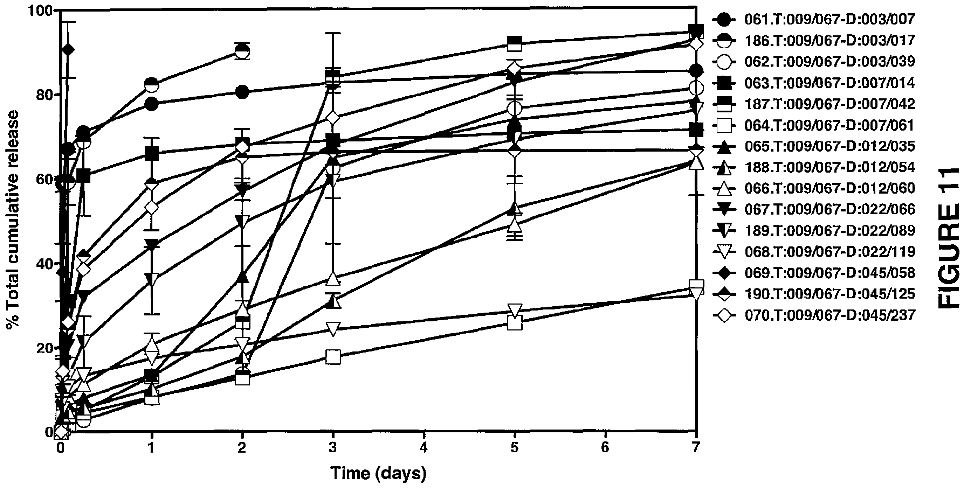

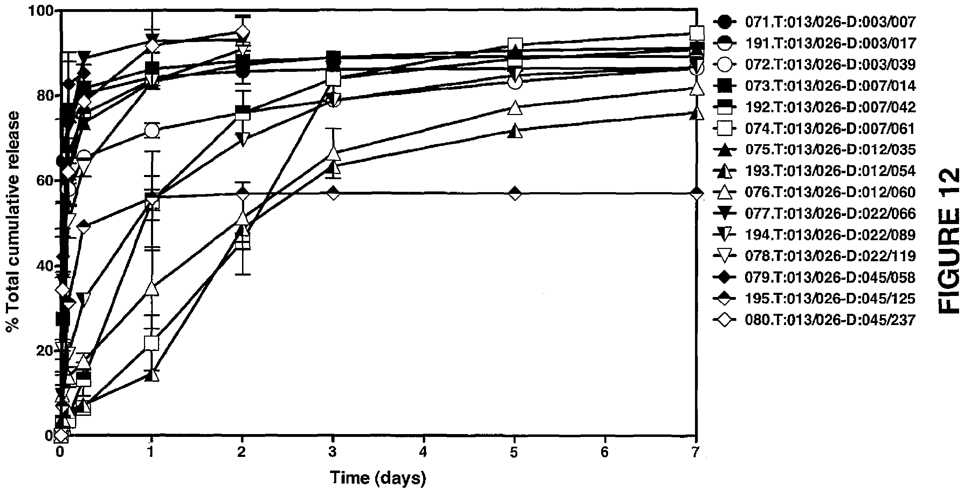

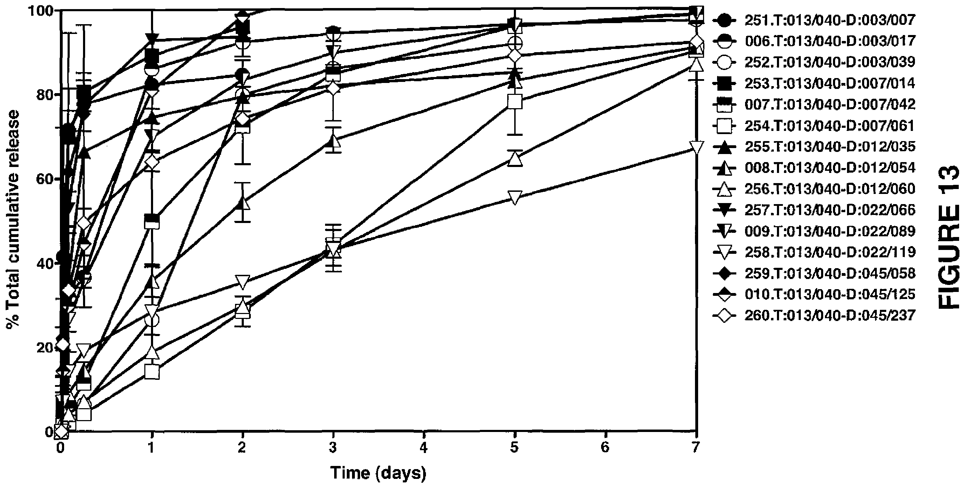

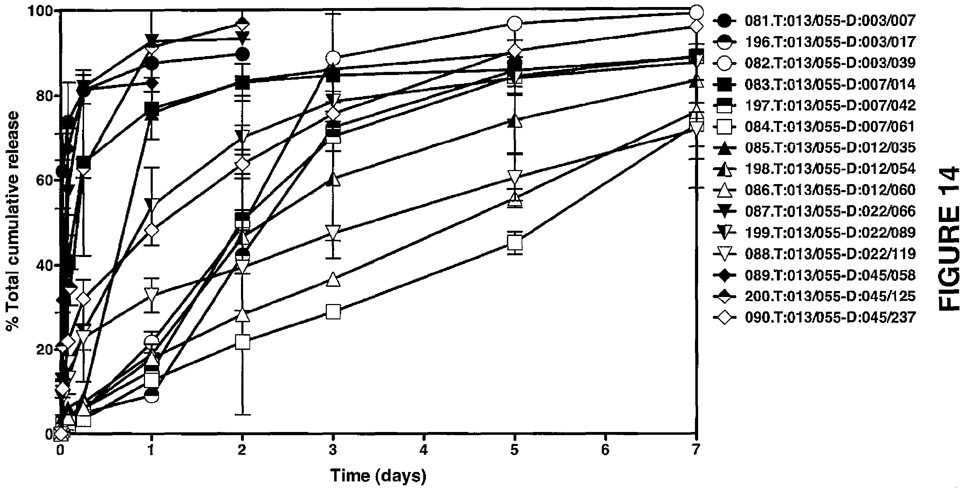

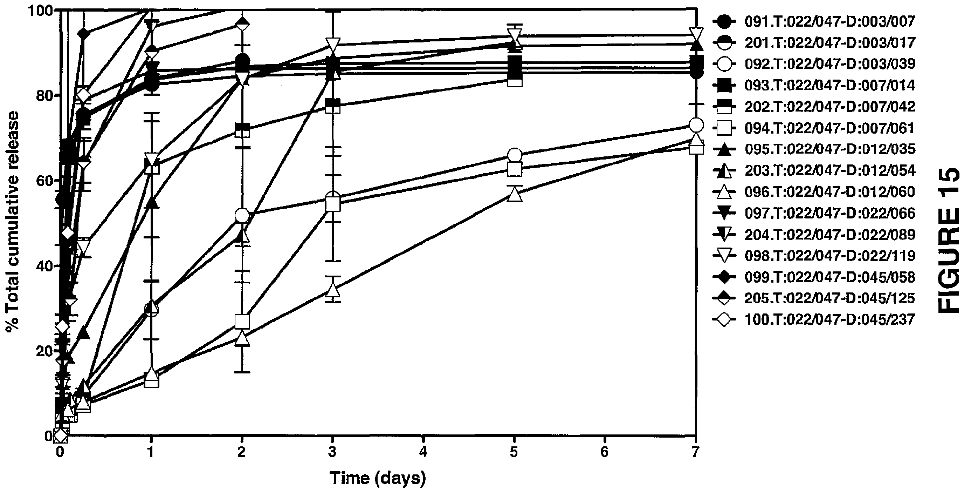

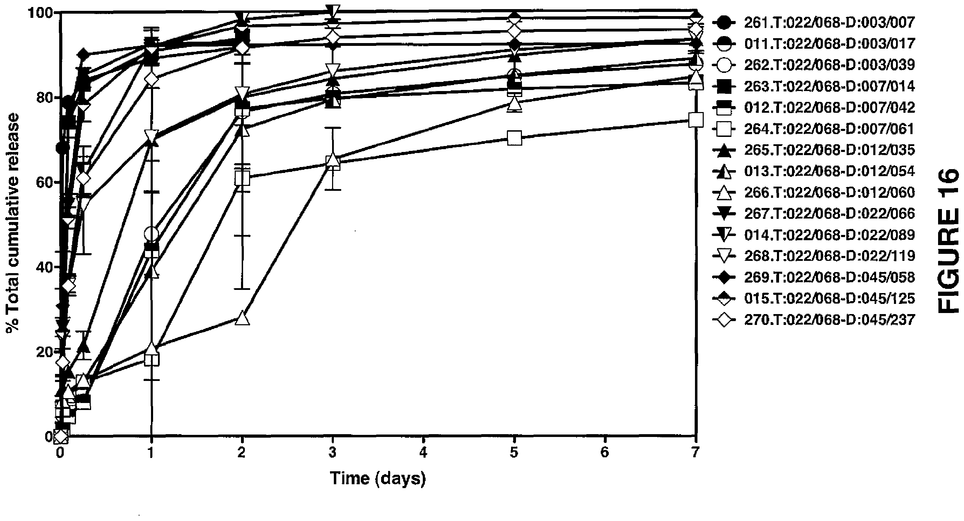

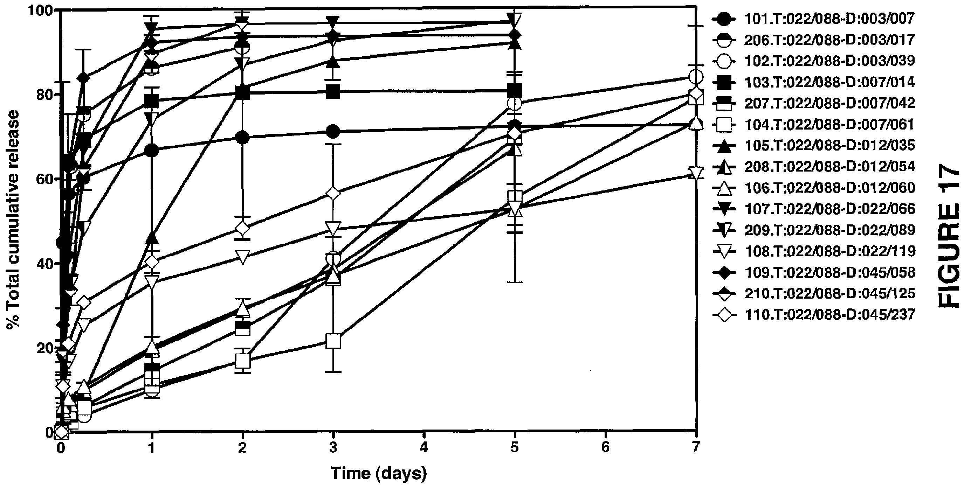

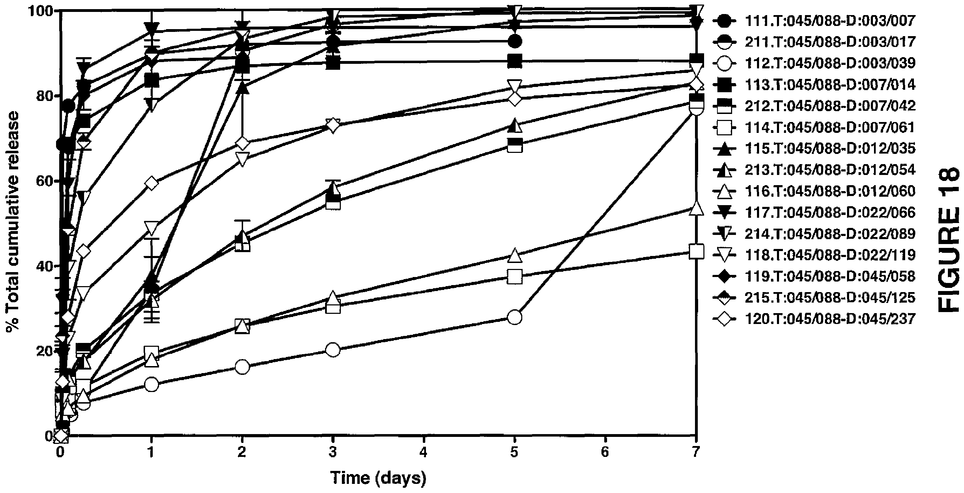

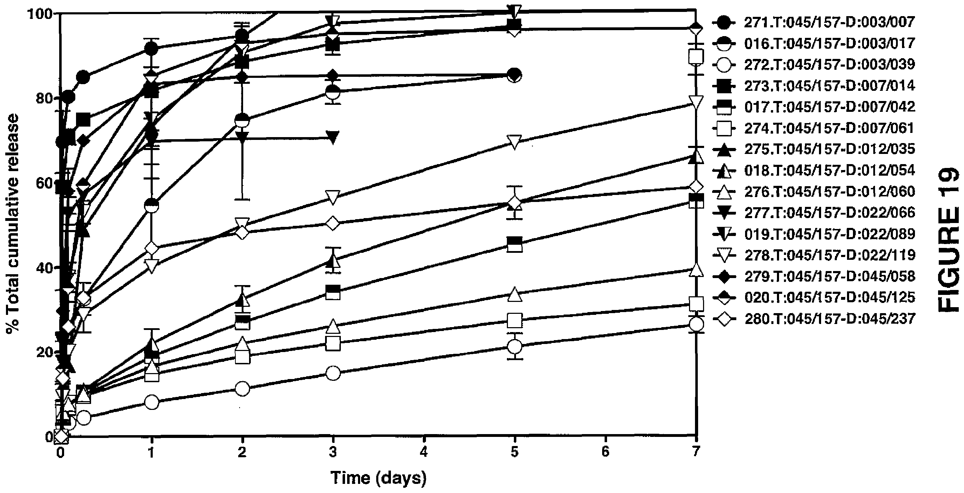

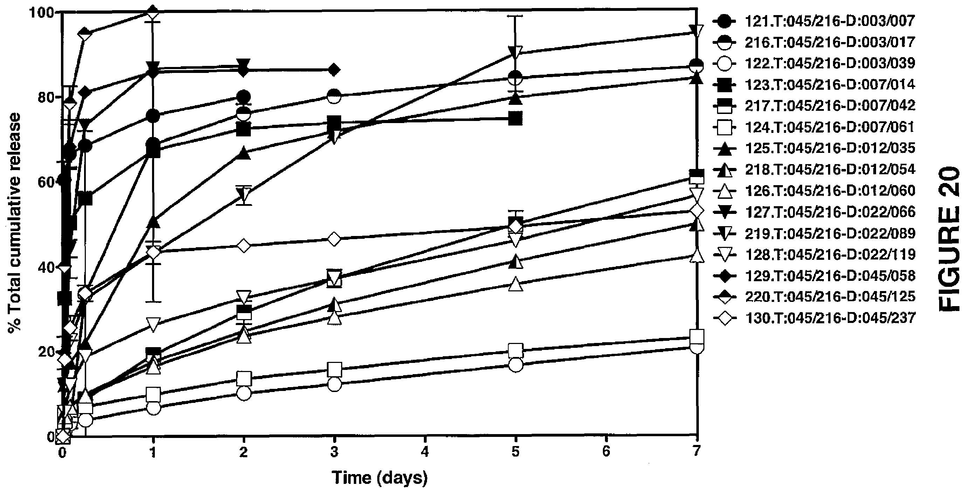

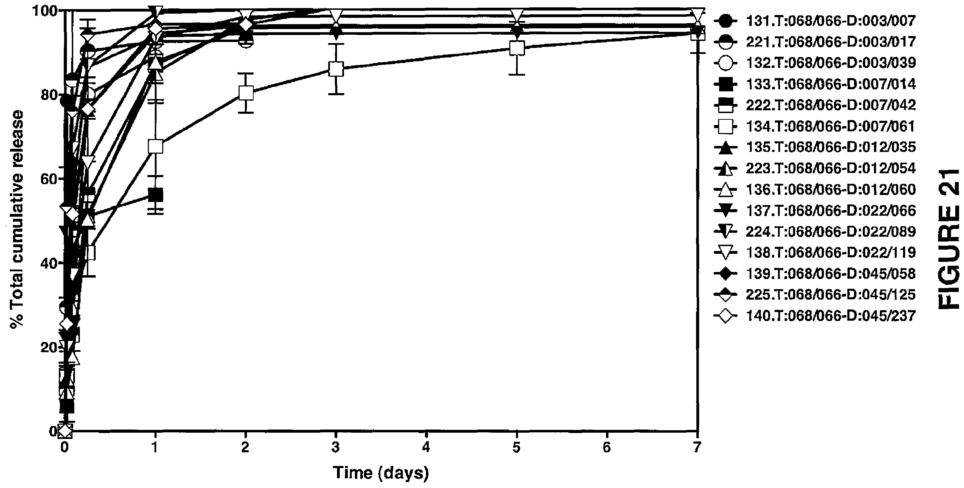

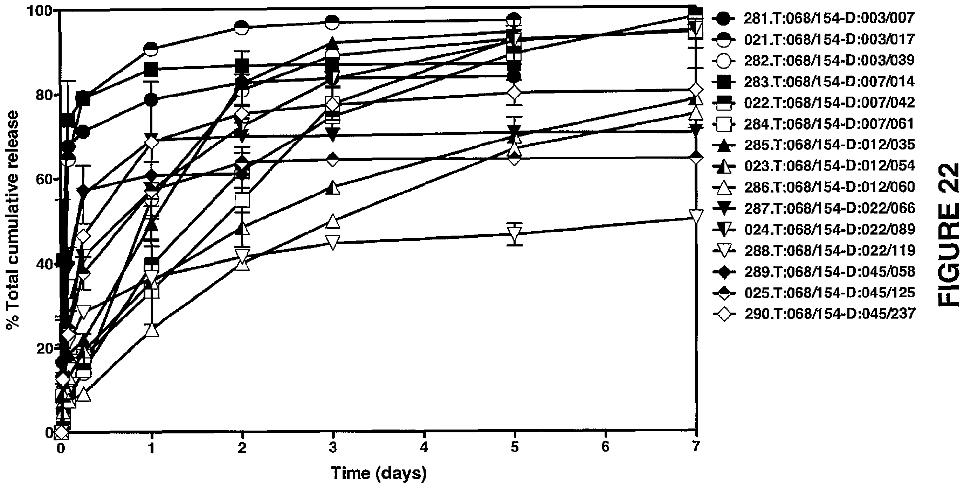

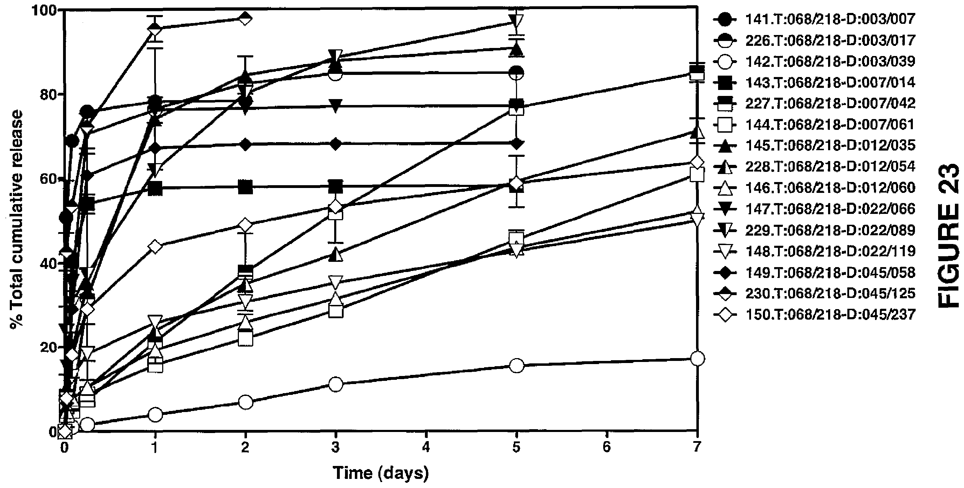

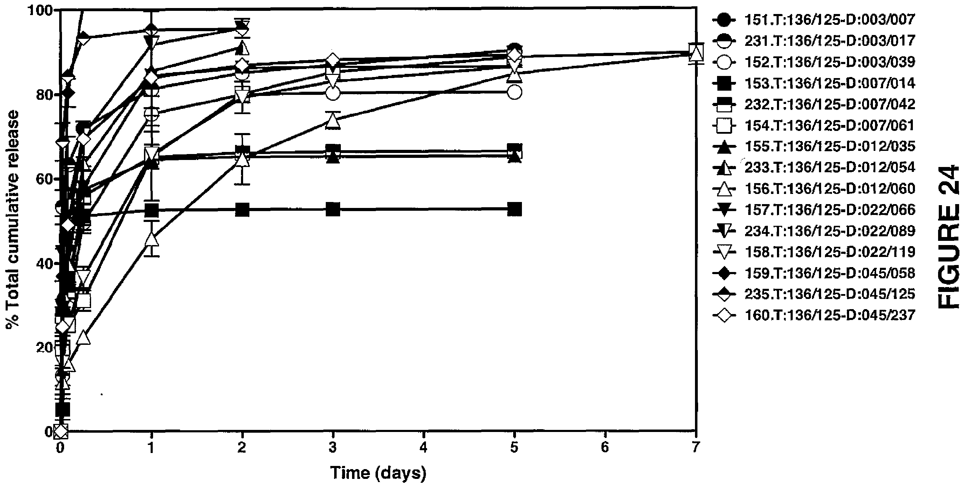

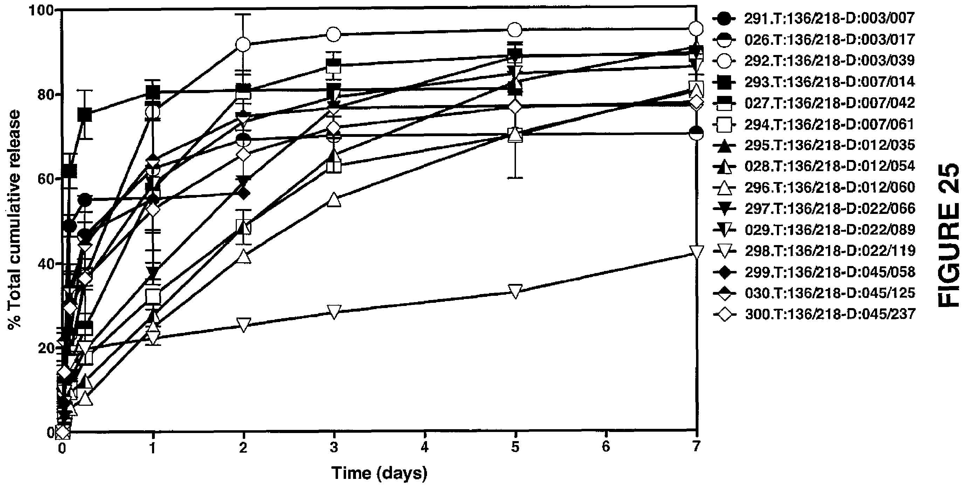

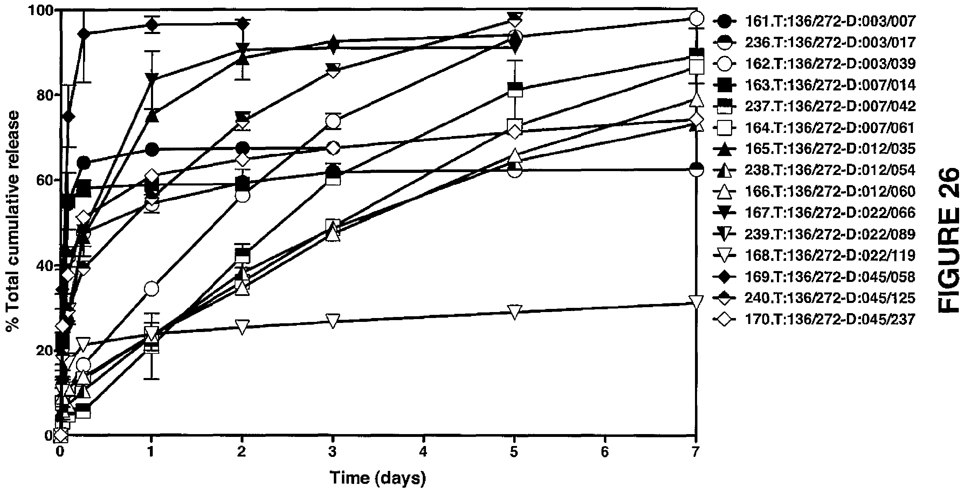

Figures 7 to 26 illustrate the results of these formulations which show all possible combinations of 15 triblock copolymers with 20 diblocks copolymers.

**Table 2**

| | | **Triblock copolymer (TB)** | | | | | | **Diblock copolymer (DB)** | | | | | | **Solvent** | |
|---|---|---|---|---|---|---|---|---|---|---|---|---|---|---|---|
| **Exp n°** | **Ratio DB/T B** | **% (w/w )** | **Cod e** | **PEG (kD a)** | **Rati o (LA/ EO)** | **DP-PEG** | **DP-PLA** | **% (w/w )** | **Cod e** | **PEG (kDa )** | **Rati ο (LA/ EO)** | **DP-PEG** | **DP-PLA** | **Name** | **% (w/w)** |
| **1** | 4,0 | 8% | P0.2 R14 | 0,2 | 14,5 | 4 | 58 | 32% | dP0. 2R6 | 0,2 | 5,8 | 3 | 17 | DMS O | 55% |
| **2** | 4,0 | 8% | P0.2 R14 | 0,2 | 14,5 | 4 | 58 | 32% | dP0. 4R6 | 0,4 | 5,8 | 7 | 42 | DMS O | 55% |
| **3** | 4,0 | 8% | P0.2 R14 | 0,2 | 14,5 | 4 | 58 | 32% | dP0. 6R5 | 0,6 | 4,6 | 12 | 54 | DMS O | 55% |
| **4** | 4,0 | 8% | P0.2 R14 | 0,2 | 14,5 | 4 | 58 | 32% | dP1 R4 | 1,0 | 4,0 | 22 | 89 | DMS O | 55% |
| **5** | 4,0 | 8% | P0.2 R14 | 0,2 | 14,5 | 4 | 58 | 32% | dP2 R3 | 2,0 | 2,8 | 45 | 125 | DMS O | 55% |
| **6** | 4,0 | 8% | P0.6 R3 | 0,6 | 3,0 | 13 | 40 | 32% | dP0. 2R6 | 0,2 | 5,8 | 3 | 17 | DMS O | 55% |
| **7** | 4,0 | 8% | P0.6 R3 | 0,6 | 3,0 | 13 | 40 | 32% | dP0. 4R6 | 0,4 | 5,8 | 7 | 42 | DMS O | 55% |
| **8** | 4,0 | 8% | P0.6 R3 | 0,6 | 3,0 | 13 | 40 | 32% | dP0. 6R5 | 0,6 | 4,6 | 12 | 54 | DMS O | 55% |
| **9** | 4,0 | 8% | P0.6 R3 | 0,6 | 3,0 | 13 | 40 | 32% | dP1 R4 | 1,0 | 4,0 | 22 | 89 | DMS O | 55% |
| **10** | 4,0 | 8% | P0.6 R3 | 0,6 | 3,0 | 13 | 40 | 32% | dP2 R3 | 2,0 | 2,8 | 45 | 125 | DMS O | 55% |
| **11** | 4,0 | 8% | P1R 3 | 1,0 | 3,1 | 22 | 68 | 32% | dP0. 2R6 | 0,2 | 5,8 | 3 | 17 | DMS O | 55% |
| **12** | 4,0 | 8% | P1R 3 | 1,0 | 3,1 | 22 | 68 | 32% | dP0. 4R6 | 0,4 | 5,8 | 7 | 42 | DMS O | 55% |
| **13** | 4,0 | 8% | P1R 3 | 1,0 | 3,1 | 22 | 68 | 32% | dP0. 6R5 | 0,6 | 4,6 | 12 | 54 | DMS O | 55% |
| **14** | 4,0 | 8% | P1R 3 | 1,0 | 3,1 | 22 | 68 | 32% | dP1 R4 | 1,0 | 4,0 | 22 | 89 | DMS O | 55% |
| **15** | 4,0 | 8% | P1R 3 | 1,0 | 3,1 | 22 | 68 | 32% | dP2 R3 | 2,0 | 2,8 | 45 | 125 | DMS O | 55% |
| **16** | 4,0 | 8% | P2R 3 | 2,0 | 3,5 | 45 | 157 | 32% | dP0. 2R6 | 0,2 | 5,8 | 3 | 17 | DMS O | 55% |
| **17** | 4,0 | 8% | P2R 3 | 2,0 | 3,5 | 45 | 157 | 32% | dP0. 4R6 | 0,4 | 5,8 | 7 | 42 | DMS O | 55% |
| **18** | 4,0 | 8% | P2R 3 | 2,0 | 3,5 | 45 | 157 | 32% | dP0. 6R5 | 0,6 | 4,6 | 12 | 54 | DMS O | 55% |
| **19** | 4,0 | 8% | P2R 3 | 2,0 | 3,5 | 45 | 157 | 32% | dP1 R4 | 1,0 | 4,0 | 22 | 89 | DMS O | 55% |
| **20** | 4,0 | 8% | P2R | 2,0 | 3,5 | 45 | 157 | 32% | dP2 R3 | 2,0 | 2,8 | 45 | 125 | DMS | 55% |
| **21** | 4,0 | 8% | P3R 2 | 3,0 | 2,3 | 68 | 154 | 32% | dP0. 2R6 | 0,2 | 5,8 | 3 | 17 | DMS O | 55% |
| **22** | 4,0 | 8% | P3R 2 | 3,0 | 2,3 | 68 | 154 | 32% | dP0. 4R6 | 0,4 | 5,8 | 7 | 42 | DMS O | 55% |
| **23** | 4,0 | 8% | P3R 2 | 3,0 | 2,3 | 68 | 154 | 32% | dP0. 6R5 | 0,6 | 4,6 | 12 | 54 | DMS O | 55% |
| **24** | 4,0 | 8% | P3R 2 | 3,0 | 2,3 | 68 | 154 | 32% | dP1 R4 | 1,0 | 4,0 | 22 | 89 | DMS O | 55% |
| **25** | 4,0 | 8% | P3R 2 | 3,0 | 2,3 | 68 | 154 | 32% | dP2 R3 | 2,0 | 2,8 | 45 | 125 | DMS O | 55% |
| **26** | 4,0 | 8% | P6R 2 | 6,0 | 1,6 | 136 | 218 | 32% | dP0. 2R6 | 0,2 | 5,8 | 3 | 17 | DMS O | 55% |
| **27** | 4,0 | 8% | P6R 2 | 6,0 | 1,6 | 136 | 218 | 32% | dP0. 4R6 | 0,4 | 5,8 | 7 | 42 | DMS O | 55% |
| **28** | 4,0 | 8% | P6R 2 | 6,0 | 1,6 | 136 | 218 | 32% | dP0. 6R5 | 0,6 | 4,6 | 12 | 54 | DMS O | 55% |
| **29** | 4,0 | 8% | P6R 2 | 6,0 | 1,6 | 136 | 218 | 32% | dP1 R4 | 1,0 | 4,0 | 22 | 89 | DMS O | 55% |
| **30** | 4,0 | 8% | P6R 2 | 6,0 | 1,6 | 136 | 218 | 32% | dP2 R3 | 2,0 | 2,8 | 45 | 125 | DMS O | 55% |
| **31** | 4,0 | 8% | P0.2 R6 | 0,2 | 5,9 | 4 | 24 | 32% | dP0. 2R2 | 0,2 | 2,2 | 3 | 7 | DMS O | 55% |
| **32** | 4,0 | 8% | P0.2 R6 | 0,2 | 5,9 | 4 | 24 | 32% | dP0. 2R1 3 | 0,2 | 13,0 | 3 | 39 | DMS O | 55% |
| **33** | 4,0 | 8% | P0.2 R6 | 0,2 | 5,9 | 4 | 24 | 32% | dP0. 4R2 | 0,4 | 2,0 | 7 | 14 | DMS O | 55% |
| **34** | 4,0 | 8% | P0.2 R6 | 0,2 | 5,9 | 4 | 24 | 32% | dP0. 4R8 | 0,4 | 8,4 | 7 | 61 | DMS O | 55% |
| **35** | 4,0 | 8% | P0.2 R6 | 0,2 | 5,9 | 4 | 24 | 32% | dP0. 6R3 | 0,6 | 3,0 | 12 | 35 | DMS O | 55% |
| **36** | 4,0 | 8% | P0.2 R6 | 0,2 | 5,9 | 4 | 24 | 32% | dP0. 6R5 | 0,6 | 5,1 | 12 | 60 | DMS O | 55% |
| **37** | 4,0 | 8% | P0.2 R6 | 0,2 | 5,9 | 4 | 24 | 32% | dP1 R3 | 1,0 | 3,0 | 22 | 66 | DMS O | 55% |
| **38** | 4,0 | 8% | P0.2 R6 | 0,2 | 5,9 | 4 | 24 | 32% | dP1 R5 | 1,0 | 5,4 | 22 | 119 | DMS O | 55% |
| **39** | 4,0 | 8% | P0.2 R6 | 0,2 | 5,9 | 4 | 24 | 32% | dP2 R1 | 2,0 | 1,3 | 45 | 58 | DMS O | 55% |
| **40** | 4,0 | 8% | P0.2 R6 | 0,2 | 5,9 | 4 | 24 | 32% | dP2 R5 | 2,0 | 5,3 | 45 | 237 | DMS O | 55% |
| **41** | 4,0 | 8% | P0.2 R22 | 0,2 | 22,3 | 4 | 89 | 32% | dP0. 2R2 | 0,2 | 2,2 | 3 | 7 | DMS O | 55% |
| **42** | 4,0 | 8% | P0.2 R22 | 0,2 | 22,3 | 4 | 89 | 32% | dP0. 2R1 3 | 0,2 | 13,0 | 3 | 39 | DMS O | 55% |
| **43** | 4,0 | 8% | P0.2 R22 | 0,2 | 22,3 | 4 | 89 | 32% | dP0. 4R2 | 0,4 | 2,0 | 7 | 14 | DMS O | 55% |
| **44** | 4,0 | 8% | P0.2 R22 | 0,2 | 22,3 | 4 | 89 | 32% | dP0. 4R8 | 0,4 | 8,4 | 7 | 61 | DMS O | 55% |
| **45** | 4,0 | 8% | P0.2 R22 | 0,2 | 22,3 | 4 | 89 | 32% | dP0. 6R3 | 0,6 | 3,0 | 12 | 35 | DMS O | 55% |
| **46** | 4,0 | 8% | P0.2 R22 | 0,2 | 22,3 | 4 | 89 | 32% | dP0. 6R5 | 0,6 | 5,1 | 12 | 60 | DMS O | 55% |
| **47** | 4,0 | 8% | P0.2 R22 | 0,2 | 22,3 | 4 | 89 | 32% | dP1 R3 | 1,0 | 3,0 | 22 | 66 | DMS O | 55% |
| **48** | 4,0 | 8% | P0.2 R22 | 0,2 | 22,3 | 4 | 89 | 32% | dP1 R5 | 1,0 | 5,4 | 22 | 119 | DMS O | 55% |
| **49** | 4,0 | 8% | P0.2 R22 | 0,2 | 22,3 | 4 | 89 | 32% | dP2 R1 | 2,0 | 1,3 | 45 | 58 | DMS O | 55% |
| **50** | 4,0 | 8% | P0.2 R22 | 0,2 | 22,3 | 4 | 89 | 32% | dP2 R5 | 2,0 | 5,3 | 45 | 237 | DMS O | 55% |
| **51** | 4,0 | 8% | P0.4 R5 | 0,4 | 4,7 | 9 | 41 | 32% | dP0. 2R2 | 0,2 | 2,2 | 3 | 7 | DMS O | 55% |
| **52** | 4,0 | 8% | P0.4 R5 | 0,4 | 4,7 | 9 | 41 | 32% | dP0. 2R1 3 | 0,2 | 13,0 | 3 | 39 | DMS O | 55% |
| **53** | 4,0 | 8% | P0.4 R5 | 0,4 | 4,7 | 9 | 41 | 32% | dP0. 4R2 | 0,4 | 2,0 | 7 | 14 | DMS O | 55% |
| **54** | 4,0 | 8% | P0.4 R5 | 0,4 | 4,7 | 9 | 41 | 32% | dP0. 4R8 | 0,4 | 8,4 | 7 | 61 | DMS O | 55% |
| **55** | 4,0 | 8% | P0.4 R5 | 0,4 | 4,7 | 9 | 41 | 32% | dP0. 6R3 | 0,6 | 3,0 | 12 | 35 | DMS O | 55% |
| **56** | 4,0 | 8% | P0.4 R5 | 0,4 | 4,7 | 9 | 41 | 32% | dP0. 6R5 | 0,6 | 5,1 | 12 | 60 | DMS O | 55% |
| **57** | 4,0 | 8% | P0.4 R5 | 0,4 | 4,7 | 9 | 41 | 32% | dP1 R3 | 1,0 | 3,0 | 22 | 66 | DMS O | 55% |
| **58** | 4,0 | 8% | P0.4 R5 | 0,4 | 4,7 | 9 | 41 | 32% | dP1 R5 | 1,0 | 5,4 | 22 | 119 | DMS O | 55% |
| **59** | 4,0 | 8% | P0.4 R5 | 0,4 | 4,7 | 9 | 41 | 32% | dP2 R1 | 2,0 | 1,3 | 45 | 58 | DMS O | 55% |
| **60** | 4,0 | 8% | P0.4 R5 | 0,4 | 4,7 | 9 | 41 | 32% | dP2 R5 | 2,0 | 5,3 | 45 | 237 | DMS O | 55% |
| **61** | 4,0 | 8% | P0.4 R8 | 0,4 | 7,7 | 9 | 67 | 32% | dP0. 2R2 | 0,2 | 2,2 | 3 | 7 | DMS O | 55% |
| **62** | 4,0 | 8% | P0.4 R8 | 0,4 | 7,7 | 9 | 67 | 32% | dP0. 2R1 3 | 0,2 | 13,0 | 3 | 39 | DMS O | 55% |
| **63** | 4,0 | 8% | P0.4 R8 | 0,4 | 7,7 | 9 | 67 | 32% | dP0. 4R2 | 0,4 | 2,0 | 7 | 14 | DMS O | 55% |
| **64** | 4,0 | 8% | P0.4 R8 | 0,4 | 7,7 | 9 | 67 | 32% | dP0. 4R8 | 0,4 | 8,4 | 7 | 61 | DMS O | 55% |
| **65** | 4,0 | 8% | P0.4 R8 | 0,4 | 7,7 | 9 | 67 | 32% | dP0. 6R3 | 0,6 | 3,0 | 12 | 35 | DMS O | 55% |
| **66** | 4,0 | 8% | P0.4 R8 | 0,4 | 7,7 | 9 | 67 | 32% | dP0. 6R5 | 0,6 | 5,1 | 12 | 60 | DMS O | 55% |
| **67** | 4,0 | 8% | P0.4 R8 | 0,4 | 7,7 | 9 | 67 | 32% | dP1 R3 | 1,0 | 3,0 | 22 | 66 | DMS O | 55% |
| **68** | 4,0 | 8% | P0.4 R8 | 0,4 | 7,7 | 9 | 67 | 32% | dP1 R5 | 1,0 | 5,4 | 22 | 119 | DMS O | 55% |
| **69** | 4,0 | 8% | P0.4 R8 | 0,4 | 7,7 | 9 | 67 | 32% | dP2 R1 | 2,0 | 1,3 | 45 | 58 | DMS O | 55% |
| **70** | 4,0 | 8% | P0.4 R8 | 0,4 | 7,7 | 9 | 67 | 32% | dP2 R5 | 2,0 | 5,3 | 45 | 237 | DMS O | 55% |
| **71** | 4,0 | 8% | P0.6 R2 | 0,6 | 1,9 | 13 | 26 | 32% | dP0. 2R2 | 0,2 | 2,2 | 3 | 7 | DMS O | 55% |
| **72** | 4,0 | 8% | P0.6 R2 | 0,6 | 1,9 | 13 | 26 | 32% | dP0. 2R1 3 | 0,2 | 13,0 | 3 | 39 | DMS O | 55% |
| **73** | 4,0 | 8% | P0.6 R2 | 0,6 | 1,9 | 13 | 26 | 32% | dP0. 4R2 | 0,4 | 2,0 | 7 | 14 | DMS O | 55% |
| **74** | 4,0 | 8% | P0.6 R2 | 0,6 | 1,9 | 13 | 26 | 32% | dP0. 4R8 | 0,4 | 8,4 | 7 | 61 | DMS O | 55% |
| **75** | 4,0 | 8% | P0.6 R2 | 0,6 | 1,9 | 13 | 26 | 32% | dP0. 6R3 | 0,6 | 3,0 | 12 | 35 | DMS O | 55% |
| **76** | 4,0 | 8% | P0.6 R2 | 0,6 | 1,9 | 13 | 26 | 32% | dP0. 6R5 | 0,6 | 5,1 | 12 | 60 | DMS O | 55% |
| **77** | 4,0 | 8% | P0.6 R2 | 0,6 | 1,9 | 13 | 26 | 32% | dP1 R3 | 1,0 | 3,0 | 22 | 66 | DMS O | 55% |
| **78** | 4,0 | 8% | P0.6 R2 | 0,6 | 1,9 | 13 | 26 | 32% | dP1 R5 | 1,0 | 5,4 | 22 | 119 | DMS O | 55% |
| **79** | 4,0 | 8% | P0.6 R2 | 0,6 | 1,9 | 13 | 26 | 32% | dP2 R1 | 2,0 | 1,3 | 45 | 58 | DMS O | 55% |
| **80** | 4,0 | 8% | P0.6 R2 | 0,6 | 1,9 | 13 | 26 | 32% | dP2 R5 | 2,0 | 5,3 | 45 | 237 | DMS O | 55% |
| **81** | 4,0 | 8% | P0.6 R4 | 0,6 | 4,2 | 13 | 55 | 32% | dP0. 2R2 | 0,2 | 2,2 | 3 | 7 | DMS O | 55% |
| **82** | 4,0 | 8% | P0.6 R4 | 0,6 | 4,2 | 13 | 55 | 32% | dP0. 2R1 3 | 0,2 | 13,0 | 3 | 39 | DMS O | 55% |
| **83** | 4,0 | 8% | P0.6 R4 | 0,6 | 4,2 | 13 | 55 | 32% | dP0. 4R2 | 0,4 | 2,0 | 7 | 14 | DMS O | 55% |
| **84** | 4,0 | 8% | P0.6 R4 | 0,6 | 4,2 | 13 | 55 | 32% | dP0. 4R8 | 0,4 | 8,4 | 7 | 61 | DMS O | 55% |
| **85** | 4,0 | 8% | P0.6 R4 | 0,6 | 4,2 | 13 | 55 | 32% | dP0. 6R3 | 0,6 | 3,0 | 12 | 35 | DMS O | 55% |
| **86** | 4,0 | 8% | P0.6 R4 | 0,6 | 4,2 | 13 | 55 | 32% | dP0. 6R5 | 0,6 | 5,1 | 12 | 60 | DMS O | 55% |
| **87** | 4,0 | 8% | P0.6 R4 | 0,6 | 4,2 | 13 | 55 | 32% | dP1 R3 | 1,0 | 3,0 | 22 | 66 | DMS O | 55% |
| **88** | 4,0 | 8% | P0.6 R4 | 0,6 | 4,2 | 13 | 55 | 32% | dP1 R5 | 1,0 | 5,4 | 22 | 119 | DMS O | 55% |
| **89** | 4,0 | 8% | P0.6 R4 | 0,6 | 4,2 | 13 | 55 | 32% | dP2 R1 | 2,0 | 1,3 | 45 | 58 | DMS O | 55% |
| **90** | 4,0 | 8% | P0.6 R4 | 0,6 | 4,2 | 13 | 55 | 32% | dP2 R5 | 2,0 | 5,3 | 45 | 237 | DMS O | 55% |
| **91** | 4,0 | 8% | P1R 2 | 1,0 | 2,1 | 22 | 47 | 32% | dP0. 2R2 | 0,2 | 2,2 | 3 | 7 | DMS O | 55% |
| **92** | 4,0 | 8% | P1R 2 | 1,0 | 2,1 | 22 | 47 | 32% | dP0. 2R1 3 | 0,2 | 13,0 | 3 | 39 | DMS O | 55% |
| **93** | 4,0 | 8% | P1R 2 | 1,0 | 2,1 | 22 | 47 | 32% | dP0. 4R2 | 0,4 | 2,0 | 7 | 14 | DMS O | 55% |
| **94** | 4,0 | 8% | P1R 2 | 1,0 | 2,1 | 22 | 47 | 32% | dP0. 4R8 | 0,4 | 8,4 | 7 | 61 | DMS O | 55% |
| **95** | 4,0 | 8% | P1R 2 | 1,0 | 2,1 | 22 | 47 | 32% | dP0. 6R3 | 0,6 | 3,0 | 12 | 35 | DMS O | 55% |
| **96** | 4,0 | 8% | P1R 2 | 1,0 | 2,1 | 22 | 47 | 32% | dP0. 6R5 | 0,6 | 5,1 | 12 | 60 | DMS O | 55% |
| **97** | 4,0 | 8% | P1R 2 | 1,0 | 2,1 | 22 | 47 | 32% | dP1 R3 | 1,0 | 3,0 | 22 | 66 | DMS O | 55% |
| **98** | 4,0 | 8% | P1R 2 | 1,0 | 2,1 | 22 | 47 | 32% | dP1 R5 | 1,0 | 5,4 | 22 | 119 | DMS O | 55% |
| **99** | 4,0 | 8% | P1R 2 | 1,0 | 2,1 | 22 | 47 | 32% | dP2 R1 | 2,0 | 1,3 | 45 | 58 | DMS O | 55% |
| **100** | 4,0 | 8% | P1R 2 | 1,0 | 2,1 | 22 | 47 | 32% | dP2 R5 | 2,0 | 5,3 | 45 | 237 | DMS O | 55% |
| **101** | 4,0 | 8% | P1R 4 | 1,0 | 4,0 | 22 | 88 | 32% | dP0. 2R2 | 0,2 | 2,2 | 3 | 7 | DMS O | 55% |
| **102** | 4,0 | 8% | P1R 4 | 1,0 | 4,0 | 22 | 88 | 32% | dP0. 2R1 3 | 0,2 | 13,0 | 3 | 39 | DMS O | 55% |
| **103** | 4,0 | 8% | P1R 4 | 1,0 | 4,0 | 22 | 88 | 32% | dP0. 4R2 | 0,4 | 2,0 | 7 | 14 | DMS O | 55% |
| **104** | 4,0 | 8% | P1R 4 | 1,0 | 4,0 | 22 | 88 | 32% | dP0. 4R8 | 0,4 | 8,4 | 7 | 61 | DMS O | 55% |
| **105** | 4,0 | 8% | P1R 4 | 1,0 | 4,0 | 22 | 88 | 32% | dP0. 6R3 | 0,6 | 3,0 | 12 | 35 | DMS O | 55% |
| **106** | 4,0 | 8% | P1R 4 | 1,0 | 4,0 | 22 | 88 | 32% | dP0. 6R5 | 0,6 | 5,1 | 12 | 60 | DMS O | 55% |
| **107** | 4,0 | 8% | P1R 4 | 1,0 | 4,0 | 22 | 88 | 32% | dP1 R3 | 1,0 | 3,0 | 22 | 66 | DMS O | 55% |
| **108** | 4,0 | 8% | P1R 4 | 1,0 | 4,0 | 22 | 88 | 32% | dP1 R5 | 1,0 | 5,4 | 22 | 119 | DMS O | 55% |
| **109** | 4,0 | 8% | P1R 4 | 1,0 | 4,0 | 22 | 88 | 32% | dP2 R1 | 2,0 | 1,3 | 45 | 58 | DMS O | 55% |
| **110** | 4,0 | 8% | P1R 4 | 1,0 | 4,0 | 22 | 88 | 32% | dP2 R5 | 2,0 | 5,3 | 45 | 237 | DMS O | 55% |
| **111** | 4,0 | 8% | P2R 2 | 2,0 | 2,0 | 45 | 88 | 32% | dP0. 2R2 | 0,2 | 2,2 | 3 | 7 | DMS O | 55% |
| **112** | 4,0 | 8% | P2R 2 | 2,0 | 2,0 | 45 | 88 | 32% | dP0. 2R1 3 | 0,2 | 13,0 | 3 | 39 | DMS O | 55% |
| **113** | 4,0 | 8% | P2R 2 | 2,0 | 2,0 | 45 | 88 | 32% | dP0. 4R2 | 0,4 | 2,0 | 7 | 14 | DMS O | 55% |
| **114** | 4,0 | 8% | P2R 2 | 2,0 | 2,0 | 45 | 88 | 32% | dP0. 4R8 | 0,4 | 8,4 | 7 | 61 | DMS O | 55% |
| **115** | 4,0 | 8% | P2R 2 | 2,0 | 2,0 | 45 | 88 | 32% | dP0. 6R3 | 0,6 | 3,0 | 12 | 35 | DMS O | 55% |
| **116** | 4,0 | 8% | P2R 2 | 2,0 | 2,0 | 45 | 88 | 32% | dP0. 6R5 | 0,6 | 5,1 | 12 | 60 | DMS O | 55% |
| **117** | 4,0 | 8% | P2R 2 | 2,0 | 2,0 | 45 | 88 | 32% | dP1 R3 | 1,0 | 3,0 | 22 | 66 | DMS O | 55% |
| **118** | 4,0 | 8% | P2R 2 | 2,0 | 2,0 | 45 | 88 | 32% | dP1 R5 | 1,0 | 5,4 | 22 | 119 | DMS O | 55% |
| **119** | 4,0 | 8% | P2R 2 | 2,0 | 2,0 | 45 | 88 | 32% | dP2 R1 | 2,0 | 1,3 | 45 | 58 | DMS O | 55% |
| **120** | 4,0 | 8% | P2R 2 | 2,0 | 2,0 | 45 | 88 | 32% | dP2 R5 | 2,0 | 5,3 | 45 | 237 | DMS O | 55% |
| **121** | 4,0 | 8% | P2R 5 | 2,0 | 4,8 | 45 | 216 | 32% | dP0. 2R2 | 0,2 | 2,2 | 3 | 7 | DMS O | 55% |
| **122** | 4,0 | 8% | P2R 5 | 2,0 | 4,8 | 45 | 216 | 32% | dP0. 2R1 3 | 0,2 | 13,0 | 3 | 39 | DMS O | 55% |
| **123** | 4,0 | 8% | P2R 5 | 2,0 | 4,8 | 45 | 216 | 32% | dP0. 4R2 | 0,4 | 2,0 | 7 | 14 | DMS O | 55% |
| **124** | 4,0 | 8% | P2R 5 | 2,0 | 4,8 | 45 | 216 | 32% | dP0. 4R8 | 0,4 | 8,4 | 7 | 61 | DMS O | 55% |
| **125** | 4,0 | 8% | P2R 5 | 2,0 | 4,8 | 45 | 216 | 32% | dP0. 6R3 | 0,6 | 3,0 | 12 | 35 | DMS O | 55% |
| **126** | 4,0 | 8% | P2R 5 | 2,0 | 4,8 | 45 | 216 | 32% | dP0. 6R5 | 0,6 | 5,1 | 12 | 60 | DMS O | 55% |
| **127** | 4,0 | 8% | P2R 5 | 2,0 | 4,8 | 45 | 216 | 32% | dP1 R3 | 1,0 | 3,0 | 22 | 66 | DMS O | 55% |
| **128** | 4,0 | 8% | P2R 5 | 2,0 | 4,8 | 45 | 216 | 32% | dP1 R5 | 1,0 | 5,4 | 22 | 119 | DMS O | 55% |
| **129** | 4,0 | 8% | P2R 5 | 2,0 | 4,8 | 45 | 216 | 32% | dP2 R1 | 2,0 | 1,3 | 45 | 58 | DMS O | 55% |
| **130** | 4,0 | 8% | P2R 5 | 2,0 | 4,8 | 45 | 216 | 32% | dP2 R5 | 2,0 | 5,3 | 45 | 237 | DMS O | 55% |
| **131** | 4,0 | 8% | P3R 1 | 3,0 | 1,0 | 68 | 66 | 32% | dP0. 2R2 | 0,2 | 2,2 | 3 | 7 | DMS O | 55% |
| **132** | 4,0 | 8% | P3R 1 | 3,0 | 1,0 | 68 | 66 | 32% | dP0. 2R1 3 | 0,2 | 13,0 | 3 | 39 | DMS O | 55% |
| **133** | 4,0 | 8% | P3R 1 | 3,0 | 1,0 | 68 | 66 | 32% | dP0. 4R2 | 0,4 | 2,0 | 7 | 14 | DMS O | 55% |
| **134** | 4,0 | 8% | P3R 1 | 3,0 | 1,0 | 68 | 66 | 32% | dP0. 4R8 | 0,4 | 8,4 | 7 | 61 | DMS O | 55% |
| **135** | 4,0 | 8% | P3R 1 | 3,0 | 1,0 | 68 | 66 | 32% | dP0. 6R3 | 0,6 | 3,0 | 12 | 35 | DMS O | 55% |
| **136** | 4,0 | 8% | P3R 1 | 3,0 | 1,0 | 68 | 66 | 32% | dP0. 6R5 | 0,6 | 5,1 | 12 | 60 | DMS O | 55% |
| **137** | 4,0 | 8% | P3R 1 | 3,0 | 1,0 | 68 | 66 | 32% | dP1 R3 | 1,0 | 3,0 | 22 | 66 | DMS O | 55% |
| **138** | 4,0 | 8% | P3R 1 | 3,0 | 1,0 | 68 | 66 | 32% | dP1 R5 | 1,0 | 5,4 | 22 | 119 | DMS O | 55% |
| **139** | 4,0 | 8% | P3R 1 | 3,0 | 1,0 | 68 | 66 | 32% | dP2 R1 | 2,0 | 1,3 | 45 | 58 | DMS O | 55% |
| **140** | 4,0 | 8% | P3R 1 | 3,0 | 1,0 | 68 | 66 | 32% | dP2 R5 | 2,0 | 5,3 | 45 | 237 | DMS O | 55% |
| **141** | 4,0 | 8% | P3R 3 | 3,0 | 3,2 | 68 | 218 | 32% | dP0. 2R2 | 0,2 | 2,2 | 3 | 7 | DMS O | 55% |
| **142** | 4,0 | 8% | P3R 3 | 3,0 | 3,2 | 68 | 218 | 32% | dP0. 2R1 3 | 0,2 | 13,0 | 3 | 39 | DMS O | 55% |
| **143** | 4,0 | 8% | P3R 3 | 3,0 | 3,2 | 68 | 218 | 32% | dP0. 4R2 | 0,4 | 2,0 | 7 | 14 | DMS O | 55% |
| **144** | 4,0 | 8% | P3R 3 | 3,0 | 3,2 | 68 | 218 | 32% | dP0. 4R8 | 0,4 | 8,4 | 7 | 61 | DMS O | 55% |
| **145** | 4,0 | 8% | P3R 3 | 3,0 | 3,2 | 68 | 218 | 32% | dP0. 6R3 | 0,6 | 3,0 | 12 | 35 | DMS O | 55% |
| **146** | 4,0 | 8% | P3R 3 | 3,0 | 3,2 | 68 | 218 | 32% | dP0. 6R5 | 0,6 | 5,1 | 12 | 60 | DMS O | 55% |
| **147** | 4,0 | 8% | P3R 3 | 3,0 | 3,2 | 68 | 218 | 32% | dP1 R3 | 1,0 | 3,0 | 22 | 66 | DMS O | 55% |
| **148** | 4,0 | 8% | P3R 3 | 3,0 | 3,2 | 68 | 218 | 32% | dP1 R5 | 1,0 | 5,4 | 22 | 119 | DMS O | 55% |
| **149** | 4,0 | 8% | P3R 3 | 3,0 | 3,2 | 68 | 218 | 32% | dP2 R1 | 2,0 | 1,3 | 45 | 58 | DMS O | 55% |
| **150** | 4,0 | 8% | P3R 3 | 3,0 | 3,2 | 68 | 218 | 32% | dP2 R5 | 2,0 | 5,3 | 45 | 237 | DMS O | 55% |
| **151** | 4,0 | 8% | P6R 0.9 | 6,0 | 0,9 | 136 | 125 | 32% | dP0. 2R2 | 0,2 | 2,2 | 3 | 7 | DMS O | 55% |
| **152** | 4,0 | 8% | P6R 0.9 | 6,0 | 0,9 | 136 | 125 | 32% | dP0. 2R1 3 | 0,2 | 13,0 | 3 | 39 | DMS O | 55% |
| **153** | 4,0 | 8% | P6R 0.9 | 6,0 | 0,9 | 136 | 125 | 32% | dP0. 4R2 | 0,4 | 2,0 | 7 | 14 | DMS O | 55% |
| **154** | 4,0 | 8% | P6R 0.9 | 6,0 | 0,9 | 136 | 125 | 32% | dP0. 4R8 | 0,4 | 8,4 | 7 | 61 | DMS O | 55% |
| **155** | 4,0 | 8% | P6R 0.9 | 6,0 | 0,9 | 136 | 125 | 32% | dP0. 6R3 | 0,6 | 3,0 | 12 | 35 | DMS O | 55% |
| **156** | 4,0 | 8% | P6R 0.9 | 6,0 | 0,9 | 136 | 125 | 32% | dP0. 6R5 | 0,6 | 5,1 | 12 | 60 | DMS O | 55% |
| **157** | 4,0 | 8% | P6R 0.9 | 6,0 | 0,9 | 136 | 125 | 32% | dP1 R3 | 1,0 | 3,0 | 22 | 66 | DMS O | 55% |
| **158** | 4,0 | 8% | P6R 0.9 | 6,0 | 0,9 | 136 | 125 | 32% | dP1 R5 | 1,0 | 5,4 | 22 | 119 | DMS O | 55% |
| **159** | 4,0 | 8% | P6R 0.9 | 6,0 | 0,9 | 136 | 125 | 32% | dP2 R1 | 2,0 | 1,3 | 45 | 58 | DMS O | 55% |
| **160** | 4,0 | 8% | P6R 0.9 | 6,0 | 0,9 | 136 | 125 | 32% | dP2 R5 | 2,0 | 5,3 | 45 | 237 | DMS O | 55% |
| **161** | 4,0 | 8% | P6R 2 | 6,0 | 2,0 | 136 | 272 | 32% | dP0. 2R2 | 0,2 | 2,2 | 3 | 7 | DMS O | 55% |
| **162** | 4,0 | 8% | P6R 2 | 6,0 | 2,0 | 136 | 272 | 32% | dP0. 2R1 3 | 0,2 | 13,0 | 3 | 39 | DMS O | 55% |
| **163** | 4,0 | 8% | P6R 2 | 6,0 | 2,0 | 136 | 272 | 32% | dP0. 4R2 | 0,4 | 2,0 | 7 | 14 | DMS O | 55% |
| **164** | 4,0 | 8% | P6R 2 | 6,0 | 2,0 | 136 | 272 | 32% | dP0. 4R8 | 0,4 | 8,4 | 7 | 61 | DMS O | 55% |
| **165** | 4,0 | 8% | P6R 2 | 6,0 | 2,0 | 136 | 272 | 32% | dP0. 6R3 | 0,6 | 3,0 | 12 | 35 | DMS O | 55% |
| **166** | 4,0 | 8% | P6R 2 | 6,0 | 2,0 | 136 | 272 | 32% | dP0. 6R5 | 0,6 | 5,1 | 12 | 60 | DMS O | 55% |
| **167** | 4,0 | 8% | P6R 2 | 6,0 | 2,0 | 136 | 272 | 32% | dP1 R3 | 1,0 | 3,0 | 22 | 66 | DMS O | 55% |
| **168** | 4,0 | 8% | P6R 2 | 6,0 | 2,0 | 136 | 272 | 32% | dP1 R5 | 1,0 | 5,4 | 22 | 119 | DMS O | 55% |
| **169** | 4,0 | 8% | P6R 2 | 6,0 | 2,0 | 136 | 272 | 32% | dP2 R1 | 2,0 | 1,3 | 45 | 58 | DMS O | 55% |
| **170** | 4,0 | 8% | P6R 2 | 6,0 | 2,0 | 136 | 272 | 32% | dP2 R5 | 2,0 | 5,3 | 45 | 237 | DMS O | 55% |
| **171** | 4,0 | 8% | P0.2 R6 | 0,2 | 5,9 | 4 | 24 | 32% | dP0. 2R6 | 0,2 | 5,8 | 3 | 17 | DMS O | 55% |
| **172** | 4,0 | 8% | P0.2 R6 | 0,2 | 5,9 | 4 | 24 | 32% | dP0. 4R6 | 0,4 | 5,8 | 7 | 42 | DMS O | 55% |
| **173** | 4,0 | 8% | P0.2 R6 | 0,2 | 5,9 | 4 | 24 | 32% | dP0. 6R5 | 0,6 | 4,6 | 12 | 54 | DMS O | 55% |
| **174** | 4,0 | 8% | P0.2 R6 | 0,2 | 5,9 | 4 | 24 | 32% | dP1 R4 | 1,0 | 4,0 | 22 | 89 | DMS O | 55% |
| **175** | 4,0 | 8% | P0.2 R6 | 0,2 | 5,9 | 4 | 24 | 32% | dP2 R3 | 2,0 | 2,8 | 45 | 125 | DMS O | 55% |
| **176** | 4,0 | 8% | P0.2 R22 | 0,2 | 22,3 | 4 | 89 | 32% | dP0. 2R6 | 0,2 | 5,8 | 3 | 17 | DMS O | 55% |
| **177** | 4,0 | 8% | P0.2 R22 | 0,2 | 22,3 | 4 | 89 | 32% | dP0. 4R6 | 0,4 | 5,8 | 7 | 42 | DMS O | 55% |
| **178** | 4,0 | 8% | P0.2 R22 | 0,2 | 22,3 | 4 | 89 | 32% | dP0. 6R5 | 0,6 | 4,6 | 12 | 54 | DMS O | 55% |
| **179** | 4,0 | 8% | P0.2 R22 | 0,2 | 22,3 | 4 | 89 | 32% | dP1 R4 | 1,0 | 4,0 | 22 | 89 | DMS O | 55% |
| **180** | 4,0 | 8% | P0.2 R22 | 0,2 | 22,3 | 4 | 89 | 32% | dP2 R3 | 2,0 | 2,8 | 45 | 125 | DMS O | 55% |
| **181** | 4,0 | 8% | P0.4 R5 | 0,4 | 4,7 | 9 | 41 | 32% | dP0. 2R6 | 0,2 | 5,8 | 3 | 17 | DMS O | 55% |
| **182** | 4,0 | 8% | P0.4 R5 | 0,4 | 4,7 | 9 | 41 | 32% | dP0. 4R6 | 0,4 | 5,8 | 7 | 42 | DMS O | 55% |
| **183** | 4,0 | 8% | P0.4 R5 | 0,4 | 4,7 | 9 | 41 | 32% | dP0. 6R5 | 0,6 | 4,6 | 12 | 54 | DMS O | 55% |
| **184** | 4,0 | 8% | P0.4 R5 | 0,4 | 4,7 | 9 | 41 | 32% | dP1 R4 | 1,0 | 4,0 | 22 | 89 | DMS O | 55% |
| **185** | 4,0 | 8% | P0.4 R5 | 0,4 | 4,7 | 9 | 41 | 32% | dP2 R3 | 2,0 | 2,8 | 45 | 125 | DMS O | 55% |
| **186** | 4,0 | 8% | P0.4 R8 | 0,4 | 7,7 | 9 | 67 | 32% | dP0. 2R6 | 0,2 | 5,8 | 3 | 17 | DMS O | 55% |
| **187** | 4,0 | 8% | P0.4 R8 | 0,4 | 7,7 | 9 | 67 | 32% | dP0. 4R6 | 0,4 | 5,8 | 7 | 42 | DMS O | 55% |
| **188** | 4,0 | 8% | P0.4 R8 | 0,4 | 7,7 | 9 | 67 | 32% | dP0. 6R5 | 0,6 | 4,6 | 12 | 54 | DMS O | 55% |
| **189** | 4,0 | 8% | P0.4 R8 | 0,4 | 7,7 | 9 | 67 | 32% | dP1 R4 | 1,0 | 4,0 | 22 | 89 | DMS O | 55% |
| **190** | 4,0 | 8% | P0.4 R8 | 0,4 | 7,7 | 9 | 67 | 32% | dP2 R3 | 2,0 | 2,8 | 45 | 125 | DMS O | 55% |
| **191** | 4,0 | 8% | P0.6 R2 | 0,6 | 1,9 | 13 | 26 | 32% | dP0. 2R6 | 0,2 | 5,8 | 3 | 17 | DMS O | 55% |
| **192** | 4,0 | 8% | P0.6 R2 | 0,6 | 1,9 | 13 | 26 | 32% | dP0. 4R6 | 0,4 | 5,8 | 7 | 42 | DMS O | 55% |
| **193** | 4,0 | 8% | P0.6 R2 | 0,6 | 1,9 | 13 | 26 | 32% | dP0. 6R5 | 0,6 | 4,6 | 12 | 54 | DMS O | 55% |
| **194** | 4,0 | 8% | P0.6 R2 | 0,6 | 1,9 | 13 | 26 | 32% | dP1 R4 | 1,0 | 4,0 | 22 | 89 | DMS O | 55% |
| **195** | 4,0 | 8% | P0.6 R2 | 0,6 | 1,9 | 13 | 26 | 32% | dP2 R3 | 2,0 | 2,8 | 45 | 125 | DMS O | 55% |
| **196** | 4,0 | 8% | P0.6 R4 | 0,6 | 4,2 | 13 | 55 | 32% | dP0. 2R6 | 0,2 | 5,8 | 3 | 17 | DMS O | 55% |
| **197** | 4,0 | 8% | P0.6 R4 | 0,6 | 4,2 | 13 | 55 | 32% | dP0. 4R6 | 0,4 | 5,8 | 7 | 42 | DMS O | 55% |
| **198** | 4,0 | 8% | P0.6 R4 | 0,6 | 4,2 | 13 | 55 | 32% | dP0. 6R5 | 0,6 | 4,6 | 12 | 54 | DMS O | 55% |
| **199** | 4,0 | 8% | P0.6 R4 | 0,6 | 4,2 | 13 | 55 | 32% | dP1 R4 | 1,0 | 4,0 | 22 | 89 | DMS O | 55% |
| **200** | 4,0 | 8% | P0.6 R4 | 0,6 | 4,2 | 13 | 55 | 32% | dP2 R3 | 2,0 | 2,8 | 45 | 125 | DMS O | 55% |
| **201** | 4,0 | 8% | P1R 2 | 1,0 | 2,1 | 22 | 47 | 32% | dP0. 2R6 | 0,2 | 5,8 | 3 | 17 | DMS O | 55% |
| **202** | 4,0 | 8% | P1R 2 | 1,0 | 2,1 | 22 | 47 | 32% | dP0. 4R6 | 0,4 | 5,8 | 7 | 42 | DMS O | 55% |
| **203** | 4,0 | 8% | P1R 2 | 1,0 | 2,1 | 22 | 47 | 32% | dP0. 6R5 | 0,6 | 4,6 | 12 | 54 | DMS O | 55% |
| **204** | 4,0 | 8% | P1R 2 | 1,0 | 2,1 | 22 | 47 | 32% | dP1 R4 | 1,0 | 4,0 | 22 | 89 | DMS O | 55% |
| **205** | 4,0 | 8% | P1R 2 | 1,0 | 2,1 | 22 | 47 | 32% | dP2 R3 | 2,0 | 2,8 | 45 | 125 | DMS O | 55% |
| **206** | 4,0 | 8% | P1R 4 | 1,0 | 4,0 | 22 | 88 | 32% | dP0. 2R6 | 0,2 | 5,8 | 3 | 17 | DMS O | 55% |
| **207** | 4,0 | 8% | P1R 4 | 1,0 | 4,0 | 22 | 88 | 32% | dP0. 4R6 | 0,4 | 5,8 | 7 | 42 | DMS O | 55% |
| **208** | 4,0 | 8% | P1R 4 | 1,0 | 4,0 | 22 | 88 | 32% | dP0. 6R5 | 0,6 | 4,6 | 12 | 54 | DMS O | 55% |
| **209** | 4,0 | 8% | P1R 4 | 1,0 | 4,0 | 22 | 88 | 32% | dP1 R4 | 1,0 | 4,0 | 22 | 89 | DMS O | 55% |
| **210** | 4,0 | 8% | P1R 4 | 1,0 | 4,0 | 22 | 88 | 32% | dP2 R3 | 2,0 | 2,8 | 45 | 125 | DMS O | 55% |
| **211** | 4,0 | 8% | P2R 2 | 2,0 | 2,0 | 45 | 88 | 32% | dP0. 2R6 | 0,2 | 5,8 | 3 | 17 | DMS O | 55% |
| **212** | 4,0 | 8% | P2R 2 | 2,0 | 2,0 | 45 | 88 | 32% | dP0. 4R6 | 0,4 | 5,8 | 7 | 42 | DMS O | 55% |
| **213** | 4,0 | 8% | P2R 2 | 2,0 | 2,0 | 45 | 88 | 32% | dP0. 6R5 | 0,6 | 4,6 | 12 | 54 | DMS O | 55% |
| **214** | 4,0 | 8% | P2R 2 | 2,0 | 2,0 | 45 | 88 | 32% | dP1 R4 | 1,0 | 4,0 | 22 | 89 | DMS O | 55% |
| **215** | 4,0 | 8% | P2R 2 | 2,0 | 2,0 | 45 | 88 | 32% | dP2 R3 | 2,0 | 2,8 | 45 | 125 | DMS O | 55% |
| **216** | 4,0 | 8% | P2R 5 | 2,0 | 4,8 | 45 | 216 | 32% | dP0. 2R6 | 0,2 | 5,8 | 3 | 17 | DMS O | 55% |
| **217** | 4,0 | 8% | P2R 5 | 2,0 | 4,8 | 45 | 216 | 32% | dP0. 4R6 | 0,4 | 5,8 | 7 | 42 | DMS O | 55% |
| **218** | 4,0 | 8% | P2R 5 | 2,0 | 4,8 | 45 | 216 | 32% | dP0. 6R5 | 0,6 | 4,6 | 12 | 54 | DMS O | 55% |
| **219** | 4,0 | 8% | P2R 5 | 2,0 | 4,8 | 45 | 216 | 32% | dP1 R4 | 1,0 | 4,0 | 22 | 89 | DMS O | 55% |
| **220** | 4,0 | 8% | P2R 5 | 2,0 | 4,8 | 45 | 216 | 32% | dP2 R3 | 2,0 | 2,8 | 45 | 125 | DMS O | 55% |
| **221** | 4,0 | 8% | P3R 1 | 3,0 | 1,0 | 68 | 66 | 32% | dP0. 2R6 | 0,2 | 5,8 | 3 | 17 | DMS O | 55% |
| **222** | 4,0 | 8% | P3R 1 | 3,0 | 1,0 | 68 | 66 | 32% | dP0. 4R6 | 0,4 | 5,8 | 7 | 42 | DMS O | 55% |
| **223** | 4,0 | 8% | P3R 1 | 3,0 | 1,0 | 68 | 66 | 32% | dP0. 6R5 | 0,6 | 4,6 | 12 | 54 | DMS O | 55% |
| **224** | 4,0 | 8% | P3R 1 | 3,0 | 1,0 | 68 | 66 | 32% | dP1 R4 | 1,0 | 4,0 | 22 | 89 | DMS O | 55% |
| **225** | 4,0 | 8% | P3R 1 | 3,0 | 1,0 | 68 | 66 | 32% | dP2 R3 | 2,0 | 2,8 | 45 | 125 | DMS O | 55% |
| **226** | 4,0 | 8% | P3R 3 | 3,0 | 3,2 | 68 | 218 | 32% | dP0. 2R6 | 0,2 | 5,8 | 3 | 17 | DMS O | 55% |
| **227** | 4,0 | 8% | P3R 3 | 3,0 | 3,2 | 68 | 218 | 32% | dP0. 4R6 | 0,4 | 5,8 | 7 | 42 | DMS O | 55% |
| **228** | 4,0 | 8% | P3R 3 | 3,0 | 3,2 | 68 | 218 | 32% | dP0. 6R5 | 0,6 | 4,6 | 12 | 54 | DMS O | 55% |
| **229** | 4,0 | 8% | P3R 3 | 3,0 | 3,2 | 68 | 218 | 32% | dP1 R4 | 1,0 | 4,0 | 22 | 89 | DMS O | 55% |
| **230** | 4,0 | 8% | P3R 3 | 3,0 | 3,2 | 68 | 218 | 32% | dP2 R3 | 2,0 | 2,8 | 45 | 125 | DMS O | 55% |
| **231** | 4,0 | 8% | P6R 0.9 | 6,0 | 0,9 | 136 | 125 | 32% | dP0. 2R6 | 0,2 | 5,8 | 3 | 17 | DMS O | 55% |
| **232** | 4,0 | 8% | P6R 0.9 | 6,0 | 0,9 | 136 | 125 | 32% | dP0. 4R6 | 0,4 | 5,8 | 7 | 42 | DMS O | 55% |
| **233** | 4,0 | 8% | P6R 0.9 | 6,0 | 0,9 | 136 | 125 | 32% | dP0. 6R5 | 0,6 | 4,6 | 12 | 54 | DMS O | 55% |
| **234** | 4,0 | 8% | P6R 0.9 | 6,0 | 0,9 | 136 | 125 | 32% | dP1 R4 | 1,0 | 4,0 | 22 | 89 | DMS O | 55% |
| **235** | 4,0 | 8% | P6R 0.9 | 6,0 | 0,9 | 136 | 125 | 32% | dP2 R3 | 2,0 | 2,8 | 45 | 125 | DMS O | 55% |
| **236** | 4,0 | 8% | P6R 2 | 6,0 | 2,0 | 136 | 272 | 32% | dP0. 2R6 | 0,2 | 5,8 | 3 | 17 | DMS O | 55% |
| **237** | 4,0 | 8% | P6R 2 | 6,0 | 2,0 | 136 | 272 | 32% | dP0. 4R6 | 0,4 | 5,8 | 7 | 42 | DMS O | 55% |
| **238** | 4,0 | 8% | P6R 2 | 6,0 | 2,0 | 136 | 272 | 32% | dP0. 6R5 | 0,6 | 4,6 | 12 | 54 | DMS O | 55% |
| **239** | 4,0 | 8% | P6R 2 | 6,0 | 2,0 | 136 | 272 | 32% | dP1 R4 | 1,0 | 4,0 | 22 | 89 | DMS O | 55% |
| **240** | 4,0 | 8% | P6R 2 | 6,0 | 2,0 | 136 | 272 | 32% | dP2 R3 | 2,0 | 2,8 | 45 | 125 | DMS O | 55% |
| **241** | 4,0 | 8% | P0.2 R14 | 0,2 | 14,5 | 4 | 58 | 32% | dP0. 2R2 | 0,2 | 2,2 | 3 | 7 | DMS O | 55% |
| **242** | 4,0 | 8% | P0.2 R14 | 0,2 | 14,5 | 4 | 58 | 32% | dP0. 2R1 3 | 0,2 | 13,0 | 3 | 39 | DMS O | 55% |
| **243** | 4,0 | 8% | P0.2 R14 | 0,2 | 14,5 | 4 | 58 | 32% | dP0. 4R2 | 0,4 | 2,0 | 7 | 14 | DMS O | 55% |
| **244** | 4,0 | 8% | P0.2 R14 | 0,2 | 14,5 | 4 | 58 | 32% | dP0. 4R8 | 0,4 | 8,4 | 7 | 61 | DMS O | 55% |
| **245** | 4,0 | 8% | P0.2 R14 | 0,2 | 14,5 | 4 | 58 | 32% | dP0. 6R3 | 0,6 | 3,0 | 12 | 35 | DMS O | 55% |
| **246** | 4,0 | 8% | P0.2 R14 | 0,2 | 14,5 | 4 | 58 | 32% | dP0. 6R5 | 0,6 | 5,1 | 12 | 60 | DMS O | 55% |
| **247** | 4,0 | 8% | P0.2 R14 | 0,2 | 14,5 | 4 | 58 | 32% | dP1 R3 | 1,0 | 3,0 | 22 | 66 | DMS O | 55% |
| **248** | 4,0 | 8% | P0.2 R14 | 0,2 | 14,5 | 4 | 58 | 32% | dP1 R5 | 1,0 | 5,4 | 22 | 119 | DMS O | 55% |
| **249** | 4,0 | 8% | P0.2 R14 | 0,2 | 14,5 | 4 | 58 | 32% | dP2 R1 | 2,0 | 1,3 | 45 | 58 | DMS O | 55% |
| **250** | 4,0 | 8% | P0.2 R14 | 0,2 | 14,5 | 4 | 58 | 32% | dP2 R5 | 2,0 | 5,3 | 45 | 237 | DMS O | 55% |
| **251** | 4,0 | 8% | P0.6 R3 | 0,6 | 3,0 | 13 | 40 | 32% | dP0. 2R2 | 0,2 | 2,2 | 3 | 7 | DMS O | 55% |
| **252** | 4,0 | 8% | P0.6 R3 | 0,6 | 3,0 | 13 | 40 | 32% | dP0. 2R1 3 | 0,2 | 13,0 | 3 | 39 | DMS O | 55% |
| **253** | 4,0 | 8% | P0.6 R3 | 0,6 | 3,0 | 13 | 40 | 32% | dP0. 4R2 | 0,4 | 2,0 | 7 | 14 | DMS O | 55% |
| **254** | 4,0 | 8% | P0.6 R3 | 0,6 | 3,0 | 13 | 40 | 32% | dP0. 4R8 | 0,4 | 8,4 | 7 | 61 | DMS O | 55% |
| **255** | 4,0 | 8% | P0.6 R3 | 0,6 | 3,0 | 13 | 40 | 32% | dP0. 6R3 | 0,6 | 3,0 | 12 | 35 | DMS O | 55% |
| **256** | 4,0 | 8% | P0.6 R3 | 0,6 | 3,0 | 13 | 40 | 32% | dP0. 6R5 | 0,6 | 5,1 | 12 | 60 | DMS O | 55% |
| **257** | 4,0 | 8% | P0.6 R3 | 0,6 | 3,0 | 13 | 40 | 32% | dP1 R3 | 1,0 | 3,0 | 22 | 66 | DMS O | 55% |
| **258** | 4,0 | 8% | P0.6 R3 | 0,6 | 3,0 | 13 | 40 | 32% | dP1 R5 | 1,0 | 5,4 | 22 | 119 | DMS O | 55% |
| **259** | 4,0 | 8% | P0.6 R3 | 0,6 | 3,0 | 13 | 40 | 32% | dP2 R1 | 2,0 | 1,3 | 45 | 58 | DMS O | 55% |
| **260** | 4,0 | 8% | P0.6 R3 | 0,6 | 3,0 | 13 | 40 | 32% | dP2 R5 | 2,0 | 5,3 | 45 | 237 | DMS O | 55% |
| **261** | 4,0 | 8% | P1R 3 | 1,0 | 3,1 | 22 | 68 | 32% | dP0. 2R2 | 0,2 | 2,2 | 3 | 7 | DMS O | 55% |
| **262** | 4,0 | 8% | P1R 3 | 1,0 | 3,1 | 22 | 68 | 32% | dP0. 2R1 3 | 0,2 | 13,0 | 3 | 39 | DMS O | 55% |
| **263** | 4,0 | 8% | P1R 3 | 1,0 | 3,1 | 22 | 68 | 32% | dP0. 4R2 | 0,4 | 2,0 | 7 | 14 | DMS O | 55% |
| **264** | 4,0 | 8% | P1R 3 | 1,0 | 3,1 | 22 | 68 | 32% | dP0. 4R8 | 0,4 | 8,4 | 7 | 61 | DMS O | 55% |
| **265** | 4,0 | 8% | P1R 3 | 1,0 | 3,1 | 22 | 68 | 32% | dP0. 6R3 | 0,6 | 3,0 | 12 | 35 | DMS O | 55% |
| **266** | 4,0 | 8% | P1R 3 | 1,0 | 3,1 | 22 | 68 | 32% | dP0. 6R5 | 0,6 | 5,1 | 12 | 60 | DMS O | 55% |
| **267** | 4,0 | 8% | P1R 3 | 1,0 | 3,1 | 22 | 68 | 32% | dP1 R3 | 1,0 | 3,0 | 22 | 66 | DMS O | 55% |
| **268** | 4,0 | 8% | P1R 3 | 1,0 | 3,1 | 22 | 68 | 32% | dP1 R5 | 1,0 | 5,4 | 22 | 119 | DMS O | 55% |
| **269** | 4,0 | 8% | P1R 3 | 1,0 | 3,1 | 22 | 68 | 32% | dP2 R1 | 2,0 | 1,3 | 45 | 58 | DMS O | 55% |
| **270** | 4,0 | 8% | P1R 3 | 1,0 | 3,1 | 22 | 68 | 32% | dP2 R5 | 2,0 | 5,3 | 45 | 237 | DMS O | 55% |
| **271** | 4,0 | 8% | P2R 3 | 2,0 | 3,5 | 45 | 157 | 32% | dP0. 2R2 | 0,2 | 2,2 | 3 | 7 | DMS O | 55% |
| **272** | 4,0 | 8% | P2R 3 | 2,0 | 3,5 | 45 | 157 | 32% | dP0. 2R1 3 | 0,2 | 13,0 | 3 | 39 | DMS O | 55% |
| **273** | 4,0 | 8% | P2R 3 | 2,0 | 3,5 | 45 | 157 | 32% | dP0. 4R2 | 0,4 | 2,0 | 7 | 14 | DMS O | 55% |
| **274** | 4,0 | 8% | P2R 3 | 2,0 | 3,5 | 45 | 157 | 32% | dP0. 4R8 | 0,4 | 8,4 | 7 | 61 | DMS O | 55% |
| **275** | 4,0 | 8% | P2R 3 | 2,0 | 3,5 | 45 | 157 | 32% | dP0. 6R3 | 0,6 | 3,0 | 12 | 35 | DMS O | 55% |
| **276** | 4,0 | 8% | P2R 3 | 2,0 | 3,5 | 45 | 157 | 32% | dP0. 6R5 | 0,6 | 5,1 | 12 | 60 | DMS O | 55% |
| **277** | 4,0 | 8% | P2R 3 | 2,0 | 3,5 | 45 | 157 | 32% | dP1 R3 | 1,0 | 3,0 | 22 | 66 | DMS O | 55% |
| **278** | 4,0 | 8% | P2R 3 | 2,0 | 3,5 | 45 | 157 | 32% | dP1 R5 | 1,0 | 5,4 | 22 | 119 | DMS O | 55% |
| **279** | 4,0 | 8% | P2R 3 | 2,0 | 3,5 | 45 | 157 | 32% | dP2 R1 | 2,0 | 1,3 | 45 | 58 | DMS O | 55% |
| **280** | 4,0 | 8% | P2R 3 | 2,0 | 3,5 | 45 | 157 | 32% | dP2 R5 | 2,0 | 5,3 | 45 | 237 | DMS O | 55% |
| **281** | 4,0 | 8% | P3R 2 | 3,0 | 2,3 | 68 | 154 | 32% | dP0. 2R2 | 0,2 | 2,2 | 3 | 7 | DMS O | 55% |
| **282** | 4,0 | 8% | P3R 2 | 3,0 | 2,3 | 68 | 154 | 32% | dP0. 2R1 3 | 0,2 | 13,0 | 3 | 39 | DMS O | 55% |
| **283** | 4,0 | 8% | P3R 2 | 3,0 | 2,3 | 68 | 154 | 32% | dP0. 4R2 | 0,4 | 2,0 | 7 | 14 | DMS O | 55% |
| **284** | 4,0 | 8% | P3R 2 | 3,0 | 2,3 | 68 | 154 | 32% | dP0. 4R8 | 0,4 | 8,4 | 7 | 61 | DMS O | 55% |
| **285** | 4,0 | 8% | P3R 2 | 3,0 | 2,3 | 68 | 154 | 32% | dP0. 6R3 | 0,6 | 3,0 | 12 | 35 | DMS O | 55% |
| **286** | 4,0 | 8% | P3R 2 | 3,0 | 2,3 | 68 | 154 | 32% | dP0. 6R5 | 0,6 | 5,1 | 12 | 60 | DMS O | 55% |
| **287** | 4,0 | 8% | P3R 2 | 3,0 | 2,3 | 68 | 154 | 32% | dP1 R3 | 1,0 | 3,0 | 22 | 66 | DMS O | 55% |
| **288** | 4,0 | 8% | P3R 2 | 3,0 | 2,3 | 68 | 154 | 32% | dP1 R5 | 1,0 | 5,4 | 22 | 119 | DMS O | 55% |
| **289** | 4,0 | 8% | P3R 2 | 3,0 | 2,3 | 68 | 154 | 32% | dP2 R1 | 2,0 | 1,3 | 45 | 58 | DMS O | 55% |
| **290** | 4,0 | 8% | P3R 2 | 3,0 | 2,3 | 68 | 154 | 32% | dP2 R5 | 2,0 | 5,3 | 45 | 237 | DMS O | 55% |
| **291** | 4,0 | 8% | P6R 2 | 6,0 | 1,6 | 136 | 218 | 32% | dP0. 2R2 | 0,2 | 2,2 | 3 | 7 | DMS O | 55% |
| **292** | 4,0 | 8% | P6R 2 | 6,0 | 1,6 | 136 | 218 | 32% | dP0. 2R1 3 | 0,2 | 13,0 | 3 | 39 | DMS O | 55% |
| **293** | 4,0 | 8% | P6R 2 | 6,0 | 1,6 | 136 | 218 | 32% | dP0. 4R2 | 0,4 | 2,0 | 7 | 14 | DMS O | 55% |
| **294** | 4,0 | 8% | P6R 2 | 6,0 | 1,6 | 136 | 218 | 32% | dP0. 4R8 | 0,4 | 8,4 | 7 | 61 | DMS O | 55% |
| **295** | 4,0 | 8% | P6R 2 | 6,0 | 1,6 | 136 | 218 | 32% | dP0. 6R3 | 0,6 | 3,0 | 12 | 35 | DMS O | 55% |
| **296** | 4,0 | 8% | P6R 2 | 6,0 | 1,6 | 136 | 218 | 32% | dP0. 6R5 | 0,6 | 5,1 | 12 | 60 | DMS O | 55% |
| **297** | 4,0 | 8% | P6R 2 | 6,0 | 1,6 | 136 | 218 | 32% | dP1 R3 | 1,0 | 3,0 | 22 | 66 | DMS O | 55% |
| **298** | 4,0 | 8% | P6R 2 | 6,0 | 1,6 | 136 | 218 | 32% | dP1 R5 | 1,0 | 5,4 | 22 | 119 | DMS O | 55% |
| **299** | 4,0 | 8% | P6R 2 | 6,0 | 1,6 | 136 | 218 | 32% | dP2 R1 | 2,0 | 1,3 | 45 | 58 | DMS O | 55% |
| **300** | 4,0 | 8% | P6R 2 | 6,0 | 1,6 | 136 | 218 | 32% | dP2 R5 | 2,0 | 5,3 | 45 | 237 | DMS O | 55% |
| **301** | 0,0 | 40% | P2R 3 | 2,0 | 3,5 | 45 | 157 | 0% | dP0. 4R6 | 0,4 | 5,8 | 7 | 42 | DMS O | 55% |
| **302** | 0,05 | 38% | P2R 3 | 2,0 | 3,5 | 45 | 157 | 2% | dP0. 4R6 | 0,4 | 5,8 | 7 | 42 | DMS O | 55% |
| **303** | 0,11 | 36% | P2R 3 | 2,0 | 3,5 | 45 | 157 | 4% | dP0. 4R6 | 0,4 | 5,8 | 7 | 42 | DMS O | 55% |
| **304** | 0,25 | 32% | P2R 3 | 2,0 | 3,5 | 45 | 157 | 8% | dP0. 4R6 | 0,4 | 5,8 | 7 | 42 | DMS O | 55% |
| **305** | 1,00 | 20% | P2R 3 | 2,0 | 3,5 | 45 | 157 | 20% | dP0. 4R6 | 0,4 | 5,8 | 7 | 42 | DMS O | 55% |
| **306** | 4,0 | 8% | P2R 3 | 2,0 | 3,5 | 45 | 157 | 32% | dP0. 4R6 | 0,4 | 5,8 | 7 | 42 | DMS O | 55% |
| **307** | 9,0 | 4% | P2R 3 | 2,0 | 3,5 | 45 | 157 | 36% | dP0. 4R6 | 0,4 | 5,8 | 7 | 42 | DMS O | 55% |
| **308** | 19,0 | 2% | P2R 3 | 2,0 | 3,5 | 45 | 157 | 38% | dP0. 4R6 | 0,4 | 5,8 | 7 | 42 | DMS O | 55% |
| **309** | ∞ | 0% | P2R 3 | 2,0 | 3,5 | 45 | 157 | 40% | dP0. 4R6 | 0,4 | 5,8 | 7 | 42 | DMS O | 55% |
| **310** | 0,0 | 40% | P2R 3 | 2,0 | 3,5 | 45 | 157 | 0% | dP0. 6R5 | 0,6 | 4,6 | 12 | 54 | DMS O | 55% |
| **311** | 0,05 | 38% | P2R 3 | 2,0 | 3,5 | 45 | 157 | 2% | dP0. 6R5 | 0,6 | 4,6 | 12 | 54 | DMS O | 55% |
| **312** | 0,11 | 36% | P2R 3 | 2,0 | 3,5 | 45 | 157 | 4% | dP0. 6R5 | 0,6 | 4,6 | 12 | 54 | DMS O | 55% |
| **313** | 0,25 | 32% | P2R 3 | 2,0 | 3,5 | 45 | 157 | 8% | dP0. 6R5 | 0,6 | 4,6 | 12 | 54 | DMS O | 55% |
| **314** | 1,00 | 20% | P2R 3 | 2,0 | 3,5 | 45 | 157 | 20% | dP0. 6R5 | 0,6 | 4,6 | 12 | 54 | DMS O | 55% |
| **315** | 4,0 | 8% | P2R 3 | 2,0 | 3,5 | 45 | 157 | 32% | dP0. 6R5 | 0,6 | 4,6 | 12 | 54 | DMS O | 55% |
| **316** | 9,0 | 4% | P2R 3 | 2,0 | 3,5 | 45 | 157 | 36% | dP0. 6R5 | 0,6 | 4,6 | 12 | 54 | DMS O | 55% |
| **317** | 19,0 | 2% | P2R 3 | 2,0 | 3,5 | 45 | 157 | 38% | dP0. 6R5 | 0,6 | 4,6 | 12 | 54 | DMS O | 55% |
| **318** | ∞ | 0% | P2R 3 | 2,0 | 3,5 | 45 | 157 | 40% | dP0. 6R5 | 0,6 | 4,6 | 12 | 54 | DMS O | 55% |
| **319** | 0,0 | 40% | P0.4 R8 | 0,4 | 7,7 | 9 | 67 | 0% | dP0. 4R8 | 0,4 | 8,4 | 7 | 61 | DMS O | 55% |
| **320** | 0,05 | 38% | P0.4 R8 | 0,4 | 7,7 | 9 | 67 | 2% | dP0. 4R8 | 0,4 | 8,4 | 7 | 61 | DMS O | 55% |
| **321** | 0,11 | 36% | P0.4 R8 | 0,4 | 7,7 | 9 | 67 | 4% | dP0. 4R8 | 0,4 | 8,4 | 7 | 61 | DMS O | 55% |
| **322** | 0,25 | 32% | P0.4 R8 | 0,4 | 7,7 | 9 | 67 | 8% | dP0. 4R8 | 0,4 | 8,4 | 7 | 61 | DMS O | 55% |
| **323** | 1,00 | 20% | P0.4 R8 | 0,4 | 7,7 | 9 | 67 | 20% | dP0. 4R8 | 0,4 | 8,4 | 7 | 61 | DMS O | 55% |
| **324** | 4,0 | 8% | P0.4 R8 | 0,4 | 7,7 | 9 | 67 | 32% | dP0. 4R8 | 0,4 | 8,4 | 7 | 61 | DMS O | 55% |
| **325** | 9,0 | 4% | P0.4 R8 | 0,4 | 7,7 | 9 | 67 | 36% | dP0. 4R8 | 0,4 | 8,4 | 7 | 61 | DMS O | 55% |
| **326** | 19,0 | 2% | P0.4 R8 | 0,4 | 7,7 | 9 | 67 | 38% | dP0. 4R8 | 0,4 | 8,4 | 7 | 61 | DMS O | 55% |
| **327** | ∞ | 0% | P0.4 R8 | 0,4 | 7,7 | 9 | 67 | 40% | dP0. 4R8 | 0,4 | 8,4 | 7 | 61 | DMS O | 55% |
| **328** | 0,0 | 40% | P1R 2 | 1,0 | 2,1 | 22 | 47 | 0% | dP0. 6R5 | 0,6 | 5,1 | 12 | 60 | DMS O | 55% |
| **329** | 0,05 | 38% | P1R 2 | 1,0 | 2,1 | 22 | 47 | 2% | dP0. 6R5 | 0,6 | 5,1 | 12 | 60 | DMS O | 55% |
| **330** | 0,11 | 36% | P1R 2 | 1,0 | 2,1 | 22 | 47 | 4% | dP0. 6R5 | 0,6 | 5,1 | 12 | 60 | DMS O | 55% |
| **331** | 0,25 | 32% | P1R 2 | 1,0 | 2,1 | 22 | 47 | 8% | dP0. 6R5 | 0,6 | 5,1 | 12 | 60 | DMS O | 55% |
| **332** | 1,00 | 20% | P1R 2 | 1,0 | 2,1 | 22 | 47 | 20% | dP0. 6R5 | 0,6 | 5,1 | 12 | 60 | DMS O | 55% |
| **333** | 4,0 | 8% | P1R 2 | 1,0 | 2,1 | 22 | 47 | 32% | dP0. 6R5 | 0,6 | 5,1 | 12 | 60 | DMS O | 55% |
| **334** | 9,0 | 4% | P1R 2 | 1,0 | 2,1 | 22 | 47 | 36% | dP0. 6R5 | 0,6 | 5,1 | 12 | 60 | DMS O | 55% |
| **335** | 19,0 | 2% | P1R 2 | 1,0 | 2,1 | 22 | 47 | 38% | dP0. 6R5 | 0,6 | 5,1 | 12 | 60 | DMS O | 55% |
| **336** | ∞ | 0% | P1R 2 | 1,0 | 2,1 | 22 | 47 | 40% | dP0. 6R5 | 0,6 | 5,1 | 12 | 60 | DMS O | 55% |
| **337** | 0,0 | 40% | P2R 5 | 2,0 | 4,8 | 45 | 216 | 0 % | dP0. 2R1 3 | 0,2 | 13,0 | 3 | 39 | DMS O | 55% |
| **338** | 0,05 | 38% | P2R 5 | 2,0 | 4,8 | 45 | 216 | 2% | dP0. 2R1 3 | 0,2 | 13,0 | 3 | 39 | DMS O | 55% |
| **339** | 0,11 | 36% | P2R 5 | 2,0 | 4,8 | 45 | 216 | 4% | dP0. 2R1 3 | 0,2 | 13,0 | 3 | 39 | DMS O | 55% |
| **340** | 0,25 | 32% | P2R 5 | 2,0 | 4,8 | 45 | 216 | 8% | dP0. 2R1 3 | 0,2 | 13,0 | 3 | 39 | DMS O | 55% |
| **341** | 1,00 | 20% | P2R 5 | 2,0 | 4,8 | 45 | 216 | 20% | dP0. 2R1 3 | 0,2 | 13,0 | 3 | 39 | DMS O | 55% |
| **342** | 4,0 | 8% | P2R 5 | 2,0 | 4,8 | 45 | 216 | 32% | dP0. 2R1 3 | 0,2 | 13,0 | 3 | 39 | DMS O | 55% |
| **343** | 9,0 | 4% | P2R 5 | 2,0 | 4,8 | 45 | 216 | 36% | dP0. 2R1 3 | 0,2 | 13,0 | 3 | 39 | DMS O | 55% |
| **344** | 19,0 | 2% | P2R 5 | 2,0 | 4,8 | 45 | 216 | 38% | dP0. 2R1 3 | 0,2 | 13,0 | 3 | 39 | DMS O | 55% |
| **345** | ∞ | 0% | P2R 5 | 2,0 | 4,8 | 45 | 216 | 40% | dP0. 2R1 3 | 0,2 | 13,0 | 3 | 39 | DMS O | 55% |

### Example 5-Buprenorphine's formulations preparation (not according to the invention)

The formulations described herein were based on organic solution of polymers prepared as in Example 1, containing as the drug, buprenorphine. Typically, 0.4 grams of polymers, corresponding to a mix of a diblock copolymer and a triblock copolymer in defined mass ratio, were dissolved in 0.5 grams of dimethyl sulfoxide at room temperature overnight under constant magnetic stirring. The next day, 100 mg of buprenorphine was added to the polymer solution and stirred until complete dissolution. The formulations were loaded in a syringe before use.

Three different formulations were selected for *in vivo* experiments. The composition of these formulations is shown in Table 3 below. The formulations were injected subcutaneously in the interscapular space of male rats (200-250 gr) at a final dose of 100 mg/kg of buprenorphine. Blood samples were withdrawn periodically and analyzed for buprenorphine concentrations by LC/MS/MS.

The formulations are shown in Table 3 below.

**Table 3**

| | | **Triblock copolymer (TB)** | | | | | | **Diblock copolymer (DB)** | | | | | | **Solvent** | |
|---|---|---|---|---|---|---|---|---|---|---|---|---|---|---|---|
| **Exp n°** | **Rati o DB/ TB** | **% (w/w)** | **Co de** | **PEG (kDa )** | **Rati o (LA/ EO)** | **DP-PEG** | **DP-PLA** | **% (w/w )** | **Cod e** | **PEG (kDa )** | **Rati o (LA/ EO)** | **DP-PEG** | **DP-PLA** | **Nam e** | **% (w/w )** |
| **1** | 4,0 | 1 0,0% | P0 .4 R8 | 0,4 | 7,7 | 9 | 70 | 40,0 % | dP0. 4R1 0 | 0,35 | 9,8 | 8 | 78 | DM SO | 40,0 % |
| **2** | 4,0 | 10,0% | P2 R2 | 2 | 2,2 | 45 | 101 | 40,0 % | dP0. 4R1 0 | 0,35 | 9,8 | 8 | 78 | DM SO | 40,0 % |
| **3** | 4,0 | 10,0% | P2 R3 | 2 | 3,3 | 45 | 150 | 40,0 % | dP0. 4R1 0 | 0,35 | 9,8 | 8 | 78 | DM SO | 40,0 % |
| **4** | 4,0 | 10,0% | P2 R4 | 2 | 4,3 | 45 | 195 | 40,0 % | dP0. 4R1 0 | 0,35 | 9,8 | 8 | 78 | DM SO | 40,0 % |
| **5** | 4,0 | 10,0% | P0 .4 R8 | 0,4 | 7,7 | 9 | 70 | 40,0 % | dP1 R4 | 1 | 4,2 | 23 | 95 | DM SO | 40,0 % |
| **6** | 4,0 | 10,0% | P2 R2 | 2 | 2,2 | 45 | 101 | 40,0 % | dP1 R4 | 1 | 4,2 | 23 | 95 | DM SO | 40,0 % |
| **7** | 4,0 | 10,0% | P2 R3 | 2 | 3,3 | 45 | 150 | 40,0 % | dP1 R4 | 1 | 4,2 | 23 | 95 | DM SO | 40,0 % |
| **8** | 4,0 | 10,0% | P2 R4 | 2 | 4,3 | 45 | 195 | 40,0 % | dP1 R4 | 1 | 4,2 | 23 | 95 | DM SO | 40,0 % |
| **9** | 4,0 | 10,0% | P0 .4 R8 | 0,4 | 7,7 | 9 | 70 | 40,0 % | dP1 R5 | 1 | 5,4 | 23 | 123 | DM SO | 40,0 % |
| **10** | 4,0 | 10,0% | P2 R2 | 2 | 2,2 | 45 | 101 | 40,0 % | dP1 R5 | 1 | 5,4 | 23 | 123 | DM SO | 40,0 % |
| **11** | 4,0 | 10,0% | P2 R3 | 2 | 3,3 | 45 | 150 | 40,0 % | dP1 R5 | 1 | 5,4 | 23 | 123 | DM SO | 40,0 % |
| **12** | 4,0 | 10,0% | P2 R4 | 2 | 4,3 | 45 | 195 | 40,0 % | dP1 R5 | 1 | 5,4 | 23 | 123 | DM SO | 40,0 % |
| **13** | 4,0 | 10,0% | P0 .4 R8 | 0,4 | 7,7 | 9 | 70 | 40,0 % | dP2 R3 | 2 | 2,7 | 45 | 120 | DM SO | 40,0 % |
| **14** | 4,0 | 10,0% | P2 R2 | 2 | 2,2 | 45 | 101 | 40,0 % | dP2 R3 | 2 | 2,7 | 45 | 120 | DM SO | 40,0 % |
| **15** | 4,0 | 10,0% | P2 R3 | 2 | 3,3 | 45 | 150 | 40,0 % | dP2 R3 | 2 | 2,7 | 45 | 120 | DM SO | 40,0 % |
| **16** | 4,0 | 10,0% | P2 R4 | 2 | 4,3 | 45 | 195 | 40,0 % | dP2 R3 | 2 | 2,7 | 45 | 120 | DM SO | 40,0 % |
| **17** | 4,0 | 10,0% | P0 .4 R8 | 0,4 | 7,7 | 9 | 70 | 40,0 % | dP2 R4 | 2 | 4,1 | 45 | 186 | DM SO | 40,0 % |
| **18** | 4,0 | 10,0% | P2 R2 | 2 | 2,2 | 45 | 101 | 40,0 % | dP2 R4 | 2 | 4,1 | 45 | 186 | DM SO | 40,0 % |
| **19** | 4,0 | 10,0% | P2 R3 | 2 | 3,3 | 45 | 150 | 40,0 % | dP2 R4 | 2 | 4,1 | 45 | 186 | DM SO | 40,0 % |
| **20** | 4,0 | 10,0% | P2 R4 | 2 | 4,3 | 45 | 195 | 40,0 % | dP2 R4 | 2 | 4,1 | 45 | 186 | DM SO | 40,0 % |
| **21** | 4,0 | 10,0% | P0 .4 R8 | 0,4 | 7,7 | 9 | 70 | 40,0 % | dP2 R5 | 2 | 5,3 | 45 | 241 | DM SO | 40,0 % |
| **22** | 4,0 | 10,0% | P2 R2 | 2 | 2,2 | 45 | 101 | 40,0 % | dP2 R5 | 2 | 5,3 | 45 | 241 | DM SO | 40,0 % |
| **23** | 4,0 | 10,0% | P2 R3 | 2 | 3,3 | 45 | 150 | 40,0 % | dP2 R5 | 2 | 5,3 | 45 | 241 | DM SO | 40,0 % |
| **24** | 4,0 | 10,0% | P2 R4 | 2 | 4,3 | 45 | 195 | 40,0 % | dP2 R5 | 2 | 5,3 | 45 | 241 | DM SO | 40,0 % |
| **26** | 4,0 | 9,0% | P0 .4 R8 | 0,4 | 7,7 | 9 | 70 | 36,0 % | dP0. 4R1 0 | 0,35 | 9,8 | 8 | 78 | DM SO | 45,0 % |
| **27** | 4,0 | 9,0% | P2 R2 | 2 | 2,2 | 45 | 101 | 36,0 % | dP0. 4R1 0 | 0,35 | 9,8 | 8 | 78 | DM SO | 45,0 % |
| **28** | 4,0 | 9,0% | P2 R3 | 2 | 3,3 | 45 | 150 | 36,0 % | dP0. 4R1 0 | 0,35 | 9,8 | 8 | 78 | DM SO | 45,0 % |
| **29** | 4,0 | 9,0% | P0 .4 R8 | 0,4 | 7,7 | 9 | 70 | 36,0 % | dP1 R4 | 1 | 4,2 | 23 | 95 | DM SO | 45,0 % |
| **30** | 4,0 | 9,0% | P2 R2 | 2 | 2,2 | 45 | 101 | 36,0 % | dP1 R4 | 1 | 4,2 | 23 | 95 | DM SO | 45,0 % |
| **31** | 4,0 | 9,0% | P2 R2 | 2 | 2,2 | 45 | 101 | 36,0 % | dP2 R3 | 2 | 2,7 | 45 | 120 | DM SO | 45,0 % |
| **32** | 4,0 | 8,0% | P0 .4 R8 | 0,4 | 7,7 | 9 | 70 | 32,0 % | dP0. 4R1 0 | 0,35 | 9,8 | 8 | 78 | DM SO | 50,0 % |
| **33** | 4,0 | 8,0% | P2 R2 | 2 | 2,2 | 45 | 101 | 32,0 % | dP0. 4R1 0 | 0,35 | 9,8 | 8 | 78 | DM SO | 50,0 % |
| **34** | 4,0 | 8,0% | P2 R3 | 2 | 3,3 | 45 | 150 | 32,0 % | dP0. 4R1 0 | 0,35 | 9,8 | 8 | 78 | DM SO | 50,0 % |
| **35** | 4,0 | 8,0% | P0 .4 R8 | 0,4 | 7,7 | 9 | 70 | 32,0 % | dP1 R4 | 1 | 4,2 | 23 | 95 | DM SO | 50,0 % |
| **36** | 4,0 | 8,0% | P2 R2 | 2 | 2,2 | 45 | 101 | 32,0 % | dP1 R4 | 1 | 4,2 | 23 | 95 | DM SO | 50,0 % |
| **37** | 4,0 | 8,0% | P2 R2 | 2 | 2,2 | 45 | 101 | 32,0 % | dP2 R3 | 2 | 2,7 | 45 | 120 | DM SO | 50,0 % |
| **38** | 4,0 | 10,0% | P0 .4 R8 | 0,4 | 7,7 | 9 | 70 | 40,0 % | dP1 R3 | 1 | 2,7 | 23 | 61 | DM SO | 40,0 % |
| **39** | 4,0 | 10,0% | P2 R2 | 2 | 2,2 | 45 | 101 | 40,0 % | dP1 R3 | 1 | 2,7 | 23 | 61 | DM SO | 40,0 % |
| **40** | 4,0 | 10,0% | P2 R3 | 2 | 3,3 | 45 | 150 | 40,0 % | dP1 R3 | 1 | 2,7 | 23 | 61 | DM SO | 40,0 % |
| **41** | 4,0 | 10,0% | P2 R4 | 2 | 4,3 | 45 | 195 | 40,0 % | dP1 R3 | 1 | 2,7 | 23 | 61 | DM SO | 40,0 % |
| **42** | 4,0 | 9,0% | P0 .4 R8 | 0,4 | 7,7 | 9 | 70 | 36,0 % | dP1 R3 | 1 | 2,7 | 23 | 61 | DM SO | 45,0 % |
| **43** | 4,0 | 9,0% | P2 R2 | 2 | 2,2 | 45 | 101 | 36,0 % | dP1 R3 | 1 | 2,7 | 23 | 61 | DM SO | 45,0 % |
| **44** | 4,0 | 9,0% | P2 R3 | 2 | 3,3 | 45 | 150 | 36,0 % | dP1 R3 | 1 | 2,7 | 23 | 61 | DM SO | 45,0 % |
| **45** | 4,0 | 9,0% | P2 R4 | 2 | 4,3 | 45 | 195 | 36,0 % | dP1 R3 | 1 | 2,7 | 23 | 61 | DM SO | 45,0 % |
| **46** | 4,0 | 8,0% | P0 .4 R8 | 0,4 | 7,7 | 9 | 70 | 32,0 % | dP1 R3 | 1 | 2,7 | 23 | 61 | DM SO | 50,0 % |
| **47** | 4,0 | 8,0% | P2 R2 | 2 | 2,2 | 45 | 101 | 32,0 % | dP1 R3 | 1 | 2,7 | 23 | 61 | DM SO | 50,0 % |
| **48** | 4,0 | 8,0% | P2 R3 | 2 | 3,3 | 45 | 150 | 32,0 % | dP1 R3 | 1 | 2,7 | 23 | 61 | DM SO | 50,0 % |
| **49** | 4,0 | 8,0% | P2 R4 | 2 | 4,3 | 45 | 195 | 32,0 % | dP1 R3 | 1 | 2,7 | 23 | 61 | DM SO | 50,0 % |
| **51** | 4,0 | 10,0% | P2 R2 | 2 | 2,2 | 45 | 101 | 40,0 % | dP0. 4R8 | 0,35 | 7,9 | 8 | 63 | DM SO | 40,0 % |
| **52** | 4,0 | 10,0% | P2 R2 | 2 | 2,2 | 45 | 101 | 40,0 % | dP0. 4R5 | 0,35 | 4,9 | 8 | 39 | DM SO | 40,0 % |
| **53** | 4,0 | 10,0% | P2 R2 | 2 | 2,2 | 45 | 101 | 40,0 % | dP1 R2 | 1 | 2,1 | 23 | 48 | DM SO | 40,0 % |
| **54** | 4,0 | 1 0,0% | P2 R2 | 2 | 2,2 | 45 | 101 | 40,0 % | dP2 R0. 8 | 2 | 0,8 | 45 | 34 | DM SO | 40,0 % |
| **55** | 4,0 | 10,0% | P2 R2 | 2 | 2,2 | 45 | 101 | 40,0 % | dP2 R2 | 2 | 1,5 | 45 | 68 | DM SO | 40,0 % |
| **56** | 4,0 | 10,0% | P0 .4 R8 | 0,4 | 7,7 | 9 | 70 | 40,0 % | dP0. 4R8 | 0,35 | 7,9 | 8 | 63 | DM SO | 40,0 % |
| **57** | 4,0 | 10,0% | P0 .4 R8 | 0,4 | 7,7 | 9 | 70 | 40,0 % | dP0. 4R5 | 0,35 | 4,9 | 8 | 39 | DM SO | 40,0 % |
| **58** | 4,0 | 10,0% | P0 .4 R8 | 0,4 | 7,7 | 9 | 70 | 40,0 % | dP1 R2 | 1 | 2,1 | 23 | 48 | DM SO | 40,0 % |
| **59** | 4,0 | 10,0% | P0 .4 R8 | 0,4 | 7,7 | 9 | 70 | 40,0 % | dP2 R0. 8 | 2 | 0,8 | 45 | 34 | DM SO | 40,0 % |
| **60** | 4,0 | 10,0% | P0 .4 R8 | 0,4 | 7,7 | 9 | 70 | 40,0 % | dP2 R2 | 2 | 1,5 | 45 | 68 | DM SO | 40,0 % |
| **61** | 4,0 | 10,0% | P0 .4 R8 | 0,4 | 7,7 | 9 | 70 | 40,0 % | dP0. 4R1 0 | 0,35 | 9,8 | 8 | 78 | DE GM EE | 40,0 % |
| **62** | 4,0 | 10,0% | P2 R4 | 2 | 4,3 | 45 | 195 | 40,0 % | dP0. 4R1 0 | 0,35 | 9,8 | 8 | 78 | DE GM EE | 40,0 % |
| **63** | 4,0 | 10,0% | P0 .4 R8 | 0,4 | 7,7 | 9 | 70 | 40,0 % | dP1 R3 | 1 | 2,7 | 23 | 61 | DE GM EE | 40,0 % |
| **64** | 4,0 | 10,0% | P2 R4 | 2 | 4,3 | 45 | 195 | 40,0 % | dP1 R3 | 1 | 2,7 | 23 | 61 | DE GM EE | 40,0 % |
| **65** | 4,0 | 10,0% | P0 .4 R8 | 0,4 | 7,7 | 9 | 70 | 40,0 % | dP2 R4 | 2 | 4,1 | 45 | 186 | DE GM EE | 40,0 % |
| **66** | 4,0 | 10,0% | P2 R4 | 2 | 4,3 | 45 | 195 | 40,0 % | dP2 R4 | 2 | 4,1 | 45 | 186 | DE GM EE | 40,0 % |
| **67** | 4,0 | 10,0% | P0 .4 R8 | 0,4 | 7,7 | 9 | 70 | 40,0 % | dP0. 4R1 0 | 0,35 | 9,8 | 8 | 78 | Digl yme | 40,0 % |
| **68** | 4,0 | 10,0% | P2 R4 | 2 | 4,3 | 45 | 195 | 40,0 % | dP0. 4R1 0 | 0,35 | 9,8 | 8 | 78 | Digl yme | 40,0 % |
| **69** | 4,0 | 10,0% | P0 .4 R8 | 0,4 | 7,7 | 9 | 70 | 40,0 % | dP1 R3 | 1 | 2,7 | 23 | 61 | Digl yme | 40,0 % |
| **70** | 4,0 | 10,0% | P2 R4 | 2 | 4,3 | 45 | 195 | 40,0 % | dP1 R3 | 1 | 2,7 | 23 | 61 | Digl vme | 40,0 % |
| **71** | 4,0 | 10,0% | P0 .4 R8 | 0,4 | 7,7 | 9 | 70 | 40,0 % | dP2 R4 | 2 | 4,1 | 45 | 186 | Digl yme | 40,0 % |
| **72** | 4,0 | 10,0% | P2 R4 | 2 | 4,3 | 45 | 195 | 40,0 % | dP2 R4 | 2 | 4,1 | 45 | 186 | Digl yme | 40,0 % |
| **73** | 4,0 | 9,0% | P0 .4 R8 | 0,4 | 7,7 | 9 | 70 | 36,0 % | dP1 R2 | 1 | 2,1 | 23 | 48 | DM SO | 45,0 % |
| **74** | 4,0 | 8,0% | P0 .4 R8 | 0,4 | 7,7 | 9 | 70 | 32,0 % | dP1 R2 | 1 | 2,1 | 23 | 48 | DM SO | 50,0 % |
| **75** | 3,0 | 10,0% | P0 .4 R8 | 0,4 | 7,7 | 9 | 70 | 30,0 % | dP1 R2 | 1 | 2,1 | 23 | 48 | DM SO | 50,0 % |
| **76** | 6,0 | 5,7% | P0 .4 R8 | 0,4 | 7,7 | 9 | 70 | 34,3 % | dP1 R2 | 1 | 2,1 | 23 | 48 | DM SO | 50,0 % |
| **77** | 4,0 | 8,0% | P0 .4 R5 | 0,4 | 4,7 | 9 | 43 | 32,0 % | dP1 R2 | 1 | 2,1 | 23 | 48 | DM SO | 50,0 % |
| **78** | 4,0 | 8,0% | P1 R2 | 1 | 2,1 | 23 | 48 | 32,0 % | dP1 R2 | 1 | 2,1 | 23 | 48 | DM SO | 50,0 % |
| **79** | 4,0 | 8,0% | P1 R3 | 1 | 2,8 | 23 | 64 | 32,0 % | dP1 R2 | 1 | 2,1 | 23 | 48 | DM SO | 50,0 % |
| **80** | 4,0 | 8,0% | P0 .4 R5 | 0,4 | 4,7 | 9 | 43 | 32,0 % | dP1 R3 | 1 | 2,7 | 23 | 61 | DM SO | 50,0 % |
| **81** | 4,0 | 8,0% | P1 R2 | 1 | 2,1 | 23 | 48 | 32,0 % | dP1 R3 | 1 | 2,7 | 23 | 61 | DM SO | 50,0 % |
| **82** | 4,0 | 8,0% | P1 R3 | 1 | 2,8 | 23 | 64 | 32,0 % | dP1 R3 | 1 | 2,7 | 23 | 61 | DM SO | 50,0 % |
| **83** | 4,0 | 8,0% | P0 .4 R5 | 0,4 | 4,7 | 9 | 43 | 32,0 % | dP0. 4R5 | 0,35 | 4,9 | 8 | 39 | DM SO | 50,0 % |
| **84** | 4,0 | 8,0% | P1 R2 | 1 | 2,1 | 23 | 48 | 32,0 % | dP0. 4R5 | 0,35 | 4,9 | 8 | 39 | DM SO | 50,0 % |
| **85** | 4,0 | 8,0% | P1 R3 | 1 | 2,8 | 23 | 64 | 32,0 % | dP0. 4R5 | 0,35 | 4,9 | 8 | 39 | DM SO | 50,0 % |
| **86** | 4,0 | 10,0% | P2 R4 | 2 | 4,3 | 45 | 195 | 40,0 % | dP2 R4 | 2 | 4,1 | 45 | 186 | DE GM EE | 40,0 % |
| **87** | 4,0 | 8,0% | P0 .4 R5 | 0,4 | 4,7 | 9 | 43 | 32,0 % | dP1 R2 | 1 | 2,1 | 23 | 48 | DE GM EE | 50,0 % |
| **88** | 4,0 | 8,0% | P1 R2 | 1 | 2,1 | 23 | 48 | 32,0 % | dP1 R2 | 1 | 2,1 | 23 | 48 | DE GM EE | 50,0 % |
| **89** | 4,0 | 8,0% | P1 R3 | 1 | 2,8 | 23 | 64 | 32,0 % | dP1 R2 | 1 | 2,1 | 23 | 48 | DE GM EE | 50,0 % |
| **90** | 4,0 | 10,0% | P2 R4 | 2 | 4,3 | 45 | 195 | 40,0 % | dP2 R4 | 2 | 4,1 | 45 | 186 | Digl yme | 40,0 % |
| **91** | 4,0 | 8,0% | P0 .4 R5 | 0,4 | 4,7 | 9 | 43 | 32,0 % | dP1 R2 | 1 | 2,1 | 23 | 48 | Digl yme | 50,0 % |
| **92** | 4,0 | 8,0% | P1 R2 | 1 | 2,1 | 23 | 48 | 32,0 % | dP1 R2 | 1 | 2,1 | 23 | 48 | Digl yme | 50,0 % |
| **93** | 4,0 | 8,0% | P1 R3 | 1 | 2,8 | 23 | 64 | 32,0 % | dP1 R2 | 1 | 2,1 | 23 | 48 | Digl yme | 50,0 % |
| **95** | 4,0 | 10,0% | P2 R4 | 2 | 4,3 | 45 | 195 | 40,0 % | dP2 R4 | 2 | 4,1 | 45 | 186 | DM SO | 40,0 % |
| **96** | 4,0 | 8,0% | P0 .4 R5 | 0,4 | 4,7 | 9 | 43 | 32,0 % | dP1 R2 | 1 | 2,1 | 23 | 48 | DM SO | 50,0 % |
| **97** | 4,0 | 8,0% | P1 R2 | 1 | 2,1 | 23 | 48 | 32,0 % | dP1 R2 | 1 | 2,1 | 23 | 48 | DM SO | 50,0 % |
| **98** | 4,0 | 8,0% | P1 R3 | 1 | 2,8 | 23 | 64 | 32,0 % | dP1 R2 | 1 | 2,1 | 23 | 48 | DM SO | 50,0 % |

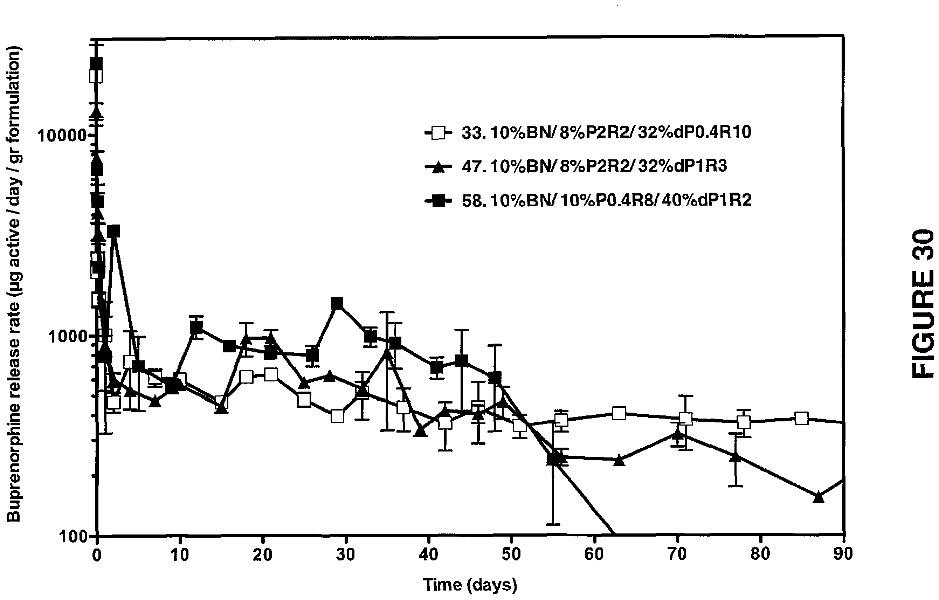

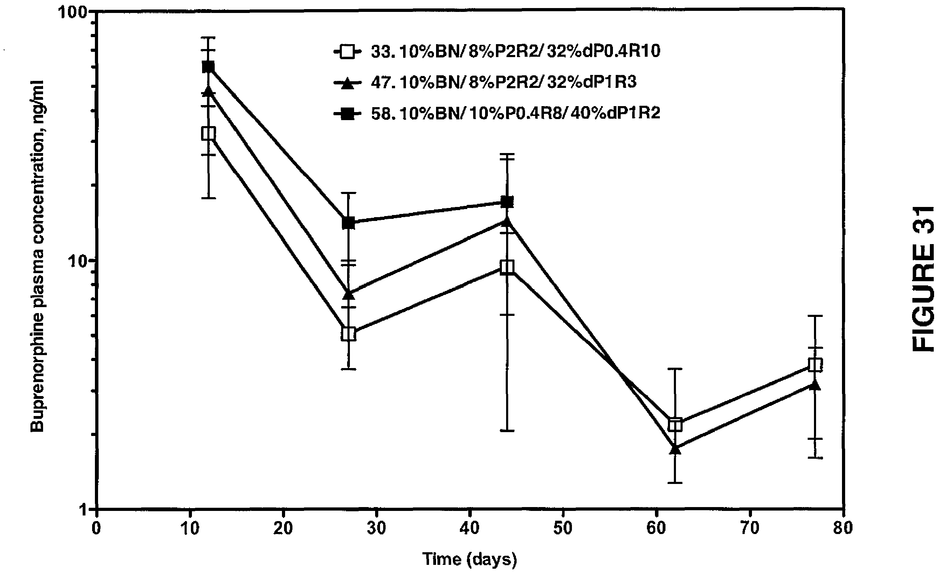

The results of these formulations are illustrated in Figures 30 and 31.

### Example 6-Risperidone's formulations preparation (not according to the invention)

The formulations described herein were based on organic solution of polymers prepared as in Example 1, containing as the drug, risperidone. Typically, 0.4 grams of polymers, corresponding to a mix of a diblock copolymer and a triblock copolymer in defined mass ratio, were dissolved in 0.5 grams of dimethyl sulfoxide at room temperature overnight under constant magnetic stirring. The next day, 100 mg of risperidone was added to the polymer solution and stirred. The formulations were loaded in a syringe before use.

Three different formulations were selected for *in vivo* experiments. The composition of these formulations is shown in Table 4 below. The formulations were injected subcutaneously in the interscapular space of male rats (300 gr) at a final dose of 21 mg/kg of risperidone. Blood samples were withdrawn periodically and analyzed for risperidone and 9-OH risperidone concentrations by LC/MS/MS.

The formulations are shown in Table 4 below.

**Table 4**

| | | **Risp** | **Triblock copolymer (TB)** | | | | | | **Diblock copolymer (DB)** | | | | | | **Solvent** | |
|---|---|---|---|---|---|---|---|---|---|---|---|---|---|---|---|---|
| **Exp n°** | **Rati o DB/T B** | **% (w/w)** | **% (w/w)** | **Co de** | **PE G (kD a)** | **Rati o (LA/ EO)** | **DPPEG** | **DPPLA** | **% (w/w )** | **Cod e** | **PEG (kDa )** | **Rati o (LA/ EO)** | **DPPEG** | **DPPLA** | **Name** | **% (w/w)** |
| 5 | 1,5 | 2,5% | 16,0% | P2 R3 | 2 | 3,5 | 45 | 158, 6 | 24,0 % | dP2 R3 | 2 | 2,7 | 45 | 122, 7 | DMS O | 57,5% |
| **6** | 1,5 | 2,5% | 16,0% | P2 R2 | 2 | 2,3 | 45 | 104, 5 | 24,0 % | dP1 R3 | 1 | 2,7 | 23 | 61,4 | DMS O | 57,5% |
| **10** | 1,5 | 5,0% | 16,0% | P2 R2 | 2 | 2,3 | 45 | 104, 5 | 24,0 % | dP2 R3 | 2 | 2,7 | 45 | 122, 7 | DMS O | 55,0% |
| **11** | 1,5 | 5,0% | 16,0% | P2 R3 | 2 | 3,5 | 45 | 158, 6 | 24,0 % | dP2 R3 | 2 | 2,7 | 45 | 122, 7 | DMS O | 55,0% |
| **12** | 1,5 | 5,0% | 16,0% | P2 R2 | 2 | 2,3 | 45 | 104, 5 | 24,0 % | dP1 R3 | 1 | 2,7 | 23 | 61,4 | DMS O | 55,0% |
| **16** | 0,7 | 5,0% | 24,0% | P2 R3 | 2 | 3,5 | 45 | 158, 6 | 16,0 % | dP0 .4R 5 | 0,35 | 4,9 | 8 | 39,0 | DMS O | 55,0% |
| **17** | 1,5 | 5,0% | 16,0% | P3 R2 | 3 | 2,3 | 68 | 156, 8 | 24,0 % | dP2 R3 | 2 | 2,9 | 45 | 131, 8 | DMS O | 55,0% |
| **19** | 1,5 | 5,0% | 16,0% | P3 R3 | 3 | 3,2 | 68 | 218, 2 | 24,0 % | dP2 R3 | 2 | 2,7 | 45 | 122, 7 | DMS O | 55,0% |
| **20** | 1,5 | 5,0% | 16,0% | P1 R4 | 1 | 3,8 | 23 | 86,4 | 24,0 % | dP2 R3 | 2 | 2,9 | 45 | 131, 8 | DMS O | 55,0% |
| **21** | 0,7 | 5,0% | 24,0% | P1 R4 | 1 | 3,8 | 23 | 86,4 | 16,0 % | dP0 .4R 5 | 0,35 | 4,9 | 8 | 39,0 | DMS O | 55,0% |
| **22** | 1,5 | 10,0% | 16,0% | P2 R2 | 2 | 2,3 | 45 | 104, 5 | 24,0 % | dP2 R3 | 2 | 2,7 | 45 | 122, 7 | DMS O | 50,0% |
| **23** | 1,5 | 10,0% | 1 6,0% | P2 R3 | 2 | 3,5 | 45 | 158, 6 | 24,0 % | dP2 R3 | 2 | 2,7 | 45 | 122, 7 | DMS O | 50,0% |
| **25** | 0,7 | 10,0% | 24,0% | P2 R3 | 2 | 3,5 | 45 | 158, 6 | 16,0 % | dP0 .4R 5 | 0,35 | 4,9 | 8 | 39,0 | DMS O | 50,0% |
| **26** | 1,5 | 10,0% | 16,0% | P3 R3 | 3 | 3,2 | 68 | 218, 2 | 24,0 % | dP2 R3 | 2 | 2,7 | 45 | 122, 7 | DMS O | 50,0% |
| **27** | 1,5. | 10,0% | 16,0% | P1 R4 | 1 | 3,8 | 23 | 86,4 | 24,0 % | dP2 R3 | 2 | 2,9 | 45 | 131, 8 | DMS O | 50,0% |
| **28** | 0,7 | 5,0% | 18,0% | P1 R4 | 1 | 3,8 | 23 | 86,4 | 12,0 % | dP0 .4R 5 | 0,35 | 4,9 | 8 | 39,0 | DMS O | 65,0% |
| **29** | 0,7 | 10,0% | 24,0% | P1 R4 | 1 | 3,8 | 23 | 86,4 | 16,0 % | dP0 .4R 5 | 0,35 | 4,9 | 8 | 39,0 | DMS O | 60,0% |
| **30** | 0,7 | 10,0% | 18,0% | P1 R4 | 1 | 3,8 | 23 | 86,4 | 12,0 % | dP0 .4R 5 | 0,35 | 4,9 | 8 | 39,0 | DMS O | 60,0% |
| **31** | 0,7 | 1 0,0% | 18,0% | P2 R3 | 2 | 3,5 | 45 | 158, 6 | 12,0 % | dP0 .4R 5 | 0,35 | 4,9 | 8 | 39,0 | DMS O | 60,0% |
| **32** | 1,5 | 10,0% | 12,0% | P1 R4 | 1 | 3,8 | 23 | 86,4 | 18,0 % | dP2 R3 | 2 | 2,9 | 45 | 131, 8 | DMS O | 60,0% |
| **33** | 1,5 | 10,0% | 12,0% | P3 R3 | 3 | 3,2 | 68 | 218, 2 | 18,0 % | dP2 R3 | 2 | 2,7 | 45 | 122, 7 | DMS O | 60,0% |
| **34** | 0,7 | 15,0% | 18,0% | P1 R4 | 1 | 3,8 | 23 | 86,4 | 12,0 % | dP0 .4R 5 | 0,35 | 4,9 | 8 | 39,0 | DMS O | 55,0% |
| **35** | 1,5 | 15,0% | 12,0% | P2 R2 | 2 | 2,3 | 45 | 104, 5 | 18,0 % | dP2 R3 | 2 | 2,7 | 45 | 122, 7 | DMS O | 55,0% |
| **36** | 0,7 | 15,0% | 1 8,0% | P2 R3 | 2 | 3,5 | 45 | 158, 6 | 12,0 % | dP0 .4R 5 | 0,35 | 4,9 | 8 | 39,0 | DMS O | 55,0% |
| **40** | 0,7 | 10,0% | 24,0% | P1 R4 | 1 | 3,8 | 23 | 86,4 | 16,0 % | dP0 .4R 5 | 0,35 | 5,02 | 8 | 39,9 | DMS O | 60,0% |
| **41** | 0,7 | 10,0% | 18,0% | P2 R3 | 2 | 3,5 | 45 | 158, 6 | 12,0 % | dP0 .4R 5 | 0,35 | 5,02 | 8 | 39,9 | DMS O | 60,0% |
| **42** | 0,7 | 10,0% | 24,0% | P1 R4 | 1 | 4,0 | 23 | 89,8 | 16,0 % | dP0 .4R 5 | 0,35 | 5,02 | 8 | 39,9 | DMS O | 60,0% |
| **43** | 0,7 | 10,0% | 24,0% | P1 R4 | 1 | 3,8 | 23 | 86,4 | 16,0 % | dP0 .4R 5 | 0,35 | 5,02 | 8 | 39,9 | DMS O | 60,0% |
| **44** | 0,7 | 10,0% | 24,0% | P1 R4 | 1 | 4,0 | 23 | 89,8 | 16,0 % | dP0 .4R 5 | 0,35 | 5,02 | 8 | 39,9 | DMS O | 60,0% |

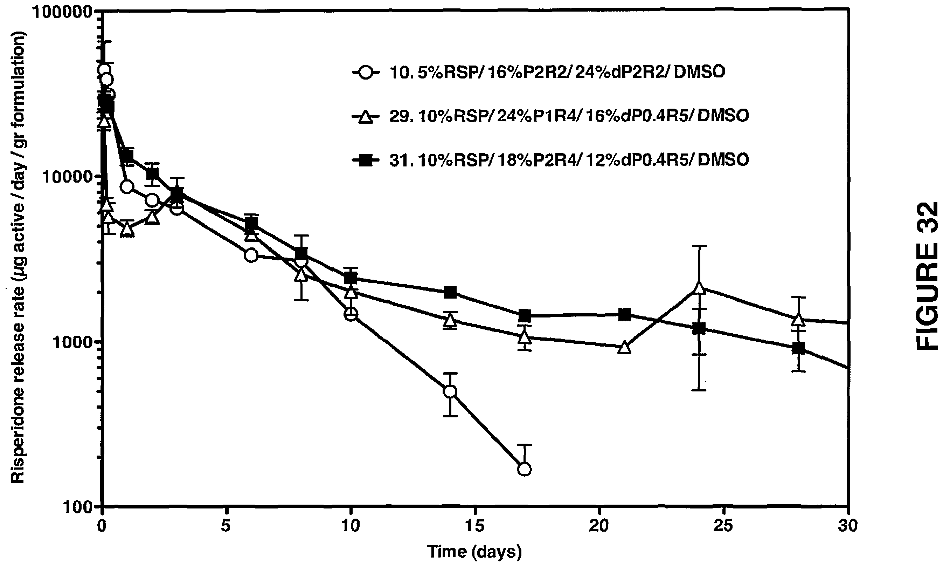

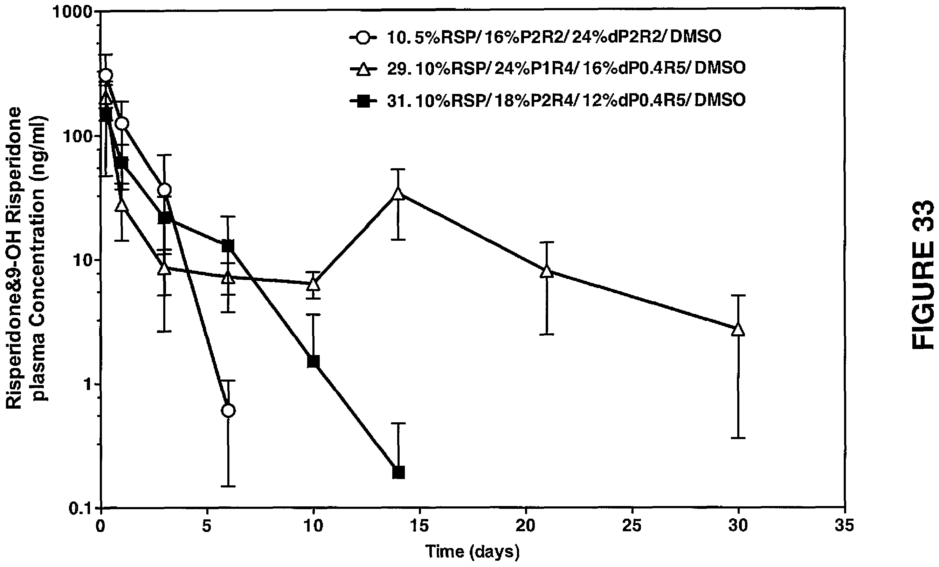

The results of these formulations are illustrated in Figures 32 and 33.

### Example 7-Ivermectin's formulations preparation (not according to the invention)

The formulations described herein were based on organic solution of polymers prepared as in Example 1, containing as the drug, ivermectin. Typically, 0.4 grams of polymers, corresponding to a mix of a diblock copolymer and a triblock copolymer in defined mass ratio, were dissolved in 0.55 grams of dimethyl sulfoxide at room temperature overnight under constant magnetic stirring. The next day, 50 mg of ivermectin was added to the polymer solution and stirred until complete dissolution. Three different formulations were selected for in vivo experiments. The composition of these formulations is shown in Table 5 below. The formulations were injected subcutaneously in the interscapular space of male dogs (10 to 17 kg) at a final dose of 0.6 mg/kg of ivermectin. Blood samples were withdrawn periodically and analyzed for ivermectin concentrations by LC/MS/MS.

The formulations are shown in Table 5.

**Table 5**

| | | **IVM** | **Triblock copolymer (TB)** | | | | | | **Diblock copolymer (DB)** | | | | | | **Solvent** | |
|---|---|---|---|---|---|---|---|---|---|---|---|---|---|---|---|---|
| **Exp n°** | **Ratio DB/TB** | **% (w/w)** | **% (w/w)** | **Code** | **PEG (kDa)** | **Ratio (LA/EO)** | **DP-PEG** | **DP-PLA** | **% (w/w)** | **Code** | **PEG (kDa)** | **Ratio (LA/EO)** | **DP-PEG** | **DP-PLA** | **Name** | **% (w/w)** |
| **9** | **1,7** | 5,0% | 15,0% | P3 R3 | 3 | 3,2 | 68 | 218 | 25,0% | dP0.4R5 | 0,35 | 4,9 | 8 | 39 | DMSO | 55,0% |
| **10** | **1,7** | 5,0% | 15,0% | P2 R3 | 2 | 3,5 | 45 | 159 | 25,0% | dP2R3 | 2 | 2,9 | 45 | 132 | DMSO | 55,0% |
| **11** | **1,7** | 5,0% | 15,0% | P2 R5 | 2 | 5,3 | 45 | 241 | 25,0% | dP2R2 | 2 | 2,3 | 45 | 105 | DMSO | 55,0% |

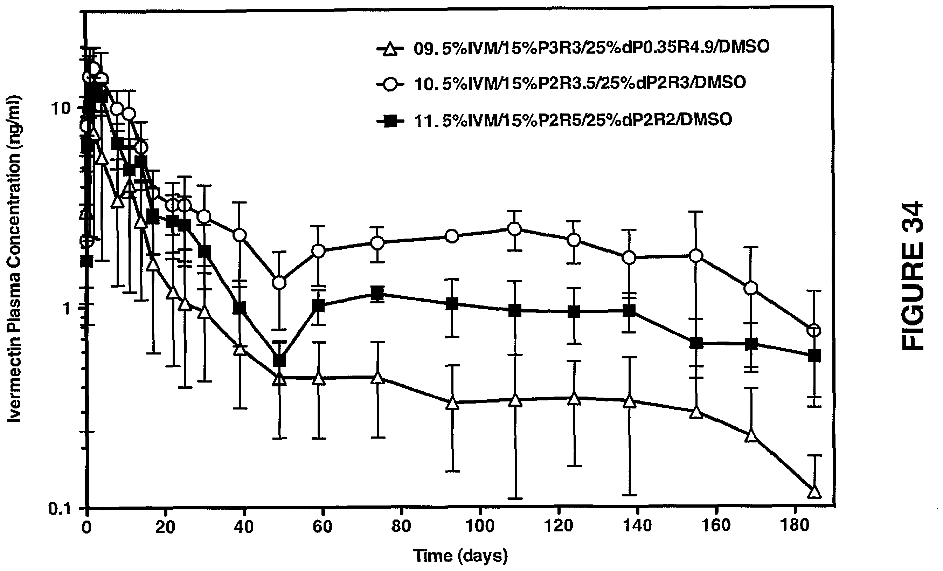

The results are illustrated in Figure 34.

### Example 8-Medroxyprogesterone Acetate's formulations preparations

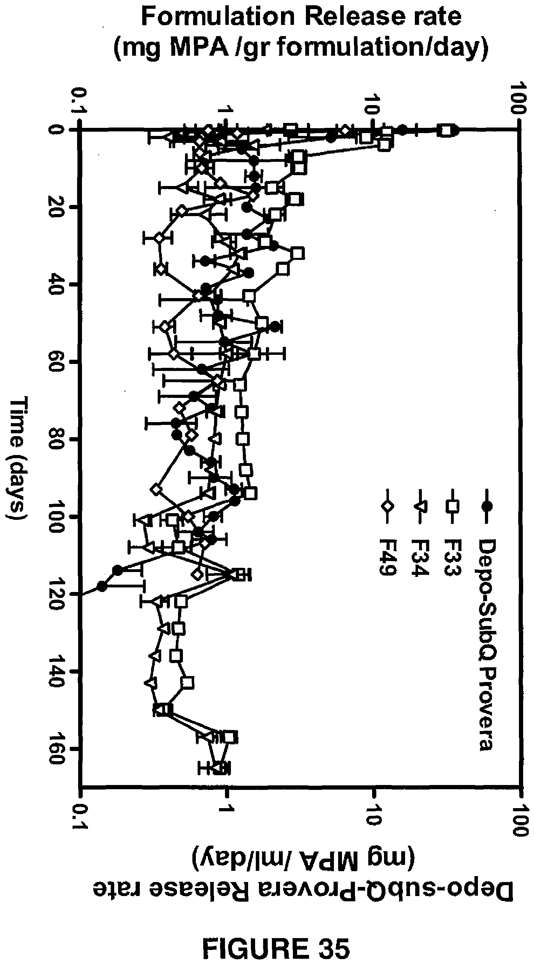

The formulations as described herein are based on organic solutions of the polymers as described in Example 1, containing as the drug medroxyprogesterone acetate. Typically 0.4 grams of polymers corresponding to a mix of diblock and triblock copolymer in a defined mass ratio were dissolved in 0.3 grams of DMSO or a combination of DMSO and benzyl alcohol at room temperature overnight with constant magnetic stirring. The next day the polymer solution was filtered through a 0.22 µm filter and 0.3 grams of medroxyprogesterone acetate was added to the filtered polymer solution and stirred until a homogeneous suspension of the drug was obtained. The formulations were loaded into a syringe before use. The compositions are shown in Table 6 below. The formulations were injected subcutaneously in the interscapular space of female dogs (11.4 to 14.1 kg). Blood samples were withdrawn periodically and analyzed for medroxyprogesterone acetate concentrations by LC/MS/MS having a below limit of quantification of 0.25 ng/ml. The results are shown in Figure 35.

The formulations are shown in Table 6.

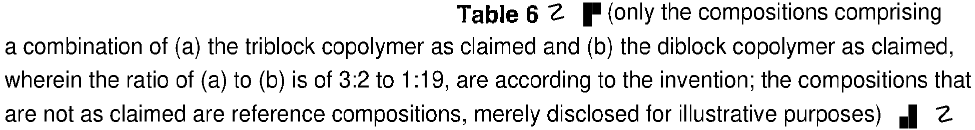

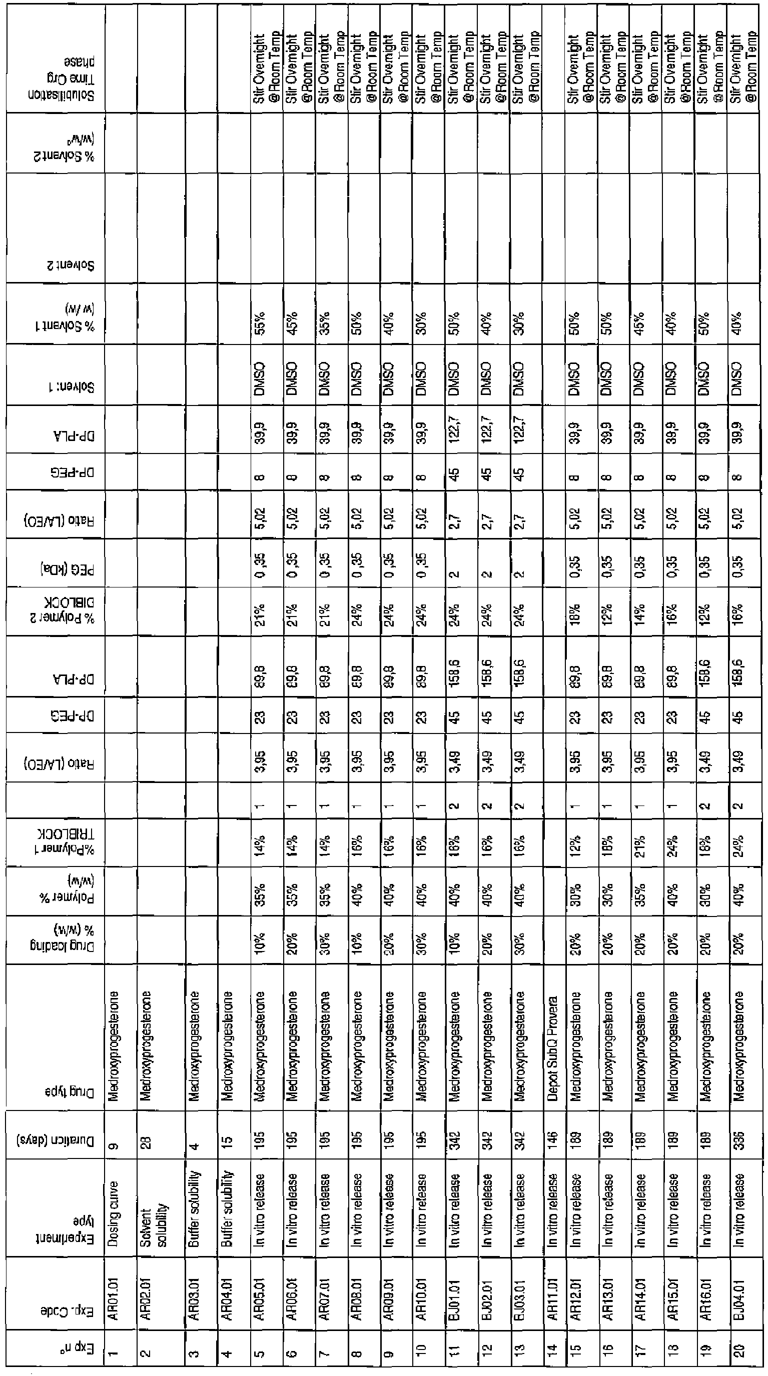

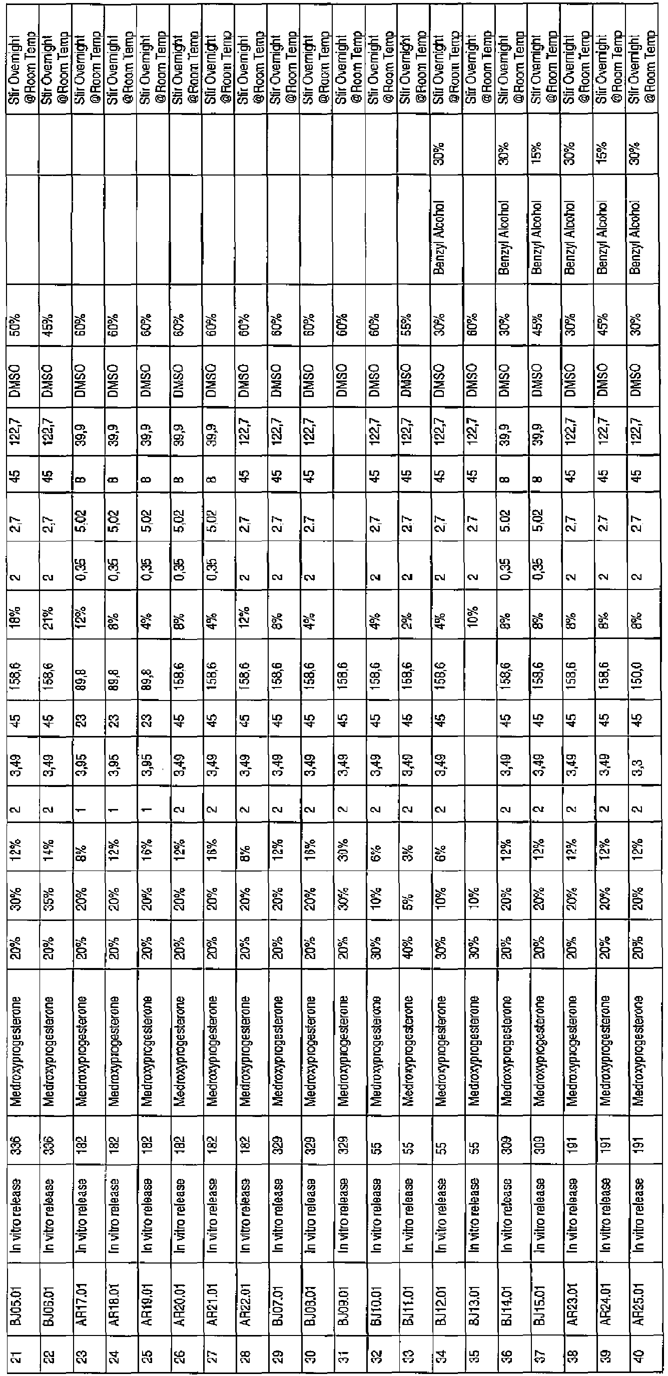

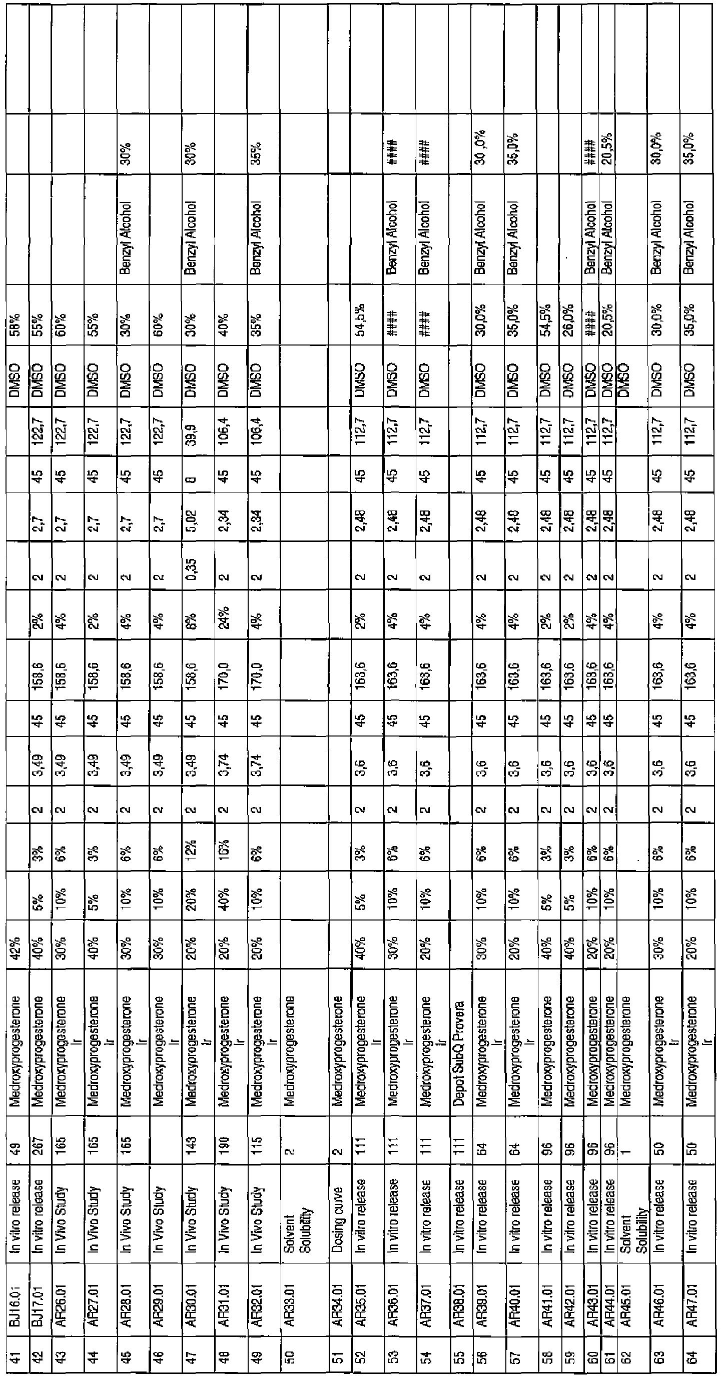

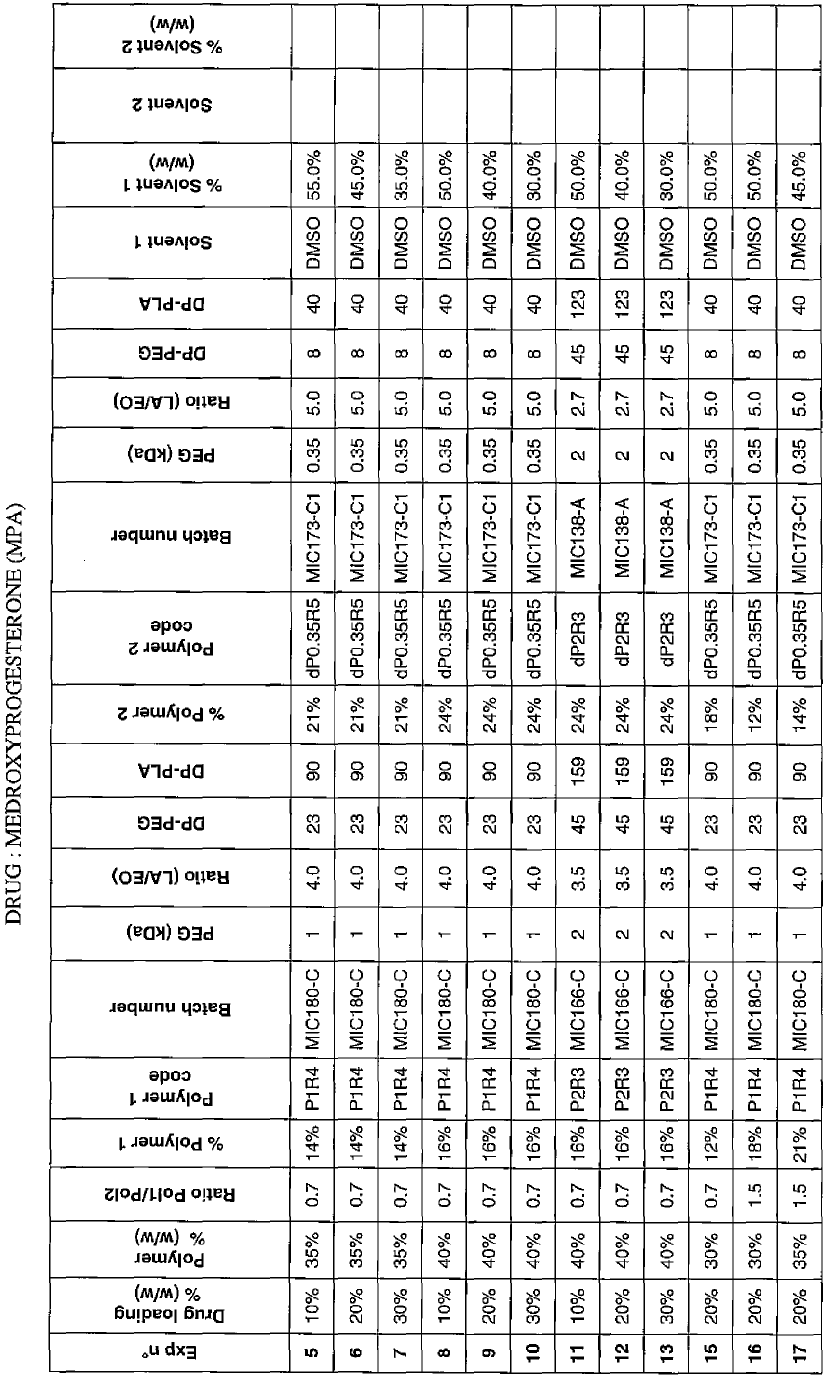

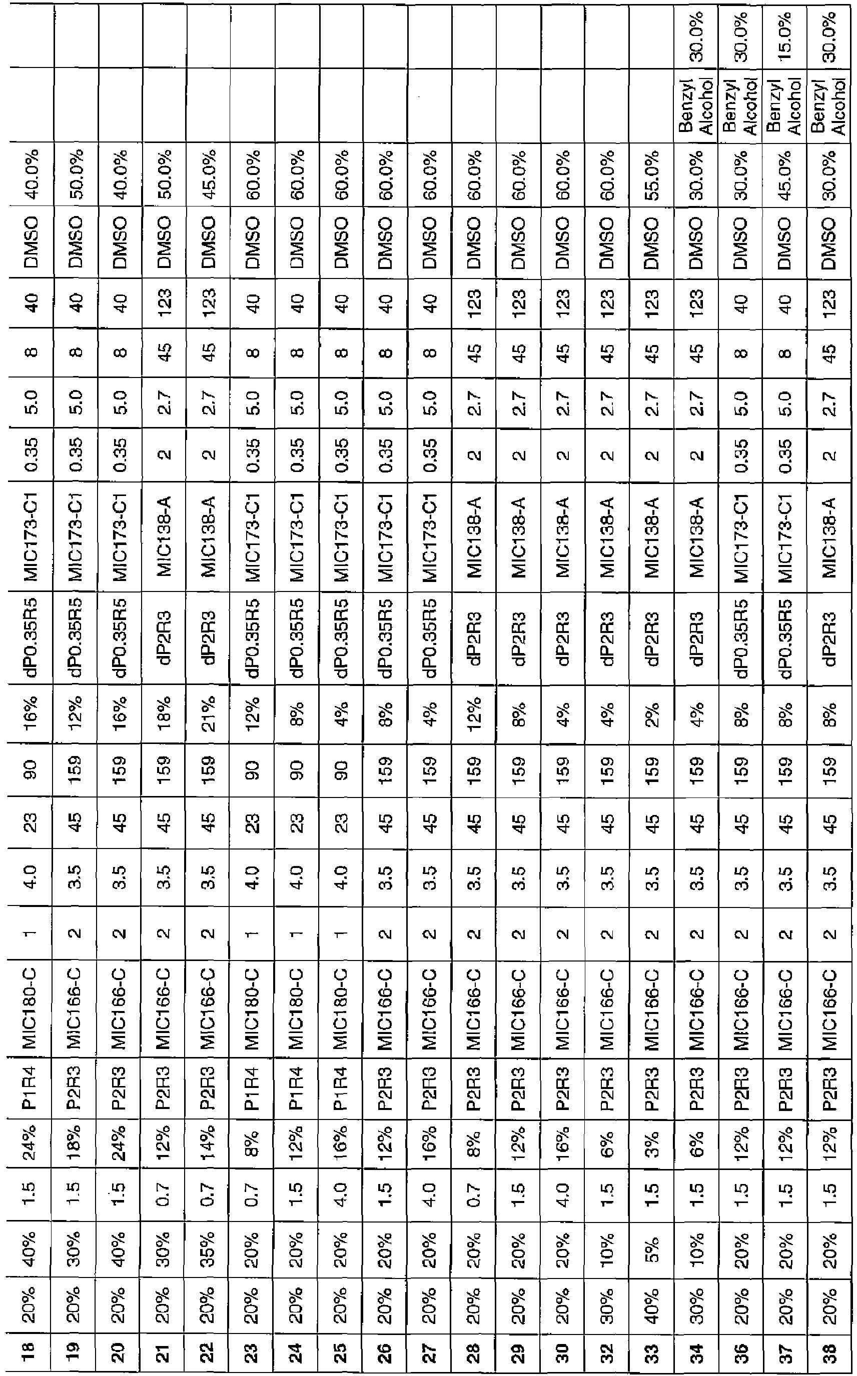

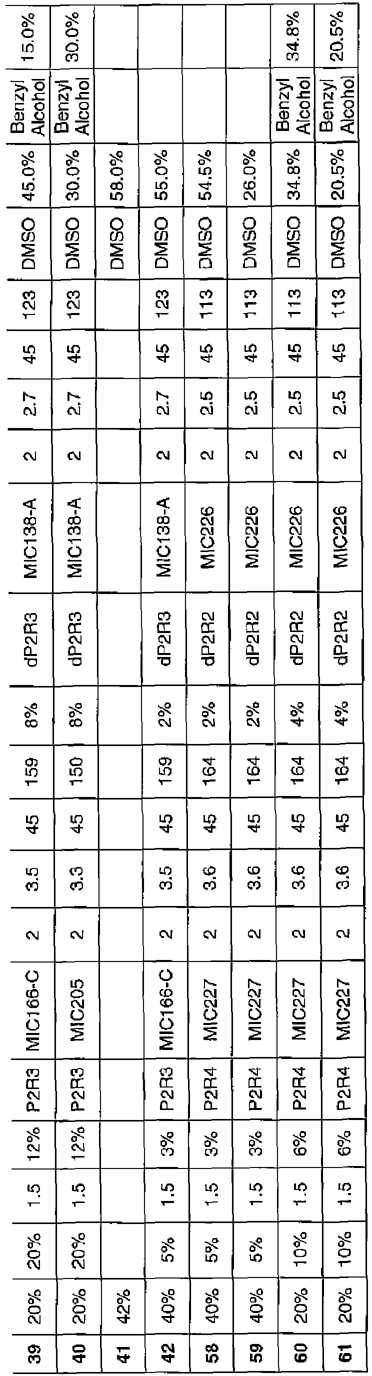

### Example 9-Progesterone formulations preparations

The formulations as described herein are based on organic solutions of the polymers as described in Example 1, containing as the drug progesterone. Typically 0.1 grams of polymers corresponding to a mix of diblock and triblock copolymer in a defined mass ratio were dissolved in 0.6 grams of DMSO at room temperature overnight with constant magnetic stirring. The next day the polymer solution was filtered through a 0.22 µm filter and 0.3 grams of progesterone was added to the filtered polymer solution and stirred until a homogeneous suspension of the drug was obtained. The formulations were loaded into a syringe before use. The compositions are shown in Table 7 below (only the compositions comprising a combination of (a) the triblock copolymer as claimed and (b) the diblock copolymer as claimed, wherein the ratio of (a) to (b) is of 3:2 to 1:19, are according to the invention; the compositions that are not as claimed are reference compositions, merely disclosed for illustrative purposes).

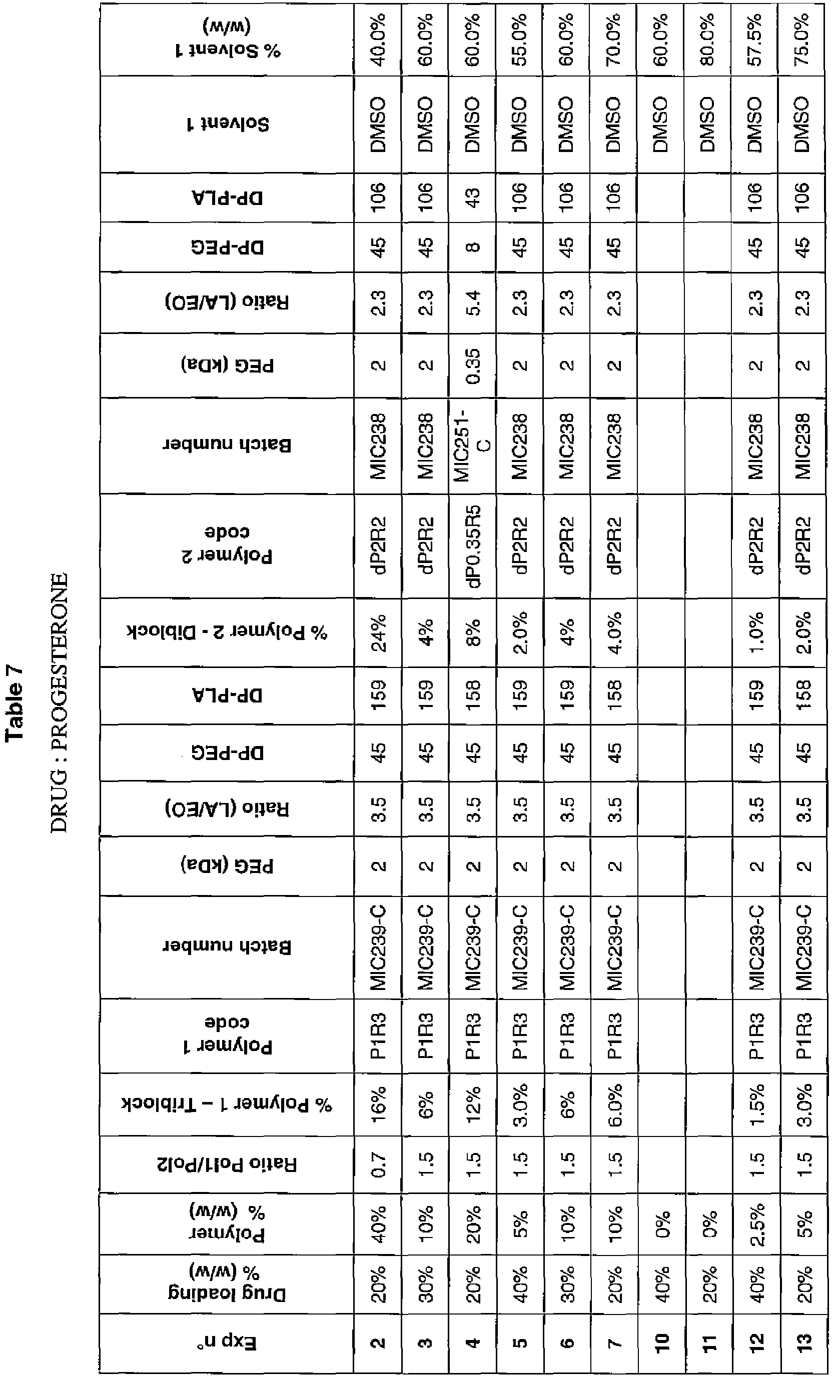

### Example 10-Levonorgestrel formulations preparations

The formulations as described herein are based on organic solutions of the polymers as described in Example 1, containing as the drug Levonorgestrel. Typically 0.1 grams of polymers corresponding to a mix of diblock and triblock copolymer in a defined mass ratio were dissolved in 0.7 grams of DMSO at room temperature overnight with constant magnetic stirring. The next day the polymer solution was filtered through a 0.22 µm filter and 0.2 grams of Levonorgestrel was added to the filtered polymer solution and stirred until a homogeneous suspension of the drug was obtained. The formulations were loaded into a syringe before use. The compositions are shown in Table 8 below (only the compositions comprising a combination of (a) the triblock copolymer as claimed and (b) the diblock copolymer as claimed, wherein the ratio of (a) to (b) is of 3:2 to 1:19, are according to the invention; the compositions that are not as claimed are reference compositions, merely disclosed for illustrative purposes).

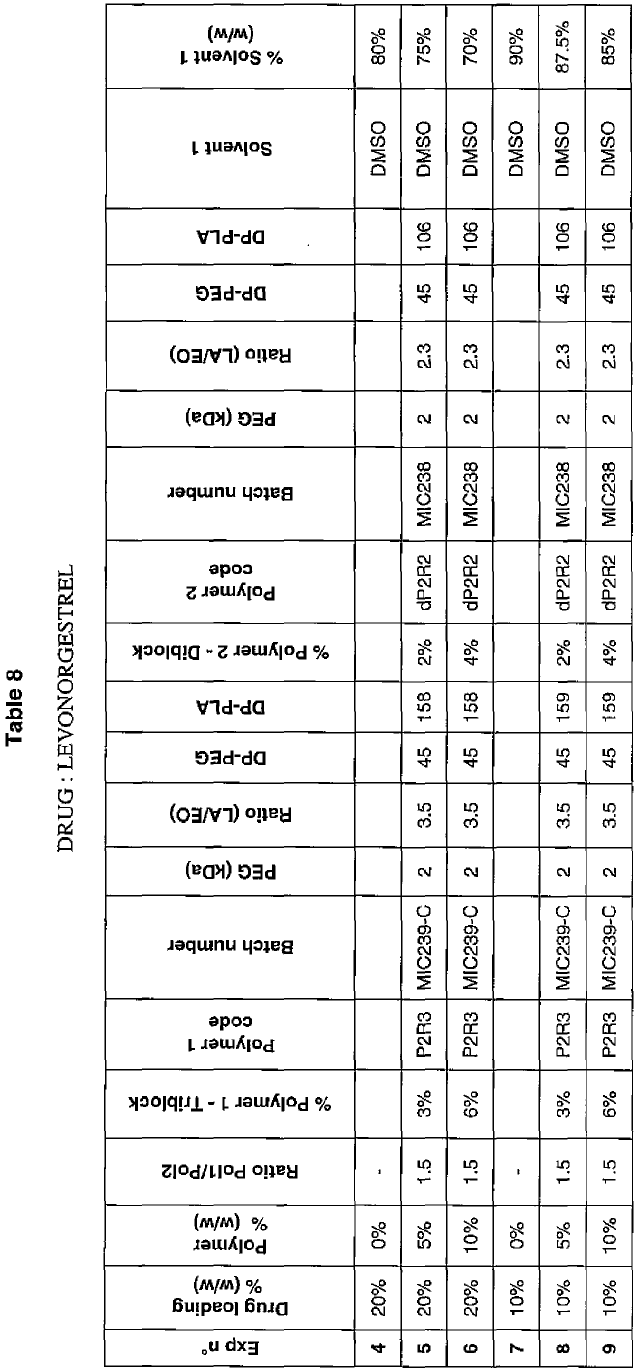

### Example 11-Cyclosporine formulations preparations

The formulations as described herein are based on organic solutions of the polymers as described in Example 1, containing as the drug cyclosporine. Typically 0.15grams of polymers corresponding to a mix of diblock and triblock copolymer in a defined mass ratio were dissolved in 0.65 grams of DMSO at room temperature overnight with constant magnetic stirring. The next day the polymer solution was filtered through a 0.22 µm filter and 0.2 grams of cyclosporine was added to the filtered polymer solution and stirred until a homogeneous suspension of the drug was obtained. The formulations were loaded into a syringe before use. The compositions are shown in Table 9 below.

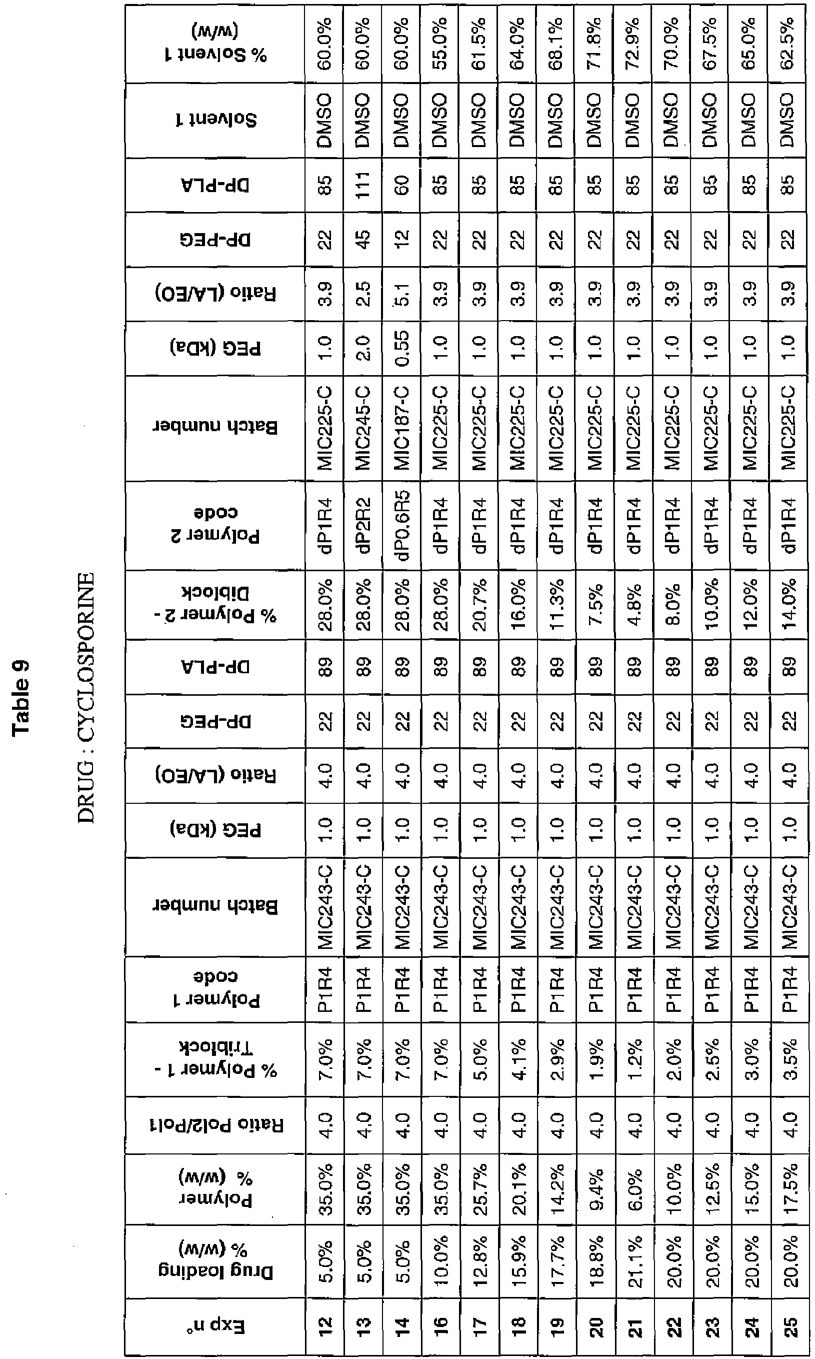

### Example 12-Bupivacaine formulations preparations

The formulations as described herein are based on organic solutions of the polymers as described in Example 1, containing as the drug Bupivacaine base. Typically 0.1 grams of polymers corresponding to a mix of diblock and triblock copolymer in a defined mass ratio were dissolved in 0.75 grams of DMSO at room temperature overnight with constant magnetic stirring. The next day the polymer solution was filtered through a 0.22 µm filter and 0.15 grams of Bupivacaine base was added to the filtered polymer solution and stirred until a homogeneous suspension of the drug was obtained. The formulations were loaded into a syringe before use. The compositions are shown in Table 10 below (only the compositions comprising a combination of (a) the triblock copolymer as claimed and (b) the diblock copolymer as claimed, wherein the ratio of (a) to (b) is of 3:2 to 1:19, are according to the invention; the compositions that are not as claimed are reference compositions, merely disclosed for illustrative purposes).

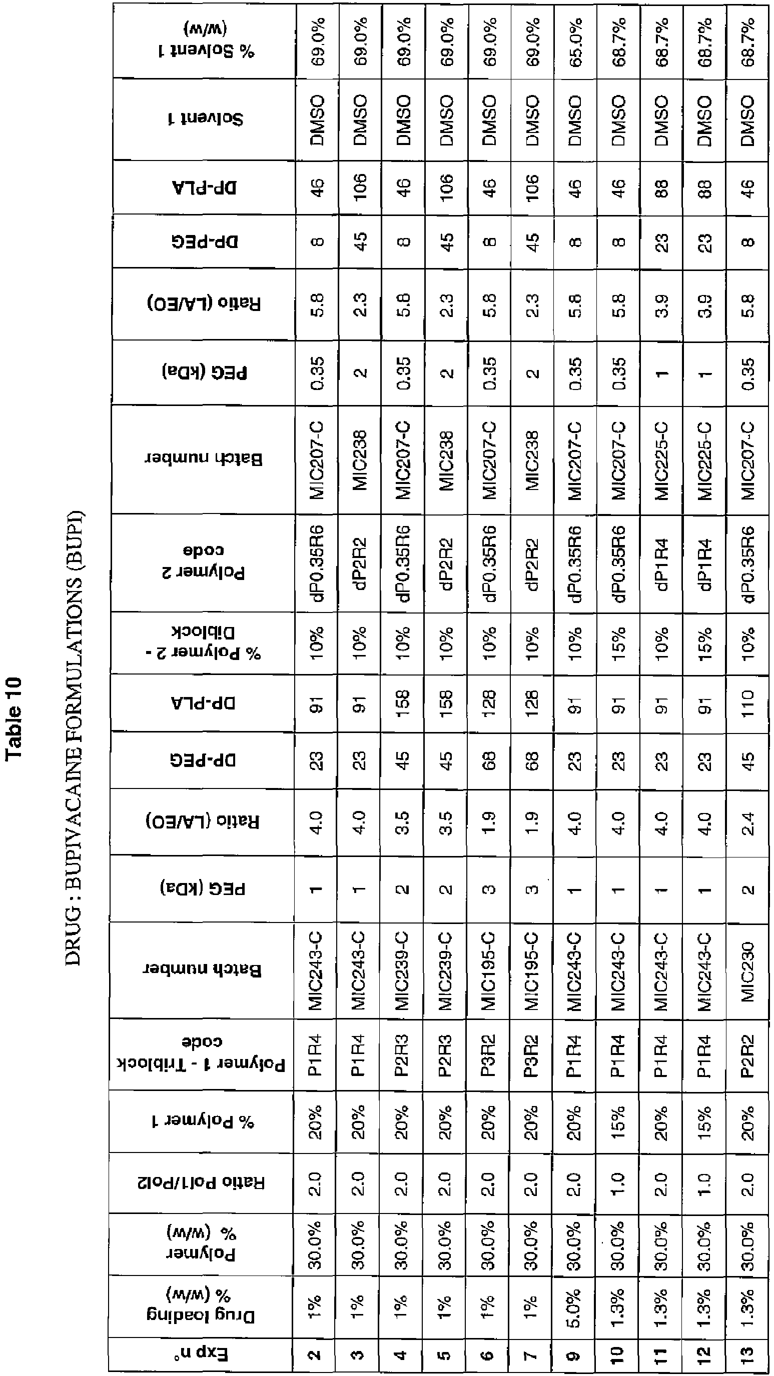

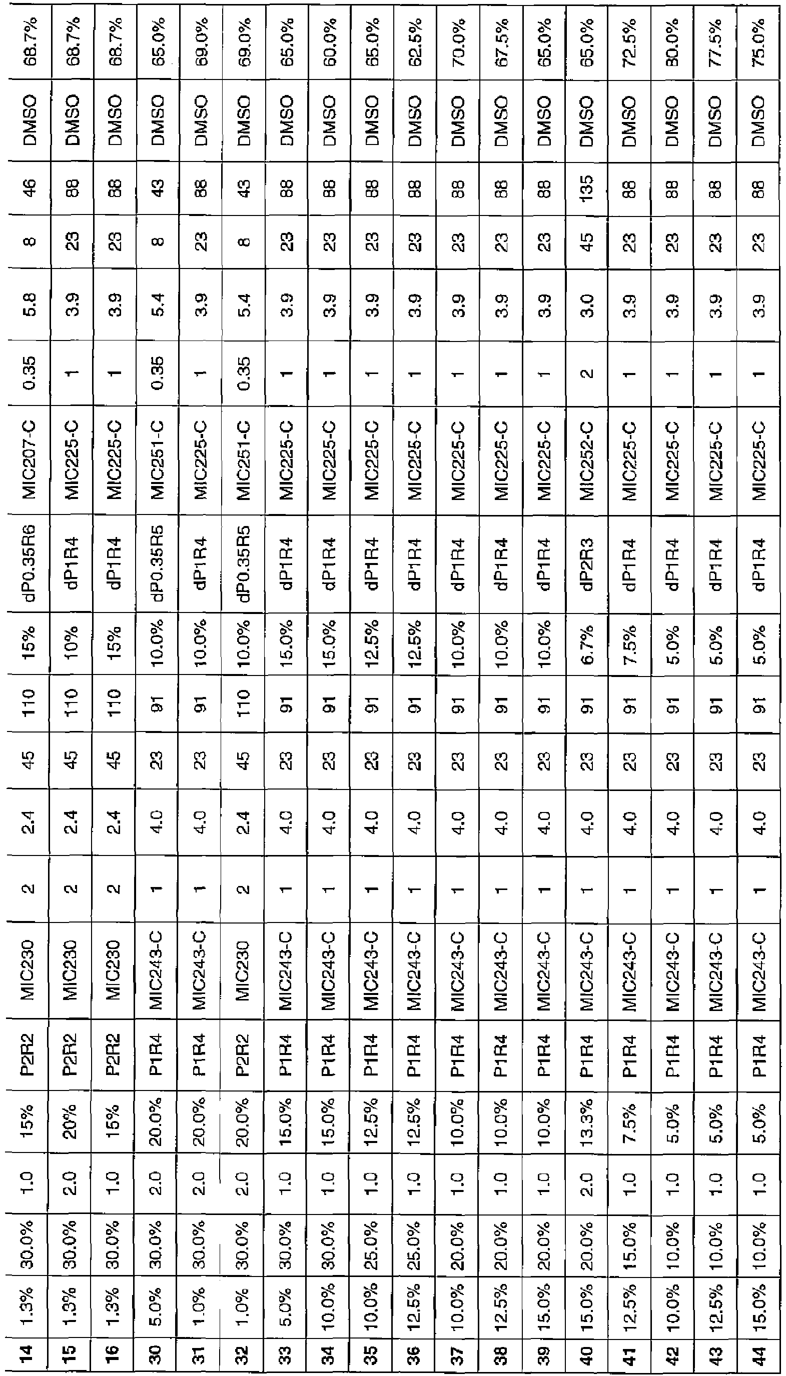

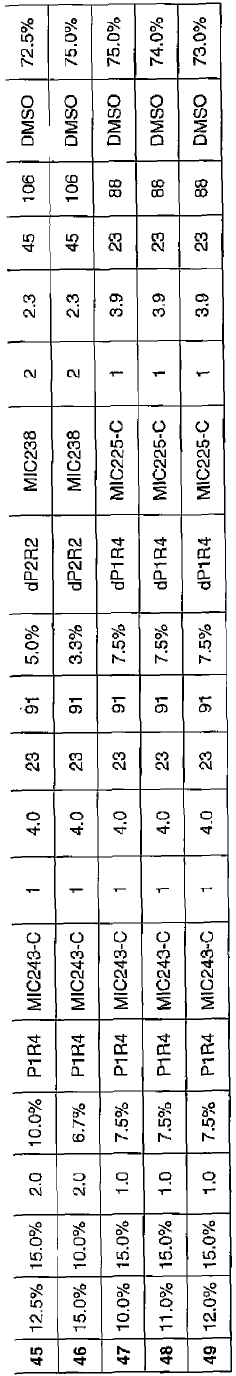

### Example 13-Injectability of differing compositions (only a ratio of 3:2 to 1:19 is according to the invention; the ratios that are not as claimed are reference ratios, merely disclosed for illustrative purposes).

Various formulations were tested for injectability using formulations with different ratios of triblock (TB) and diblock (DB). Different solutions in DMSO based on a mixture of the triblock copolymer P6R1(TB) and the diblock copolymer dP2R4(DB) were prepared.

A 50% weight%/weight % polymer/formulation mass was used in these viscosity experiments. The weight%/weight % of triblock to diblock that was used in this experiment were the following: 50 wt. %:0 wt. %, 45 wt. %:5 wt. %, 20 wt. %:5 wt. %, 35 wt. %:15 wt. %, 15 wt. %:10 wt. %, 25 wt. %:25 wt. %, 10 wt. %:15 wt. %, 15 wt. %:35 wt. %, 5 wt. %:20 wt. %, 5 wt. %:45 wt. % and 0 wt. %:50 wt. %.

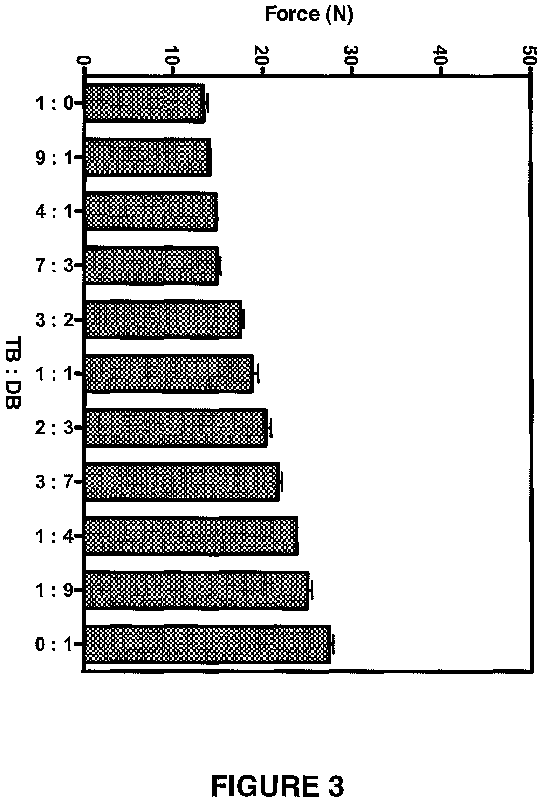

The injectability results are shown in Figure 3.

### Example 14- In vitro Release Assay (not according to the invention)

100 to 500 mg of formulation was added to 20 to 50 ml of physiological buffer. The physiological buffer that was used was KRT containing 50 ml Krebs / Ringer / Tris (KRT) buffer pH 7.4, which is 143 mM Sodium Chloride, 5.1 mM Potassium Chloride, 2.7 mM Calcium Chloride, 1.34 mM Magnesium Sulfate, 25 mM Tris-CI pH 7.4 and 0.1% sodium azide. Upon injection, the solvent diffused away from the formulation and the remaining polymer formed a solid biodegradable implant within the aqueous environment.

In order to maintain sink conditions, for drug release, the release medium was maintained under constant shaking at 180 rpm (Unimax 1010 apparatus, Heidolph) at 37°C. At pre-determined time intervals, media are collected and analyzed by HPLC. The amount of the GLP-1 analogue peptide M53, released from the formulation was calculated from a calibration curve. The concentration of M53 ranged between 0 and 5 mg/ml or it ranged between 0 and 200 µg/ml.

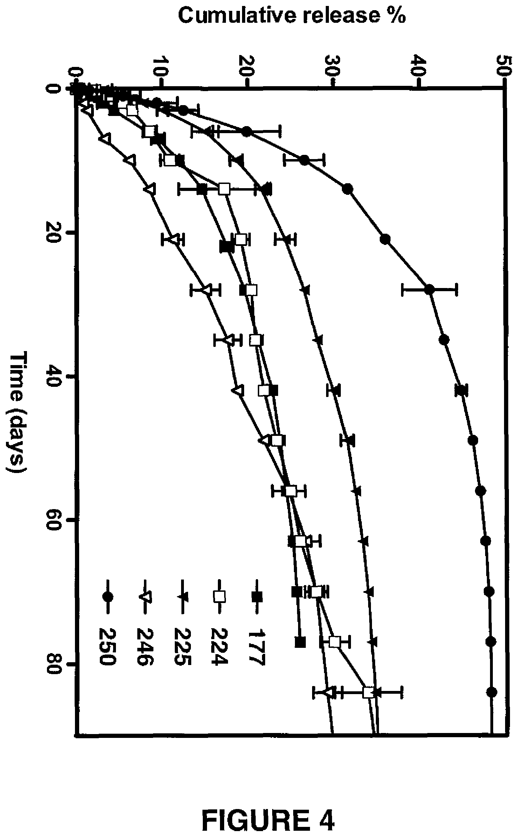

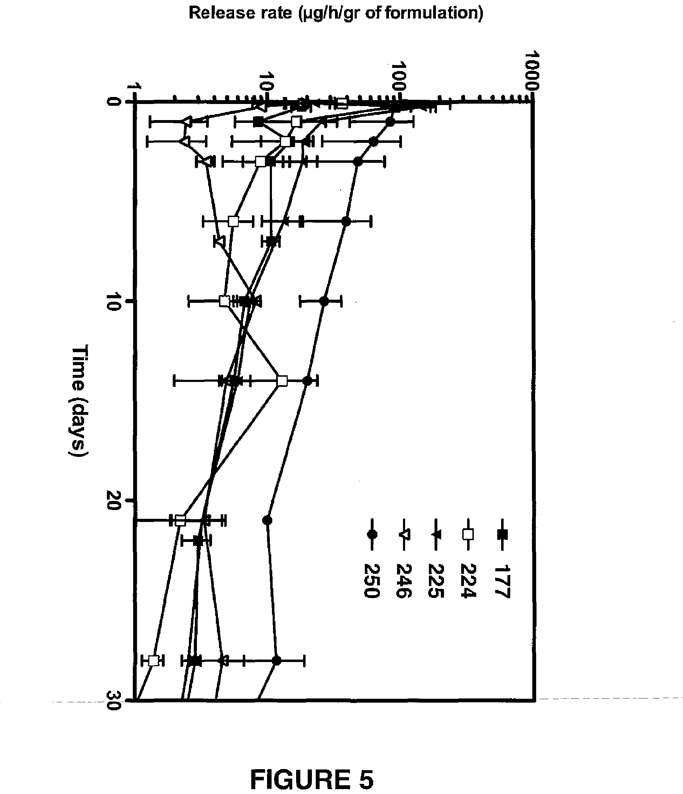

The results are shown in Figure 4 and Figure 5. Figure 5 illustrates the release rate of formulations 177, 224, 225, 246 and 250 as shown in Table 1, while Figure 4 shows the cumulative release of drug from the indicated formulations.

When the GPL-1 analogue was incorporated into the polymer solution, it was encapsulated within the polymer matrix as it solidified. The drug was then released either by diffusion inside the matrix or by biodegradation of the matrix.

### Example 15- Pharmacokinetic study (not according to the invention)

Several formulations were tested in a pharmacokinetic study in rats. Compositions containing 1 mg of drug per animal of the formulations of 177, 224, 225, 246 and 250, as set forth in Table 1 were subcutaneously administered to rats. Blood samples were collected into EDTA tubes at different time points, centrifuged and the plasma from each time point was retained. The plasma samples were analyzed by LC/MS/MS and quantified for drug content. Results are presented as ng/ml of plasma measured over time.

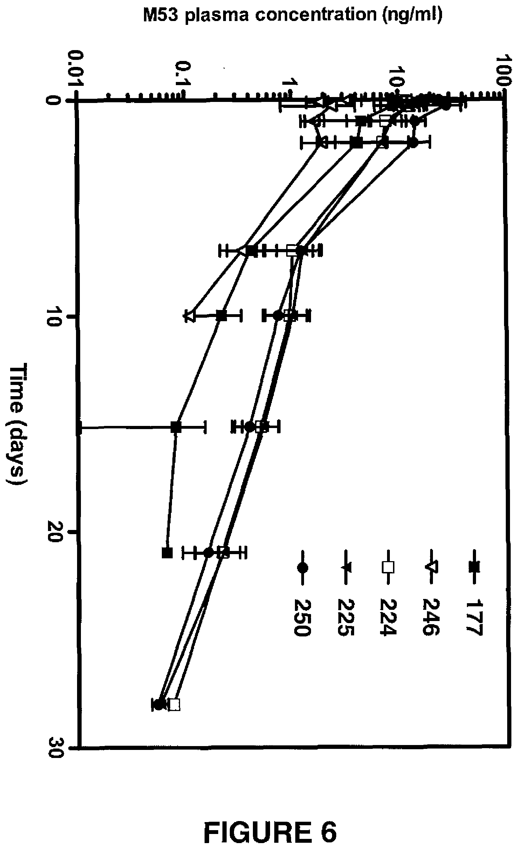

The results of one pharmacokinetic study are shown in Figure 6. As shown in this Figure three of the five formulations sustain plasma concentration higher than 0.1 ng/ml for more than 28 days while giving a moderate initial drug burst release below 30 ng/ml.

### Example 16- Blood Glucose Levels (not according to the invention)

Blood glucose levels with patients suffering from diabetes type 2 are taken prior to treatment. A control group having no treatment is used for this study. Patients of either gender are used in this study provided that they have diabetes type 2 and are between the ages of 35 and 60.

A GPL-1 analogue is formulated according to Examples 1 and 2 and has the chemical characteristics of number 230 in Table 1. The injectable liquid that is obtained is then injected into several patients at a dosage of 8 mg/ml. The control group is given PBS.

The amount of blood sugar levels and fructosamine is then measured for a period of 30 days, twice weekly, before meals and 2 hours after meals. The amounts of blood glucose after treatment are measured and the results are averaged. The values are shown in Table 11:

**Table 11**

| Week number | Patient number | Blood Glucose Level Before Meals in mmol/l | Blood Glucose Level After Meals In mmol/I | Fructosamine µmol |
|---|---|---|---|---|
| Prior to Treatment | 1 | 150 | 190 | 300 |
| | 2 | 130 | 175 | 320 |
| | 3 | 200 | 230 | 330 |
| | 4 | 220 | 240 | 360 |
| 1 | 1 | 90 | 150 | 280 |
| | 2 | 98 | 110 | 290 |
| | 3 | 120 | 160 | 330 |
| | 4 | 215 | 240 | 365 |
| 2 | 1 | 92 | 120 | 275 |
| | 2 | 95 | 100 | 287 |
| | 3 | 118 | 158 | 300 |
| | 4 | 210 | 230 | 370 |
| 3 | 1 | 92 | 110 | 270 |
| | 2 | 98 | 101 | 275 |
| | 3 | 115 | 155 | 280 |
| | 4 | 211 | 222 | 385 |
| 4 | 1 | 93 | 110 | 260 |
| | 2 | 85 | 100 | 260 |
| | 3 | 110 | 150 | 265 |
| | 4 | 223 | 244 | 365 |

Normal results for the glucose levels before meals range from 80 to 120 mmol/l. Normal results for the glucose levels after meals should be 160 mmol/l or less. Normal fructosamine levels are under 265. Between 265 and 280 indicates excellent blood glucose control; 280 and 500 indicates good blood glucose control;, between 320 and 340 indicates fair blood glucose control; and over 350 indicates poor blood glucose control.

Patient 4 was administered the placebo.

These results show that when administered the biodegradable drug delivery compositions of the present invention are effective to treat diabetes type 2.

The scope of the present invention is limited by the scope of the claims.

## Claims

1. A biodegradable drug delivery composition comprising:
(a) a biodegradable triblock copolymer having the formula:
poly(lactic acid)ᵥ-poly(ethylene glycol)w-poly(lactic acid)x
wherein v, w and x are the number of repeat units ranging from 4 to 1090 or 6 to 1090 and v=x or v≠x; and
(b) a biodegradable diblock copolymer having the formula:
methoxy poly(ethylene glycol)_{y}-(polylactic acid)_{z}
wherein y and z are the number of repeat units ranging from 7 to 371 or 3 to 237,
wherein the ratio of the biodegradable triblock copolymer of (a) and the biodegradable diblock copolymer of (b) is 3:2 to 1:19 in said biodegradable drug composition; and (c) at least one pharmaceutically active hydrophobic principle,
wherein said at least one pharmaceutically active hydrophobic principle is levonorgestrel, cyclosporine, progesterone, bupivacaine or medroxyprogesterone acetate.

2. A biodegradable drug delivery composition according to claim 1 wherein said biodegradable drug delivery composition is a partial suspension.

3. A biodegradable drug delivery composition according to claim 1 or claim 2, wherein the biodegradable triblock copolymer of (a) is present in an amount of 3.0% to 45% (w%/w%) or 2% to 45% (w%/w%) of the total composition;
the biodegradable diblock copolymer of (b) is present in an amount of 8.0% to 50% (w%/w%) of the total composition; and the at least one pharmaceutically active hydrophobic principle is present in an amount of 1% to 20% (w%/w%) or 1% to 40% (w%/w%) of the total composition.

4. A biodegradable drug delivery composition according to any preceding claim, wherein a lactic acid to ethylene oxide molar ratio in said composition is between 0.5 to 3.5 or between 0.5 to 2.5 or between 0.5 to 22.3 for the triblock copolymer and between 2 to 6 or between 0.8 to 13 or between 3 to 5 for the diblock copolymer.

5. A biodegradable drug delivery composition according to any preceding claim, wherein said composition is an injectable liquid that when inserted into the body of an animal or plant becomes a hardened implant.

6. A method for preparing a biodegradable drug delivery composition, said method comprising:
(i) dissolving in an organic solvent
(a) a biodegradable ABA type block copolymer having the formula:
poly(lactic acid)ᵥ-poly(ethylene glycol)w-poly(lactic acid)x
wherein v, w and x are the number of repeat units ranging from 6 to 1090 or 4 to 1090 and v=x or v≠x; and
(b) a biodegradable diblock copolymer having the formula:
methoxy poly(ethylene glycol)_{y}-(polylactic acid)_{z}
wherein y and z are the number of repeat units ranging from 7 to 371 or 3 to 237, in a ratio of 3:2 to 1:19 (a):(b) to form a polymer mixture; and
(ii) adding at least one pharmaceutically active hydrophobic principle to said polymer mixture, wherein said at least one pharmaceutically active hydrophobic principle is levonorgestrel, cyclosporine, progesterone, bupivacaine or medroxyprogesterone acetate.

7. A method for preparing the biodegradable drug delivery composition according to claim 6, which method further comprises (iii) evaporating said solvent.

8. A method for preparing the biodegradable drug delivery composition according to claim 7, wherein said biodegradable drug delivery composition is a partial suspension.

9. A method according to any one of claims 6 to 8, wherein the organic solvent is present in an amount of 40% to 74% (w%/w%) or 30% to 70% (w%/w%) or 26% to 90% (w%/w%) of the total composition.

## Patentansprüche

1. Biologisch abbaubare Arzneistoffzuführungszusammensetzung, umfassend:
(a) ein biologisch abbaubares Triblockcopolymer mit der Formel:
Poly(milchsäure)ᵥ-poly(ethylenglykol)_{w}-poly(milch-säure)ₓ,
wobei v, w und x für die Zahl von Wiederholungseinheiten im Bereich von 4 bis 1090 oder 6 bis 1090 stehen und v=x oder v≠x; und
(b) ein biologisch abbaubares Diblockcopolymer mit der Formel:
Methoxypoly(ethylenglykol)_{y}-poly(milchsäure)_{z},
wobei y und z für die Zahl von Wiederholungseinheiten im Bereich von 7 bis 371 oder 3 bis 237 stehen, wobei das Verhältnis von biologisch abbaubarem Triblockcopolymer von (a) zu biologisch abbaubarem Diblockcopolymer von (b) in der biologisch abbaubaren Arzneistoffzusammensetzung 3:2 bis 1:19 beträgt; und
(c) mindestens einen hydrophoben pharmazeutischen Wirkstoff, wobei es sich bei dem mindestens einen hydrophoben pharmazeutischen Wirkstoff um Levonorgestrel, Cyclosporin, Progesteron, Bupivacain oder Medroxyprogesteronacetat handelt.

2. Biologisch abbaubare Arzneistoffzuführungszusammensetzung nach Anspruch 1, wobei es sich bei der biologisch abbaubaren Arzneistoffzuführungszusammensetzung um eine partielle Suspension handelt.

3. Biologisch abbaubare Arzneistoffzuführungszusammensetzung nach Anspruch 1 oder 2, wobei das biologisch abbaubare Triblockcopolymer von (a) in einer Menge von 3,0 % bis 45 % (Gew.-%/Gew.-%) oder 2 % bis 45 % (Gew.-%/Gew.-%) der gesamten Zusammensetzung vorliegt;
das biologisch abbaubare Diblockcopolymer von (b) in einer Menge von 8,0 % bis 50 % (Gew.-%/Gew.-%) der gesamten Zusammensetzung vorliegt und
der mindestens eine hydrophobe pharmazeutische Wirkstoff in einer Menge von 1 % bis 20 % (Gew.%/Gew.-%) oder 1 % bis 40 % (Gew.-%/Gew.-%) der gesamten Zusammensetzung vorliegt.

4. Biologisch abbaubare Arzneistoffzuführungszusammensetzung nach einem der vorhergehenden Ansprüche, wobei das Molverhältnis von Milchsäure zu Ethylenoxid in der Zusammensetzung zwischen 0,5 bis 3,5 oder zwischen 0,5 bis 2,5 oder zwischen 0,5 bis 22,3 für das Triblockcopolymer und zwischen 2 bis 6 oder zwischen 0,8 bis 13 oder zwischen 3 bis 5 für das Diblockcopolymer liegt.

5. Biologisch abbaubare Arzneistoffzuführungszusammensetzung nach einem der vorhergehenden Ansprüche, wobei es sich bei der Zusammensetzung um eine injizierbare Flüssigkeit handelt, die bei Einführung in den Körper eines Tieres oder einer Pflanze zu einem gehärteten Implantat wird.

6. Verfahren zur Herstellung einer biologisch abbaubaren Arzneistoffzuführungszusammensetzung, wobei das Verfahren Folgendes umfasst:
(i) Lösen
(a) eines biologisch abbaubaren Blockcopolymers vom A-B-A-Typ mit der Formel:
Poly(milchsäure)ᵥ-poly(ethylenglykol)_{w}-poly(milch-säure)ₓ,
wobei v, w und x für die Zahl von Wiederholungseinheiten im Bereich von 6 bis 1090 oder 4 bis 1090 stehen und v=x oder v≠x; und
(b) eines biologisch abbaubaren Diblockcopolymers mit der Formel:
Methoxypoly(ethylenglykol)_{y}-poly(milchsäure)_{z},
wobei y und z für die Zahl von Wiederholungseinheiten im Bereich von 7 bis 371 oder 3 bis 237 stehen, in einem Verhältnis von 3:2 bis 1:19 (a):(b) in einem organischen Lösungsmittel zur Bildung einer Polymermischung; und
(ii) Zugeben mindestens eines hydrophoben pharmazeutischen Wirkstoffs zu der Polymermischung, wobei es sich bei dem mindestens einen hydrophoben pharmazeutischen Wirkstoff um Levonorgestrel, Cyclosporin, Progesteron, Bupivacain oder Medroxyprogesteronacetat handelt.

7. Verfahren zur Herstellung der biologisch abbaubaren Arzneistoffzuführungszusammensetzung nach Anspruch 6, wobei das Verfahren ferner (iii) das Verdampfen des Lösungsmittels umfasst.

8. Verfahren zur Herstellung der biologisch abbaubaren Arzneistoffzuführungszusammensetzung nach Anspruch 7, wobei es sich bei der biologisch abbaubaren Arzneistoffzuführungszusammensetzung um eine partielle Suspension handelt.

9. Verfahren nach einem der Ansprüche 6 bis 8, wobei das organische Lösungsmittel in einer Menge von 40 % bis 74 % (Gew.-%/Gew.-%) oder 30 % bis 70 % (Gew.- %/Gew.-%) oder 26 % bis 90 % (Gew.-%/Gew.-%) der gesamten Zusammensetzung vorliegt.

## Revendications

1. Composition d'administration de médicament biodégradable comprenant :
(a) un copolymère tribloc biodégradable possédant la formule :
poly(acide lactique)ᵥ-poly(éthylèneglycol)_{w}-poly(acide lactique) ₓ,
v, w et x étant le nombre de motifs répétitifs dans la plage de 4 à 1 090 ou de 6 à 1 090 et v = x ou v ≠ x ; et
(b) un copolymère dibloc biodégradable possédant la formule :
méthoxy poly(éthylèneglycol)_{y}-poly(acide lactique)_{z},
y et z étant le nombre de motifs répétitifs dans la plage de 7 à 371 ou de 3 à 237,
le rapport du copolymère tribloc biodégradable de (a) et du copolymère dibloc biodégradable de (b) étant de 3 : 2 à 1 : 19 dans ladite composition de médicament biodégradable et
(c) au moins un principe hydrophobe pharmaceutiquement actif, ledit au moins un principe hydrophobe pharmaceutiquement actif étant le lévonorgestrel, la cyclosporine, la progestérone, la bupivacaïne ou l'acétate de médroxyprogestérone.

2. Composition d'administration de médicament biodégradable selon la revendication 1, ladite composition d'administration de médicament biodégradable étant une suspension partielle.

3. Composition d'administration de médicament biodégradable selon la revendication 1 ou la revendication 2, le copolymère tribloc biodégradable de (a) étant présent en une quantité de 3,0 % à 45 % (% en poids/% en poids) ou de 2 % à 45 % (% en poids/% en poids) de la composition totale ;
le copolymère dibloc biodégradable de (b) étant présent en une quantité de 8,0 % à 50 % (% en poids/% en poids) de la composition totale ; et l'au moins un principe hydrophobe pharmaceutiquement actif étant présent en une quantité de 1 % à 20 % (% en poids/% en poids) ou de 1 % à 40 % (% en poids/% en poids) de la composition totale.

4. Composition d'administration de médicament biodégradable selon une quelconque revendication précédente, un rapport molaire d'acide lactique sur oxyde d'éthylène dans ladite composition étant compris entre 0,5 et 3,5 ou entre 0,5 et 2,5 ou entre 0,5 et 22,3 pour le copolymère tribloc et entre 2 et 6 ou entre 0,8 et 13 ou entre 3 et 5 pour le copolymère dibloc.

5. Composition d'administration de médicament biodégradable selon une quelconque revendication précédente, ladite composition étant un liquide injectable qui, lorsqu'il est inséré dans le corps d'un animal ou d'un végétal, devient un implant durci.

6. Procédé pour la préparation d'une composition d'administration de médicament biodégradable, ledit procédé comprenant :
(i) la dissolution dans un solvant organique
(a) d'un copolymère à blocs de type ABA biodégradable possédant la formule :
poly(acide lactique)ᵥ-poly(éthylèneglycol) poly(acide lactique)ₓ,
v, w et x étant le nombre de motifs répétitifs dans la plage de 6 à 1 090 ou de 4 à 1 090 et v = x ou v ≠ x ; et
(b) d'un copolymère dibloc biodégradable possédant la formule :
méthoxy poly(éthylèneglycol)_{y}-poly(acide lactique)_{z},
y et z étant le nombre de motifs répétitifs dans la plage de 7 à 371 ou de 3 à 237,
en un rapport de 3 : 2 à 1 : 19 (a) : (b) pour former un mélange de polymères ; et
(ii) l'ajout d'au moins un principe hydrophobe pharmaceutiquement actif audit mélange de polymères, ledit au moins un principe hydrophobe pharmaceutiquement actif étant le lévonorgestrel, la cyclosporine, la progestérone, la bupivacaïne ou l'acétate de médroxyprogestérone.

7. Procédé pour la préparation de la composition d'administration de médicament biodégradable selon la revendication 6, lequel procédé comprend en outre (iii) l'évaporation dudit solvant.

8. Procédé pour la préparation de la composition d'administration de médicament biodégradable selon la revendication 7, ladite composition d'administration de médicament biodégradable étant une suspension partielle.

9. Procédé selon l'une quelconque des revendications 6 à 8, le solvant organique étant présent en une quantité de 40 % à 74 % (% en poids/% en poids) ou de 30 % à 70 % (% en poids/% en poids) ou de 26 % à 90 % (% en poids/% en poids) de la composition totale.
